# EUROPEAN PATENT SPECIFICATION

(11) **EP 3 965 751 B1**
(45) Date of publication and mention of the grant of the patent: **28.12.2022**
(21) Application number: 20725500.1
(22) Date of filing: 08.05.2020
(51) Int. Cl.: A61K 31/166, A61K 31/661, A61K 41/00, A61K 45/06, A61P 13/12, A61K 47/50, A61K 31/00, A61K 31/195, A61K 51/04, A61K 51/08

(54) **PARA-AMINOHIPPURIC ACID (PAH) AS A RENAL PROTECTIVE SUBSTANCE**
PARA-AMINOHIPPURIC-SÄURE (PAH) ALS NIERENSCHUTZSUBSTANZ
ACIDE PARA-AMINOHIPPURIQUE (PAH) EN TANT QUE SUBSTANCE PROTECTRICE RÉNALE

(30) Priority: 08.05.2019 WO PCT/EP2019/061882
(43) Date of publication of application: 16.03.2022
(73) Proprietor: ITM Isotope Technologies Munich SE, 85748 Garching bei München (DE)
(72) Inventor: MECKEL, Marian, 85748 Garching bei München (DE); OSL, Theresa, 85748 Garching bei München (DE); ZHERNOSEKOV, Konstantin, 85748 Garching bei München (DE)
(74) Representative: Graf von Stosch Patentanwaltsgesellschaft mbH
(86) International application number: PCT/EP2020/062950
(87) International publication number: WO 2020/225447

(56) References cited:
- NATOCHIN Y V ET AL: "Cisplatin: Nephrotoxic action in vertebrates and its prevention", COMPARATIVE BIOCHEMISTRY AND PHYSIOLOGY. C. COMPARATIVEPHARMACOLOGY, PERGAMON PRESS , OXFORD, GB, vol. 94, no. 1, 1 January 1989 (1989-01-01), pages 115-120, XP023865230, ISSN: 0306-4492, DOI: 10.1016/0742-8413(89)90153-9 [retrieved on 1989-01-01]
- BASINGER M A ET AL: "Dithiocarbamate-induced biliary platinum excretion and the control of cis-platinum nephrotoxicity", TOXICOLOGY AND APPLIED PHARMACOLOGY, ACADEMIC PRESS, AMSTERDAM, NL, vol. 97, no. 2, 1 February 1989 (1989-02-01), pages 279-288, XP024885697, ISSN: 0041-008X, DOI: 10.1016/0041-008X(89)90333-5 [retrieved on 1989-02-01]

## Description

The present invention relates to para-aminohippuric acid (PAH) or a pharmaceutically acceptable salt thereof for use in a method for the reduction of nephrotoxic side effects of radiolabeled therapeutic and diagnostic (e.g. for imaging purposes) compounds in a subject.

It also relates to a pharmaceutical composition for kidney protection during imaging or therapy using radiolabeled compounds, wherein the composition comprises a radiolabeled pharmaceutical compound in combination with para-aminohippuric acid (PAH) or a pharmaceutically acceptable salt thereof, and a pharmaceutically acceptable excipient, diluent, carrier or a combination thereof. The present application also relates to PAH or a pharmaceutically acceptable salt thereof a method for the reduction of nephrotoxic side effects of radiolabeled therapeutic and diagnostic compounds in a subject, the method comprising administering para-aminohippuric acid (PAH) or a pharmaceutically acceptable salt thereof in combination with a radiolabeled therapeutic or diagnostic compound, wherein the administration of PAH is prior and/or during and/or after administration of the radiolabeled diagnostic compound, and a method comprising administering a pharmaceutical composition according to the present invention to a subject during imaging or therapy using a radiolabeled compound.

In particular, the present application relates to the use of PAH or a pharmaceutically acceptable salt thereof for inhibition of renal uptake and improvement of the biodistribution of radiolabeled molecules *in vivo* that are potentially damaging the kidney, especially for therapeutic radiopharmaceuticals, and/or to improve contrast in case of diagnostic radiopharmaceuticals by using para-aminohippuric acid (PAH) or a salt thereof (e.g. aminohippurate sodium).

In the context of advanced medical treatment, patients are exposed to an expanding variety of drugs for diagnostic and therapeutic purposes. Some of these agents cause adverse drug effects linked with systemic toxicity, including impairment of renal function. Most of these compounds are cleared through the kidneys and reabsorbed and partially retained in the proximal tubules, causing dose-limiting nephrotoxicity. Nephrotoxicity results in serious clinical syndromes, including acute kidney injury (AKI). Nephrotoxic agents have been implicated as etiologic factors in 17-26% of in-hospital AKI. Drug-induced renal impairment involves various classes of drugs and includes prescription agents as well as commonly encountered over-the-counter drugs.

Toxicity of therapeutic and potentially diagnostic agents may be inherent to the pharmacological compound itself, and the potential for toxicity may be heightened in the kidney microenvironment. For example, the aim of chemotherapy is to kill malignant cells via various mechanisms aimed at arresting cellular division. Since the cell cycle operates normally in non-malignant cells, healthy tissues, including renal parenchymal cells, are also affected. The kidney as filtration organ is especially exposed as a target for toxic compounds. Since it receives a significant percentage of cardiac output, robust blood flow through the kidney exposes the kidney to drugs and drug metabolites. Some of these agents have the required charge and size for filtration at the glomerulus and subsequently gain entry into renal tubular epithelial cells via pinocytosis or endocytosis. Other drugs are transported via peritubular capillaries and gain access to renal tubular epithelial cells at the basolateral surface, where they are taken up by organic anion and organic cation transporters (OATs and OCTs, respectively) and are effluxed into tubular lumina where they may contribute to clinically significant nephrotoxicity.

Some anti-cancer therapeutics, e.g. cisplatin, are known to be nephrotoxic. Cisplatin (SP-4-2)-diamindichloridoplatinum (II)) is a Platin atom complexed by NH3 and Cl (square planar complex). Its nephrotoxicity is based on its uptake into renal cells and its binding to the cell's DNA, thus inhibiting cellular mechanisms, in particular cellular replication. The nephrotoxicity of cisplatin was described by Natochin et al. (Comp. Biochem. Physiol Vol. 94C, No.1 pp115-120, 1989) disclosing the co-administration of PAH in order to counter nephrotoxic effects to the therapeutic agent cisplatin. Choline chloride, PAH, fursosemide and ethacrynic acid were described to reduce cisplatin's nephrotoxic effects in rats.

Nephrotoxicity is also known as an undesired side effect when adminisetring radionuclide-based therapeutics/diagnostics.

Diagnostic agents are - due to their less frequent administration for a given subject - rarely highly nephrotoxic for that subject. However, such diagnostic agents for imaging purposes (in particular for SPECT or PET imaging purposes) require an advantageous biodistribution and contrast.

In particular, radionuclide labeled peptides, peptidomimetics, antibody fragments, knottings or labeled inhibitors and other compounds undergo renal clearance, uptake and renal retention leading to e.g. a high renal radiation dose and, thereby, to an increased risk of kidney radiotoxicity. Furthermore, fast renal clearance of e.g. radiopharmaceuticals leads to suboptimal biodistribution and low uptake of the agents in target organs. The radionuclide based nephrotoxicity is based on a mechanism fundamentally distinct from the nephrotocixity exerted by other anti-cancer drugs, e.g. cisplatin. Typically, radionuclides evoke oxydative stress for the cells by their radioactivity without entering into the cells.

Renal toxicity is thus a well-known adverse effect of Radio Ligand Therapy (RLT) with e.g. the kidney being the dose limiting organ. In prior art patent publications EP 1196154 B1, EP 0094378, EP 2021012 B1 and US 2016/0143926 A1 it has been described that co-administration of amino acids like lysine and arginine or mixtures thereof with other compounds, like amifostine or gelatin, can reduce the uptake and retention of [¹⁷⁷Lu-DOTA⁰,Tyr3]octreotate, [¹¹⁷Lu-DOTA°-Tyr3]-octreotide and [¹¹¹In-DTPA-D-Phe1]octreotide. The usage of renal protecting agents is highly critical. LUTATHERA, the first ¹⁷⁷Lu-labeled drug approved by the FDA is administered only in combination with an amino acid cocktail for infusion (Receptor-mediated radionuclide therapy with ⁹⁰Y-DOTATOC in association with amino acid infusion: a phase I study; Lisa Bodei et al. Eur J Nucl Med (2003) 30: 207-216; ⁸⁶Y-DOTA⁰)-D-Phe1-Tyr3-octreotide (SMT487) - a phase 1 clinical study: pharmacokinetics, biodistribution and renal protective effect of different regimens of amino acid co-infusion. Jamar F et al., Eur J Nucl Med Mol Imaging. 2003 Apr; 30(4): 510-8.)

The radiation dose absorbed by the kidneys can be reduced by co-infusion of agents that competitively inhibit the reabsorption of the radiolabeled compound, such as positively charged amino acids, Gelofusine, trypsinised albumin or FRALB-C (bovine serum albumin fragmented by cyanogen bromide) (Albumin derived peptides efficiently reduce renal uptake of radiolabeled peptides, Vegt E et al., Eur J Nucl Med Mol Imaging (2010) 37: 226). Others claim to decrease the kidney uptake much more significantly by using lysine/arginine solutions in combination with compounds, like amifostine and gelatin (EP 1196154 B1, EP 2021012 B1). To reduce the renal reabsorption of radiolabeled peptides more efficiently, a combination of two or more competitive inhibitors of endocytosis and other approaches have been envisaged.

Another kidney protective approach is the application of radiolabeled PSMA inhibitors with co-medication of structurally related PSMA binding molecules, such as 2-(phosphonomethyl)pentanedioic acid (PMPA), which improves the kidney-to-tumor ratio. That concept is based on the compound-specific kinetics for their uptake in kidney and tumors, respectively (PMPA for nephroprotection in PSMA-targeted radionuclide therapy of prostate cancer, Kratochwil et al., JNM 2015 Feb; 56(2): 293; US 2018/207299).

The mechanism of action of currently developed and clinically utilized kidney protective co-medication, such as amino acids solution, or PMPA, is based on the accelerated clearance of the radiopharmaceuticals from the kidney. Their similarity - in terms of their binding properties - with radiopharmaceuticals is used. Therefore, clinical use of those protective agents must be combined with radiopharmaceuticals exhibiting similar binding properties in the kidney. Furthermore, infusion of amino acids as kidney radio protectants may result in clinically disadvantageous side effects. Vomiting and nausea caused by large volume infusions of non-isotonic amino acid solutions are frequently observed. A severe life threatening side effect of such infusions, reported in the literature is hyperkalemia (Effect of amino acid infusion on potassium serum levels in neuroendocrine tumor patients treated with targeted radiopeptide therapy. Giovacchini G et al., Eur J Nucl Med Mol Imaging. 2011 Sep; 38(9): 1675-82). Therefore the amount of amino acids for administration is usually limited to 25 g of lysine and 25 g of arginine. Kidney uptake reduction is reported by Rolleman EJ et al. as follows: "(1) commercially available amino acid solution (AA) (21%+/-14%, P<0.02), (2) by 25 g (17%+/-9%, P<0.04), 50 g (15%+/-13%, P<0.04) or 75 g of lysine (44%+/-11%, P<0.001) and (3) by a combination of 25 g of lysine plus 25 g of arginine (LysArg) (33%+/- 23%, P<0.01). Fluid infusion alone (500, 1,000 or 2,000 ml of saline/glucose) did not change renal uptake of radioactivity. In patients studied with 75 g of lysine (Lys75) and LysArg, serum potassium levels rose significantly" (Safe and effective inhibition of renal uptake of radiolabeled octreotide by a combination of lysine and arginine. Rolleman EJ et al., Eur J Nucl Med Mol Imaging. 2003 Jan; 30(1): 9-15). Hyperkalemia is still an unsolved issue of that kidney protection approach, as high concentrations of lysine and arginine are applied as part of the mixture (Giovacchini G et al., supra).

Lysine and arginine are reabsorbed by sodium independent amino acid transporters (SCL3A1) in the proximal tubulus. The clearance mechanism of radiopharmaceuticals, like [¹⁷⁷Lu-DOTA°-Tyr3]-octreotide, has not been fully elucidated. The published data indicate that the SCL3A1 amino acid transporter may play a role in the clearance of radiopharmaceuticals, but it is obviously known that this observation does not represent the major, let alone the only mechanism involved.

It is therefore the object of the present invention to provide an innovative approach of inhibiting renal uptake to be used as a co-therapy for a broader spectrum of (radio)pharmaceuticals, irrespective of the underlying mechanism of their renal accumulation, thereby reducing the nephrotoxic side effects of (radio)pharmaceuticals as therapeutic or, more rarely, diagnostic compounds. However, it is highly desired for diagnostics in medical imaging to improve their biodistribution in vivo and enhance contrast by increasing their take at the site to be imaged, e.g. uptake by whatever tissue.

Those objects are solved by the subject-matter as defined by the claims.

The present invention is based on the finding that para-aminohippuric acid (PAH) or a pharmaceutically acceptable salt thereof, can be suitably used to e.g. improve the biodistribution of therapeutic and diagnostic compounds comprising radionuclides. The clearance of the agents, for example chemotherapeutic agents or radiopharmaceuticals, such as [¹⁷⁷Lu-DOTA°-Tyr3]-octreotide, ¹⁷⁷Lu PSMA-inhibitors or others, that are potentially damaging for the kidneys, in a subject may be modified by the innovative approach according to the invention. Moreover, it has been found that PAH administration can be used to down-regulate renal drug resorption. Thereby, serum levels of the drug may be (but not necessarily) increased thus leading to enhanced bioavailability of the drug.

The invention is thus particularly suitable for inhibiting the renal uptake of all sorts of proteinaceous molecules, such as proteins, and peptides or their fragments or antibody fragments which exhibit inherently nephrotoxicity. That holds even more so, whatever such proteinaceous molecules are conjugated to a toxin, a radionuclide, a cytostatic agent or other potentially cell-toxic agents. In particular, it was an unexpected finding that the nephrotoxicity exerted by radiopharmaceuticals due to their radioactivity was observed to be reduced by the inventive co-administration with PAH as well. That finding was even more surprising, as the underlying mechanism of radionuclide based therapeutics/diagnostics is unique (radioactivity) and distinctdistinct from other mechanisms as e.g. observed for anti-cancer drugs, like cisplatin.

In addition, the present invention allows imaging agents (in particular for SPECT and PET purposes) to accumulate in the tissues to be identified (e.g. at the tumor target sites) and reduces thus its presence in off-target tissues. Thereby, the present invention allows to improve the image contrast for a given e.g. conjugate molecule containing a radionuclide, as its clearance by the kidneys is reduced.

### Para-aminohippuric acid (PAH)

Aminohippuric acid or *para*-aminohippuric acid (PAH), a derivative of hippuric acid, is an amide derivative of the (a) amino acid glycine and (b) *para*-aminobenzoic acid that is not naturally found in humans. They are covalently linked by an amide bond. PAH's sodium salt, aminohippurate sodium, is a diagnostic agent which is widely used in diagnostic testing of the kidney function, in particular for measuring renal plasma flow. In the following, aminohippuric acid, *para*-aminohippuric acid and aminohippurate salt(in particular, alkali or earthalkali salt), in particular sodium salt, are synonymously used and referred to as "PAH".

### para-aminohippuric acid (PAH):

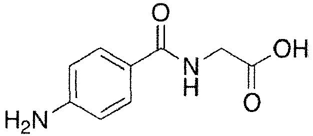

### aminohippurate sodium:

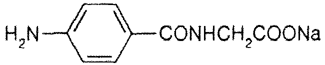

Typically, PAH is provided as a sterile, non-preserved 20 % aqueous solution for injection. PAH is filtered by the glomeruli and is actively secreted by the proximal tubules. At low plasma concentrations (1.0 to 2.0 mg/100 mL), an average of 90 percent of PAH is cleared by the subject's kidneys from the renal blood stream by a single circulation. PAH is also used to measure the functional capacity of the renal tubular secretory mechanism or transport maximum (Tm PAH). This is accomplished by elevating the plasma concentration to levels (40-60 mg/100 mL) sufficient to saturate the maximum capacity of the tubular cells to secrete PAH. PAH does essentially not exhibit any side effects and is of negligible toxicity (the intravenous LD 50 in female mice is 7.22 g/kg). Phenomena like vomiting and nausea or hyperkalemia are not or, if at all, rarely reported only.

In contrast to the established prior art co-medication (e.g. by using amino acid mixtures), para-aminohippuric acid (aminohippurate) allows to unspecifically modulate the clearance of (radio)pharmaceuticals, as it is a substrate and/or a functional inhibitor for various transporters. PAH is actively secreted by transporters into the urine. As of today, PAH is known as a substrate or an inhibitor of 14 different transporters, including organic cation transmembrane transporters (e.g. OCT1, OCT1A, OCT2, OCT3, OCTN1, OCTN2, OCTN3), and organic anion transmembrane transporters (e.g. OAT1, OAT2, OAT3, OAT4, OAT5, OATP, URAT1). Substrates of PAH are also hMRPs, ATP-dependent efflux transporters.

Therefore, para-aminohippuric acid or a pharmaceutically acceptable salt thereof may be employed in co-medication with a broad spectrum of radiolabeled therapeutic and diagnostic compounds, irrespective of the nature of carrier molecule (i.e. peptide, antibody fragment, peptidomimetic, small molecule, etc.). The modulation of renal clearance by PAH was identified to lead to prolongation of the administered drug's blood circulation and to its increased bioavailability. Aminohippuric acid or its salt can be administered prior and/or during and/or after the administration of the (radio)pharmaceuticals to be cleared by the kidneys, e.g. by infusion or injection.

### General comments

It is to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" e.g., a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The term "about" in relation to a numerical value x means x ± 10%.

The term "subject" as used herein generally includes humans and non-human animals and preferably mammals (e.g., non-human primates, including marmosets, tamarins, spider monkeys, owl monkeys, vervet monkeys, squirrel monkeys, and baboons, macaques, chimpanzees, orangutans, gorillas; cows; horses; sheep; pigs; chicken; cats; dogs; mice; rat; rabbits; guinea pigs etc.), including chimeric and transgenic animals and disease models. In the context of the present invention, the term "subject" preferably refers a non-human primate or a human, most preferably a human.

Any references in the description to methods of treatment refer to the compounds, pharmaceutical compositions and medicaments of the present invention for use in a method for treatment of the human (or animal) body by therapy.

In a first aspect, the present invention relates to para-aminohippuric acid (PAH) or a pharmaceutically acceptable salt thereof for use in a method for the reduction of undesired nephrotoxic properties of radiolabeled therapeutic and diagnostic compounds in a subject to be treated with such compounds as a radiopharmaceutical in in radio ligand therapy or diagnosis as defined by claim 1.

As mentioned above, PAH or a salt thereof is a substrate and/or an inhibitor of various transporters in the kidney. Therefore, it can be utilized with a broad spectrum of radiolabeled therapeutic and diagnostic compounds which are expected to enter into renal tubular cells, e.g. via uptake through organic anionic transporters (OATs) and organic cationic transporters (OCTs), thus exerting their potential nephrotoxicity. It can also be utilized to protect the renal cells from radioactivity of radiolabelled therapeutics or diagnostics, e.g. when evoking oxydative stress due to the radioactivity. Also the protection of nephrotoxicity may specifically imply protection from glomerulotoxicity.

Examples for therapeutic nephrotoxic compounds include, without implying any limitation, non-steroidal anti-inflammatory drugs which are widely used to relieve pain and signs of inflammation. Still, they can trigger a larger variety of renal complications, such as prerenal azotemia, acute tubular necrosis, acute papillary necrosis, acute interstitial nephritis, chronic tubulointerstitial nephritis (analgesic nephropathy) minimal change disease, membranous nephropathy, hyperkalemia and metabolic acidosis (hyporeninemic hypoaldosteronism, hyponatremia, hypertension); angiotensin-converting enzyme inhibitors and angiotensin II receptor blockers which are used in the treatment of hypertension and congestive heart failure and for delaying the progression of diabetic nephropathy and which can result in a higher risk for acute kidney injury (AKI) and hyperkalemia; antimicrobial agents, such as neomycin, gentamycin, tobramycin, amikacin and streptomycin, which are commonly used in the treatment of Gram-negative bacterial infections and whose intracellular accumulation results either in tubular cell death or in functional alteration of cell membrane transporters leading to electrolyte abnormalities (hypokalemia, hypomagnesemia, and hypocalcemia); sulfa-based antibiotics, such as sulfamethoxazole-trimethoprim, which can result in hyperkalemia by inhibiting the epithelium sodium channel at the distal convoluted tubule, which provides the driving force for potassium excretion, and sulfadiazine which can cause acute interstitial nephritis and crystal nephropathy; glycopeptide antibiotics, such as Vanomycin, which can result in nephrotoxicity due to acute tubular necrosis; fluoroquinolone antibiotics, such as Ciprofloxacin, which can cause acute interstitial nephritis and crystalluria; other antibiotics, such penicillins and cephalosporins, which cause acute interstitial nephritis and acute tubular necrosis; and polymyxins, such as colistin and polymyxin B, which cause acute interstitial nephritis by toxic tubular injury; antiviral agents, such as Acyclovir, which can induce acute kidney injury secondary to crystal precipitation in the renal tubules, Fosarnet, which can lead to acute tubular necrosis, acute kidney injury, electrolyte abnormalities, such as hypocalcemia, hypomagnesimia, hypokalemia and hypo or hyperphosphatemia; antiretroviral drugs, such as tenofovir, which is toxic to tubular cells leading to acute kidney injury with or without proximal tubulopathy which may result in chronic kidney disease, and protease inhibitors, such as indinavir, abacavir, ritonavir and atazanavir, which can crystallize in renal tubules resulting in crystal-related kidney injury and nephrolithiasis; antifungal agents, such as Amphotericin B, which may cause acute tubular necrosis and tubular dysfunction which manifests as renal tubular acidosis, urinary concentration defects and electrolyte disturbances; immunosuppressive agents, such as calcineurin inhibitors (e.g. Tacrolismus, cyclosporine), which are in particular used in immunosuppressive therapy after solid organ transplantation, may cause acute kidney injury; Lithium, which is the mainstay of treatment for patients with bipolar disorder and which can result in various forms of nephrotoxicity, such as nephrogene diabetes insipidus (NDI) chronic kidney disease and chronic tubulointestinal. Further therapeutic agents leading to acute kidney injury are proton-pump inhibitors; acetaminophen; HMG-CoA reductase inhibitors; and osmotic agents.

Therapeutic nephrotoxic compounds also include chemotherapeutic agents which play a central role in the treatment of various neoplasms and can result in a wide spectrum of renal complications, such as renal syndromes associated with acute kidney injury or chronic kidney disease, and renal syndromes associated with electrolyte disorders. Examples of such chemotherapeutic agents include Cisplatin (acute tubular necrosis, proximal tubulopathies, hypernatremia, hypomagnesimia, hypocalcemia, distal renal tubular acidosis, thrombotic microangiopathy), Pemetrexed (acute tubular necrosis), Streptozocin (also referred to as Streptozotocin) (acute tubular necrosis, proximal tubulopathies), Mithramycin (acute tubular necrosis), Zoledronate (acute tubular necrosis), Interferon (acute intestinal nephritis), Allopurinol (acute interstitial nephritis), Gemcitabine (thrombotic microangiopathy), Mitomycin C (thrombotic microangiopathy), Anti-angiogenic agents (thrombotic microangiopathy) Methotrexate (crystal nephropathy) Ifosfamide (proximal tubulopathies, hypernatremia), Cyclophosphamide (hyponatremia), Vincristine (hyponatremia), Cetuximab (hypomagnesemia), Methotrexate (acute kidney injury).

For example, cisplatin and Ifosfamide, which are standard components in the treatment regimens for various solid organ tumors, including those affecting children, are known to undergo cellular uptake at the proximal tubule via organic cationic transporters (OCT2) (Shirali A, Perazella M, Advances in Chronic Kidney Disease, Vol 21, No 1 (January), 2014: pp56-63). Therefore, it is assumed that co-injection with PAH can effectively reduce the nephrotoxic side effects of these chemotherapeutics.

Further nephrotoxic compounds include radiocontrast agents, such as iodinated radiocontrast agents, which are necessary for several diagnostic and interventional radiology procedures and which can result in contrast-induced nephropathy (CIN) and acute kidney injury. Examples for iodinated ionic contrast agents are diatrizoate (Hypaque 50), metrizoate (Isopaque 370), iothalamate (Conray), ioxaglate (Hexabrix); non-ionic contrast agents include iopamidol (Isovue 370), iohexol (Omnipaque 350), ioxilan (Oxilan 350), iopromide (Ultravist 370), iodixanol (Visipaque 320), ioversol.

According to the present invention, PAH is used to reduce the nephrotoxic side effects of radiopharmaceuticals which are used as diagnostic and therapeutic agents.

Radiopharmaceuticals may comprise nonmetallic (organic) radionuclides (¹⁸F, ¹¹C, ¹³N, ¹⁵O, ¹²⁴I, etc.) or radiometals (e.g. ⁹⁰Y, ^{99m}Tc, ¹¹¹In, ¹³¹I, ⁶⁷Ga, ⁶⁸Ga, ⁶⁴Cu, ¹⁶¹Tb, ²²⁵Ac, ⁴⁴Sc, ⁴⁷Sc, ⁶⁷Cu, ⁸⁹Zr, ¹⁷⁷Lu, etc.). Although some radiometals can target a particular tissue as a metal salt or as a metal complex, it is mostly required to conjugate the radionuclide/radiometal with a targeting biomolecule so that the radionuclide is delivered to the target site, e.g. the tumor tissue in a targeted manner. The biomolecules can e.g. be small organic molecules, peptides, monoclonal antibodies (mAbs) or mAbs fragments. They serve as the vehicle ("carrier") to carry the radionuclide to the target tissue.

Commercially available small complex radiopharmaceuticals include for example ^{99m}Tc-Sestamibi (Cardiolite^{®}), which is used for myocardial perfusion imaging; ^{99m}Tc-Tetrofosmin (Myoview^{®}), which is used for myocardial perfusion imaging; ^{99m}Tc-Pentetate (DTPA) (Technescan^{®}), which is used for renal imaging and function studies; ^{99m}Tc-Bicisate (ECD) (Neurolite^{®}), which is used for cerebral perfusion imaging; ^{99m}Tc-MDP (Medronate^{®}), which is used for skeletal scintigraphy; ^{99m}Tc-Teboroxime (Cardiotec^{®}), which is used for myocardial perfusion imaging; ¹¹¹In-Oxyquinoline (Indium-111 oxine^{®}), which is used for leukocyte scintigraphy; ¹¹¹In-Pentetate (Indium-111 DTPA^{®}), which is used for imaging of CSF kinetics; ¹⁵³Sm-EDTMP (Quadramet^{®}), which is used for the treatment of bone pain (therapy); ¹⁸⁸Re-HEDP, which is used in metastatic bone pain therapy.

According to the invention, the radionuclide is a tri-valent radionuclide.

The most elegant approach according to the invention to establish a stable conjugation of a radionuclide and a targeting biomolecule (carrier) is to use a suitable bifunctional chelator or chelating agent which binds or coordinates the radionuclide tightly and, at the same time, presents functional moieties for its conjugation with the biomolecule.

Examples for commercially available peptido- or immuno-conjugates include e.g. ^{99m}Tc-Depreotide (Neo Tect^{®}) which is used to evaluate certain lung lesions; ^{99m}Tc-Arcitumumab (CEA-Scan^{®99m}Tc-mAb) which is used for colorectal cancer imaging; ¹¹¹In-Capromab pendetide (ProstaScint^{®}) which is used in prostate cancer imaging; ¹¹¹In-Pentetreotide (Octreoscan^{®}) which is used in neuroendocrine tumor imaging; ¹¹¹In-Imciromab pentetate (MyoScint^{®}) which is used in imaging of chest pain, suspected to be caused by myocardial infarction; ¹¹¹In-Satumomab pendetide (OncoScint^{®}) which is used in imaging of metastatic disease associated with colorectal and ovarian cancer; ⁹⁰Y-Ibritumomab tiuxetan (Zevalin^{®}) which is used in treatment of non-Hodgkin's lymphoma (NHL); ⁶⁸Ga-Edotreotide or ⁶⁸Ga-DOTATE for imaging of neuroendocrine tumors; ¹⁷⁷Lu-B DOTATETE (Lutathera) for therapy of neuroendocrine tumors.

Thus, according to the present invention, PAH is used for the reduction of nephrotoxic side effects of radiopharmaceuticals based on a tri-valent radionuclide, as for example mentioned above, in radio chemical/ligand therapy and/or diagnosis.

In radioligand therapies (also known as PRRT - peptide-receptor radionuclide therapy) radiopharmaceuticals are labeled by a radioligand, which specifically binds to a (tumor) cell target, e.g. a tumor cell surface protein or marker. After binding of the compound to the tumor target, for example to a receptor, the radionuclide releases energetic beta particle radiation to precisely target cells at the targeted site.

In radioligand diagnosis, the radiolabeled compound binds to the (tumor) target cell, e.g. a receptor. The decay of radioactive isotope can be measured by positron emission tomography (PET) or single photon emission computed tomography (SPECT).

Therefore, PAH or a pharmaceutically acceptable salt thereof is used for the reduction of nephrotoxic side effects of a pharmaceutical which is a radiolabelled ("radiopharmaceutical"). The radiopharmaceutical isa conjugate molecule which comprises (i) a carrier molecule which binds to the (tumor) cell target structure (e.g. a receptor or an antigen), a (ii) chelating agent (or chelator) and (iii) a radionuclide. The chelating agent typically coordinates the radionuclide, thus forming a radiolabeled complex, which is conjugated to the carrier molecule.

### Radionuclide

The term "radionuclide" (or "radioisotope") refers to isotopes of natural or artificial origin with an unstable neutron to proton ratio that disintegrates with the emission of corpuscular (i.e. proton (alpha-radiation) or electron (beta-radiation)) or electromagnetic radiation (gamma-radiation)). In other words, radionuclides undergo radioactive decay. In the radiolabeled complex of the radiopharmaceutical, any known radionuclide may be complexed by the chelating agent. Such radionuclides may include, without limitation, ¹⁸F, ¹³¹I, ⁹⁴Tc, ^{99m}Tc, ⁹⁰In, ¹¹¹In, ⁶⁷Ga, ⁶⁸Ga, ⁸⁶Y, ⁹⁰Y, ¹⁷⁷Lu, ¹⁶¹Tb, ¹⁸⁶Re, ¹⁸⁸Re, ⁶⁴Cu, ⁶⁷Cu, ⁵⁵Co, ⁵⁷Co, ⁴³Sc, ⁴⁴Sc, ⁴⁷Sc, ²²⁵Ac, ²¹³Bi, ²¹²Bi, ²¹²Pb, ²²⁷Th, ¹⁵³Sm, ¹⁶⁶Ho, ¹⁵²Gd, ¹⁵³Gd, ¹⁵⁷Gd, or ¹⁶⁶Dy, in particular ⁶⁸Ga, ¹⁷⁷Lu or ^{99m}Tc. According to the invention, ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, ²²⁵Ac, ²²⁷Th, ¹⁵³Sm, ¹⁶⁶Ho, ¹⁵²Gd, ¹⁵³Gd, ¹⁵⁷Gd, or ¹⁶⁶Dy, in particular ⁶⁸Ga, or ¹⁷⁷Lu may be used.

The choice of suitable radionuclides depends *inter alia* on the chemical structure and chelating capability of the chelating agent, and, most prominently, on the intended application of the resulting (complexed) conjugate molecule (e.g. diagnostic vs. therapeutic and e.g. on the disease to be treated). For instance, the beta-emitters such as ⁹⁰Y, ¹³¹I, ¹⁶¹Tb and ¹⁷⁷Lu may be used for concurrent systemic radionuclide therapy. Providing DOTA, DOTAGA or DOTAM as a chelator may advantageously enable the use of either ⁶⁸Ga, ^{43,44,47}Sc, ¹⁷⁷Lu, ¹⁶¹Tb, ²²⁵Ac, ²¹³Bi, ²¹²Bi, ²¹²Pb as radionuclides. According to the invention, ⁶⁸Ga, ^{43,44,47}Sc, ¹⁷⁷Lu, ¹⁶¹Tb, or ²²⁵Ac, may be used.

According to the invention, a tri-valent radionuclide is employed.

In some preferred embodiments, the radionuclide may be ¹⁷⁷Lu. In some preferred embodiments, the radionuclide may be ¹¹In. In some preferred embodiments, the radionuclide may be ⁹⁰Y. In some preferred embodiments, the radionuclide may be ⁶⁸Ga.

### Chelator

As mentioned above, the carrier molecule, e.g. a compound targeting the (cancer) cell, is linked to the chelating agent or chelator coordinating the radionuclide.

The terms "chelator" or "chelating agent" are used interchangeably herein. They refer to polydentate (multiple bonded) ligands capable of forming two or more separate coordinate bonds with ("coordinating") a central (metal) ion. Specifically, such molecules or molecules sharing one electron pair may also be referred to as "Lewis bases". The central (metal) ion is usually coordinated by two or more electron pairs to the chelating agent. The terms, "bidentate chelating agent", "tridentate chelating agent", and "tetradentate chelating agent" are art-recognized and refer to chelating agents having, respectively, two, three, and four electron pairs readily available for simultaneous donation to a metal ion coordinated by the chelating agent. Usually, the electron pairs of a chelating agent forms coordinate bonds with a single central (metal) ion; however, in certain examples, a chelating agent may form coordinate bonds with more than one metal ion, with a variety of binding modes being possible.

The terms "coordinating" and "coordination" refer to an interaction in which one multi-electron pair donor coordinatively bonds (is "coordinated") to, i.e. shares two or more unshared pairs of electrons with, preferably one central (metal) ion.

The chelator or chelating agent is preferably a macrocyclic bifunctional chelator having a metal chelating group at one end and a reactive functional group at the other end, which is capable to bind to other moieties, e.g. peptides. Preferably, the chelator may be selected such that the chelator forms a square bi-pyramidal complex for complexing the radionuclide. In another embodiment, the chelator does not form a planar or a square planar complex.

The chelating agent is preferably chosen based on its ability to coordinate the desired central (metal) ion, usually a radionuclide as specified herein.

Accordingly, the chelator may be characterized by one of the following Formulas (4a)-(4kk):

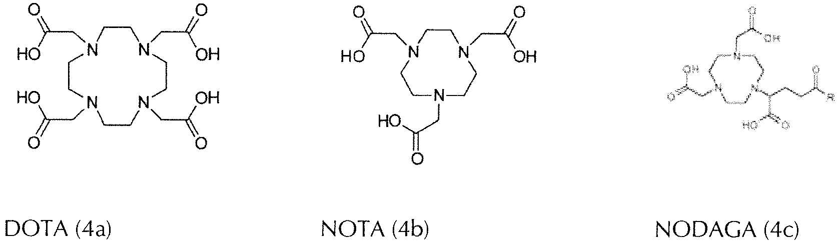

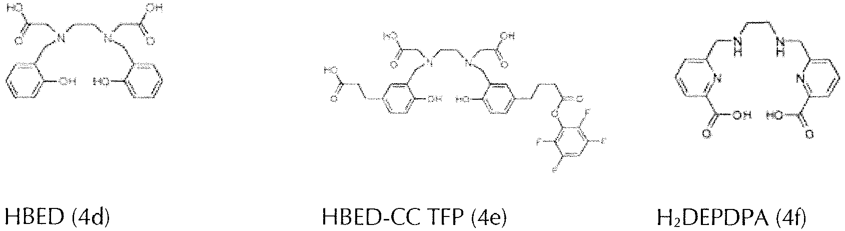

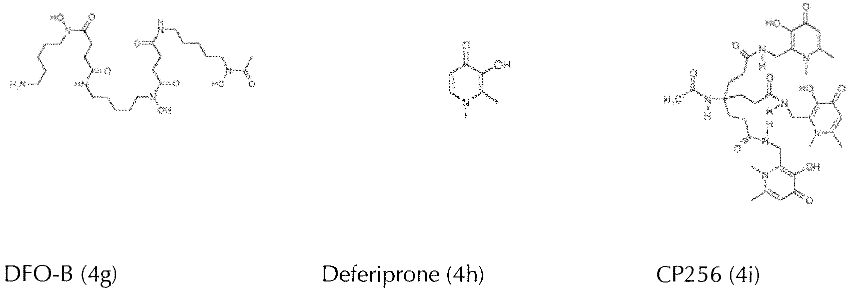

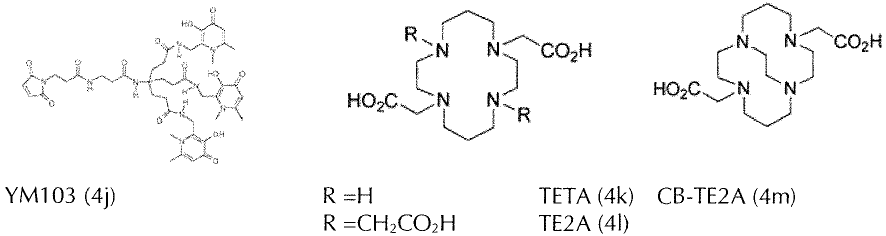

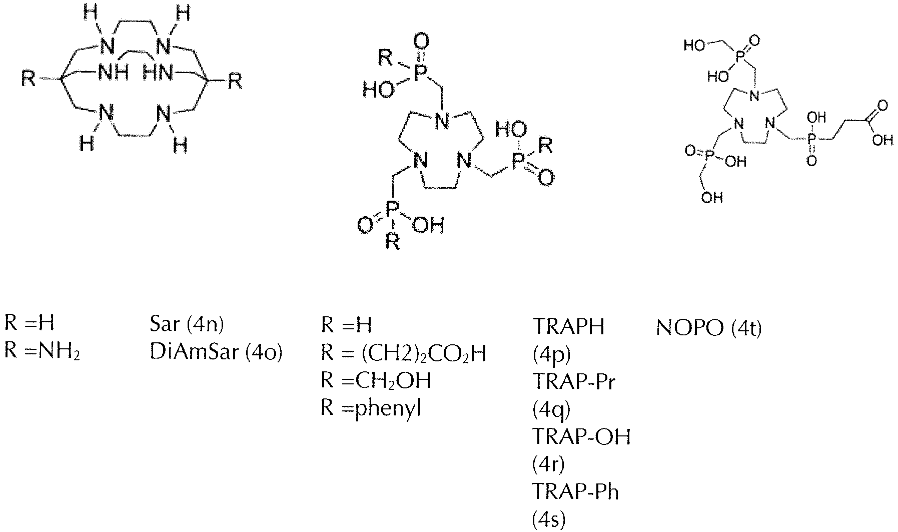

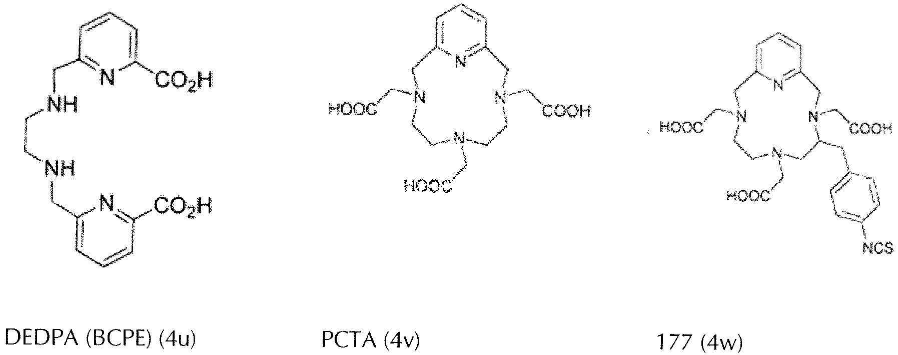

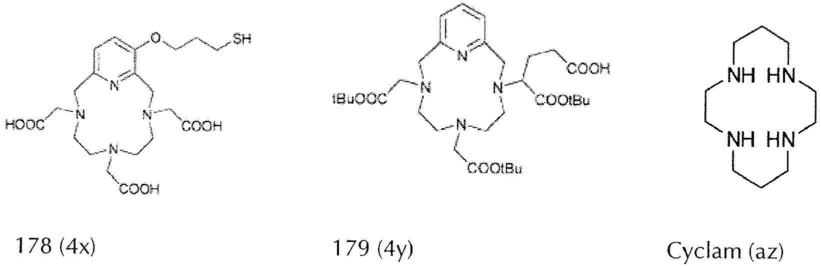

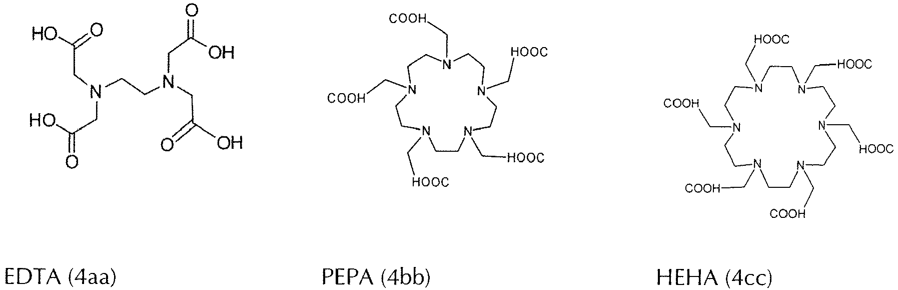

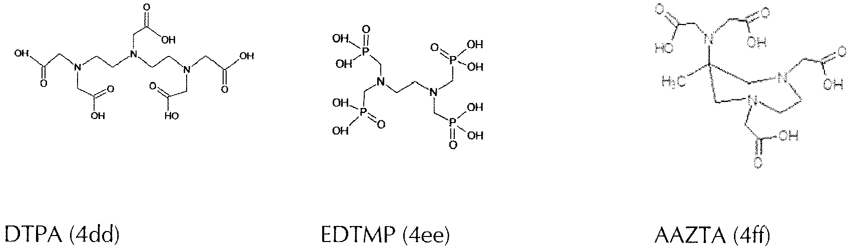

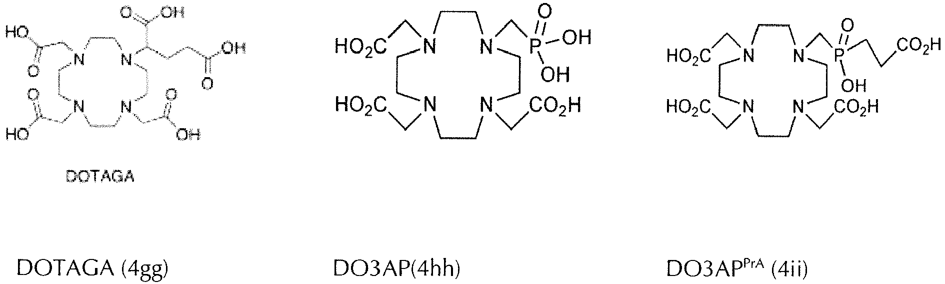

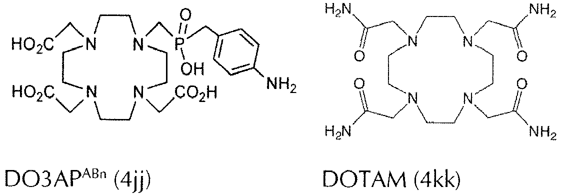

Preferably, the chelator may be DOTA (1,4,7,10-tetraazacyclododecane-1,4,7,10-tetraacetic acid, which may be characterized by Formula (4a)), HBED-CC (N,N"-bis[2-hydroxy-5-(carboxyethyl)benzyl]ethylenediamine-N,N"-diacetic acid, which may be characterized by Formula (4e)), DOTAGA (2-[1,4,7,10-Tetraazacyclododecane-4,7,10-tris(acetate)]-pentanedioic acid) (Formula (4gg), DOTAM (1,4,7,10-Tetrakis(carbamoylmethyl)-1,4,7,10-tetraazacyclododecane) (Formula (4kk)) or derivatives thereof.

Advantageously, DOTA effectively forms complexes with diagnostic (e.g. ⁶⁸Ga) and therapeutic (e.g. ⁹⁰Y or ¹⁷⁷Lu) radionuclides and thus enables the use of the same conjugate for both imaging (diagnostic) and therapeutic purposes, i.e. as a theragnostic agent. DOTA derivatives capable of complexing Scandium radionuclides (⁴³Sc, ⁴⁴Sc, ⁴⁷Sc), including DO3AP (which may be characterized by Formula (4hh)), DO3AP^{PrA} (which may be characterized by Formula (4ii)), or DO3AP^{ABn} (which may be characterized by Formula (4jj)) may also be preferred and are described in Kerdjoudj et al. (Dalton Trans., 2016, 45, 1398-1409).

Advantageously, HBED-CC effectively forms complexes with diagnostic radionuclides (e.g. ⁶⁸Ga, ^{99m}Tc).

Other preferred chelators in the context of the present invention include, (2-(4,7-bis(carboxymethyl)-1,4,7-triazonan-1-yl)-pentanedioic acid (NODAGA)1,4,7-triazacyclononane-1,4,7-triacetic acid (NOTA), 2-(4,7,10-tris(carboxymethyl)-1,4,7,10-tetra-azacyclododecan-1-yl)-pentanedioic acid (DOTAGA), 1,4,7-triazacyclononane phosphinic acid (TRAP), 1,4,7-triazacydo-nonane-1-[methyl(2-carboxyethyl)-phosphinic acid]-4,7-bis-[methyl(2-hydroxymethyl)-phosphinic acid] (NOPO),3,6,9,15-tetra-azabicyclo[9,3,1]-pentadeca-1(15),11,13-triene-3,6,9-triacetic acid (PCTA), N'-{5-[Acetyl(hydroxy)amino]-pentyl}-N-[5-({4-[(5-aminopentyl)(hydroxy)amino]-4-oxobutanoyl}-amino)pentyl]-N-hydroxysuccinamide (DFO), diethylene-triaminepentaacetic acid (DTPA), and hydrazinonicotinamide (HYNIC).

The chelator group, for example the DOTA group, may be preferably complexed with a central (metal) ion, in particular a radionuclide as defined herein. Alternatively, the chelator group, for example DOTA, may not be complexed with a central (metal) ion, in particular a radionuclide as defined herein, and may thus be present in uncomplexed form. Should the chelator (e.g. DOTA) not be complexed with said metal ion, the carboxylic acid groups of the chelator can be in the form of a free acid, or in the form of a salt.

It is within the skill and knowledge of the skilled person in the art to select suitable combinations of the chelator and radionuclides. For instance, in some preferred embodiments, the chelator may be DOTA and the radionuclide may be ¹⁷⁷Lu. In other preferred embodiments, the chelator may be DOTA and the radionuclide may be ⁶⁸Ga. In other preferred embodiments, the chelator may be HYNIC and the radionuclide may be ^{99m}Tc.

### Carrier molecule

The carrier molecule may be any molecule which binds to the (tumor) cell target, e.g. a receptor or another cell (surface) molecule. In a preferred embodiment of the present invention, the carrier molecule is selected from a peptide, a peptidomimetic, an antibody fragment, an antibody mimetic, small molecules, and knottings.

The cell target may be any which is present in or, preferably, on the target cells to which the radionuclide conjugate molecule is intended to bind for radiotherapy or diagnostic.

For example, the carrier molecule may be directed to a receptor or a cell surface molecule present on a disease cell, e.g. a tumor cell. In the following, examples of receptors and cell surface molecules present on tumor cells, which may be a target structure for the carrier molecule, are described in detail. However, the target structures are not limited to the receptors and cell surface molecules described below. Further receptors and cell surface molecules present on cancer or other disease cells are contemplated as target structures for the carrier molecules. Moreover, further carrier molecules targeting the receptors and cell surface molecules present on cancer or other disease cells are contemplated.

### Somatostatin receptor targeting compounds

According to the invention, PAH or a salt thereof can suitably be used to reduce nephrotoxic side effects of (radio)pharmaceuticals targeting the somatostatin receptor (SSTR).

For the treatment of well to moderately differentiated neuroendocrine tumors (NET), e.g. peptides targeting the somatostatin receptor (SSTR) may be used. In NET, radioligand therapy is well-established and may achieve high rates of long lasting tumor remission and stabilization. Peptides targeting the somatostatin receptor are e.g. somatostatin analogs tyr3-octreotide (D-Phe-c(Cys-Tyr-D-Trp-Lys-Thr-Cys)-Thr(ol)) and tyr3-octeotrate (D-Phe-c(Cys-Tyr-D-Trp-Lys-Thr-Cys)-Thr) (Capello A et al.: Tyr3-octreotide and Tyr3-octreotate radiolabeled with 177Lu or 90Y: peptide receptor radionuclide therapy results in vitro, Cancer Biother Radiopharm, 2003 Oct; 18(5): 761-8). Further examples of somatostatin agonists are the peptides octreotide (D-Phe-cyclo(Cys-Phe-D-Trp-Lys-Thr-Cys)Thr(ol)), and NOC (D-Phecyclo(Cys-1-Nal-D-Trp-Lys-Thr-Cys)Thr(ol)).

Others examples of compounds targeting the somatostatin-receptor are somatostatin antagonistic peptides such as JR10 (p-NO₂-Phe-c(D-Cys-Tyr-D-Aph(Cbm)-Lys-Thr-Cys)D-Tyr-NH2); JR11 (Cpa-c(D-Cys-Aph(Hor)-d-Aph(Cbm)-Lys-Thr-Cys)D-Tyr-NH2); BASS (p-NO₂-Phe-cyclo(D-Cys-Tyr-D-Trp-Lys-Thr-Cys)D-Tyr-NH₂; LM3 (p-Cl-Phe-cyclo(D-Cys-Tyr-D-Aph(Cbm)-Lys-Thr-Cys)D-Tyr-NH2.

In a particular preferred embodiment of the present invention, PAH is used to reduce the nephrotoxic side effects of (radio)pharmaceuticals based on somatostatin analogues; examples thereof include: ¹⁷⁷Lu-DOTATOC (¹⁷⁷Lu-DOTA°-[Tyr3]-octreotide) (¹⁷⁷Lu-DOTA-D-Phe-cyclo(Cys-Tyr-D-Trp-Lys-Thr-Cys]-Thr(ol), ¹⁷⁷Lu-DOTANOC (¹⁷⁷Lu-DOTA-D-Phecyclo(Cys-1-Nal-D-Trp-Lys-Thr-Cys)Thr(ol)), ¹⁷⁷Lu-DOTATATE (¹⁷⁷Lu-DOTA-D-Phecyclo(Cys-Tyr-D-Trp-Lys-Thr-Cys)Thr), ⁶⁸Ga-DOTATOC (⁶⁸Ga-DOTA-D-Phe-cyclo(Cys-Tyr-D-Trp-Lys-Thr-Cys)Thr(ol)), ⁶⁸Ga-DOTANOC (⁶⁸Ga-DOTA-D-Phe-cyclo(Cys-1-Nal-D-Trp-Lys-Thr-Cys)Thr(ol)), ⁹⁰Y-DOTATOC (⁹⁰Y-DOTA-D-Phe-cyclo(Cys-Tyr-D-Trp-Lys-Thr-Cys)Thr(ol)), ⁹⁰Y-DOTATATE (⁹⁰Y-DOTA-D-Phe-cyclo(Cys-Tyr-D-Trp-Lys-Thr-Cys)Thr), ¹¹¹In-DTPA-octreotide(¹¹¹In-DTPA-D-Phe-cyclo(Cys-Phe-D-Trp-Lys-Thr-Cys)Thr(ol)).

In another preferred embodiment PAH or a saltthereof is used to reduce the nephrotoxic side effects of (radio)pharmaceuticals based on somatostatin antagonistic compounds; examples include: ¹¹¹In-DOTA-BASS (¹¹¹In-DOTA-p-NO₂-Phe-cyclo-(D-Cys-Tyr-D-Trp-Lys-Thr-Cys)D-Tyr-NH₂, ¹¹¹In-DOTA-JR11 (¹¹¹In-DOTA-Cpa-cyclo[D-Cys-Aph(Hor)-D-Aph(Cbm)-Lys-Thr-Cys]D-Tyr-NH₂), ⁶⁸Ga-DOTA-JR11 (Ga-OpS201) (⁶⁸Ga-DOTA-Cpa-cyclo[D-Cys-Aph(Hor)-D-Aph(Cbm)-Lys-Thr-Cys]D-Tyr-NH₂), ⁶⁸Ga-DODAGA-JR11 (Ga-OPS202) (⁶⁸Ga-NODAGA-Cpa-cyclo[D-Cys-Aph(Hor)-D-Aph(Cbm)-Lys-Thr-Cys]D-Tyr-NH2), ¹⁷⁷Lu-DOTA-JR11 (Lu-OPS201) (¹⁷⁷Lu-DOTA-Cpa-cyclo[D-Cys-Aph(Hor)-D-Aph(Cbm)-Lys-Thr-Cys]D-Tyr-NH₂).

### PSMA-targeting compounds

PAH or a salt thereof can also be suitably used to reduce nephrotoxic side effects of (radio)pharmaceuticals targeting the prostate-specific membrane antigen (PSMA).

Human Prostate-specific membrane antigen (PSMA) (also referred to as glutamate carboxypeptidase II (GCPII), folate hydrolase 1, folypoly-gamma-glutamate carboxypeptidase (FGCP), and N-acetylated-alpha-linked acidic dipeptidase I (NAALADase I)) is a type II transmembrane zinc metallopeptidase that is highly expressed in the nervous system, prostate, kidney, and small intestine. It is commonly considered also as a tumor marker in prostate cancer.

In the earlier art, various PSMA-targeting agents carrying therapeutic or diagnostic moieties have been developed.

More recently, various small-molecule PSMA targeting agents capable of binding to the extracellular domain of PSMA were developed for PET/CT and SPECT/CT imaging, including radiolabeled N-[N-[(S)-1,3-dicarboxypropyl]carbamoyl]-S-[11C]methyl-I-cysteine (DCFBC) and several urea-based peptidomimetic PSMA-inhibitors (cf. Bouchelouche et al. Discov Med. 2010 Jan; 9(44): 55-61), including MIP-1095 (Hillier et al. Cancer Res. 2009 Sep 1;69(17):6932-40), a PSMA ligand currently in clinical evaluation, and DOTA-conjugated PSMA-inhibitor PSMA-617 developed by Benesová et al (JNM 2015, 56: 914-920 and EP 2862 857 A1).

Urea-based PSMA ligands usually comprise three components: the binding motif (Glu-urea-Lys), a linker, and a radiolabel-bearing moiety (chelator molecule for radiolabeling or a prosthetic group for fluorinated agents). Examples of the most commonly used low-molecular-weight PSMA ligands are ¹²³I-MIP-1072 and ¹²³I-MIP-1095 (Barrett JA et al. J Nucl Med. 2013; 54:380-387); ^{99m}Tc-MIP-1404 and ^{99m}Tc-1405 (Hillier SM et al. J Nucl Med. 2013; 54: 1369-1376) for SPECT imaging in clinical trials. N-[N-[(S)-1,3-dicarboxypropyl]carbamoyl]-4-¹⁸F-fluorobenzyl-L-cysteine (¹⁸F-DCFBC) (Cho SY et al., J nucl. Med. 2012; 53: 1883-1891) and ⁶⁸Ga-PSMA-11 (⁶⁸Ga-PSMA-N,N'-bis-[2-hydroxy-5-(carboxyethyl)benzyl]ethylenediamine-N,N'-diacetic acid) (Eder M et al, Pharmaceuticals (Basel) 2014; 7:779-796) are agents for PET imaging. Further threranostic agents are e.g. ¹³¹I-MIP-1095 (Zechmann et al., Eur J Nucl Med Mol Imaging. 2014; 41:1280-1292), chelator based PSMA-617 (Afshar-Oromieh A et al., J Nucl Med. 2015; 56:1697-1705) and PSMA-I&T (Weineisen M et al., J Nucl Med. 2015; 56:1169-1176), PSMA-I&S (Robu S et al., J Nucl Med. 2017; 58:235-242). As further ¹⁸F-labeled small-molecule urea derivatives ¹⁸F-DCFPyL (Chen Y et al., Clin Cancer Res. 2011; 17:7645-7653) and ¹⁸F-PSMA-1007 (Giesel FL et al., Eur J Nucl Med Molecular Imaging. 2017; 44:678-688) are mentioned.

Recently, Kelly et al. (J Nucl Med. 2017 pii: jnumed.116.188722. doi: 10.2967/jnumed.116.188722. [Epub ahead of print]) evaluated agents exhibiting affinity for both PSMA and for human serum albumin (HSA). The ligands developed by Kelly et al. comprise a *p*-(iodophenyl)butyric acid entity for HSA binding and an urea-based PSMA binding entity. In the compounds developed by Kelly et al., radiotherapeutic iodine (¹³¹I) is covalently attached to the HSA binding moiety, which is in turn directly connected to the PSMA binding entity via a hydrocarbyl chain. Another example is a ¹⁷⁷Lu-labeled phosphoramidate-based PSMA inhibitor with an albumin-binding entity (Choy et al. Theranostics 2017; 7(7): 1928-1939). A DOTA chelator complexing the ¹⁷⁷Lu radionuclide was ether-linked to the irreversible PSMA inhibitor CTT1298 (EP 2970345 A1).

Thus, in a preferred embodiment of the present invention, PAH or a salt thereof is used to reduce nephrotoxic side effects of (radio)pharmaceuticals comprising a PSMA-targeting ligand bound to a chelator molecule, as defined above, complexed with a radionuclide, as defined above, e.g. selected from ⁶⁸Ga, ¹⁷⁷Lu, ²²⁵Ac, and ¹¹¹In.

In a particular preferred embodiment, PAH is used to reduce the nephrotoxic side effects of ⁶⁸Ga-PSMA-11.

### Folate conjugates

The FR-α attracted most interest as a tumor-associated target for imaging purposes and targeted therapy concepts. Targeting of FR-positive tumor cells *in vitro* and *in vivo* has been exemplified by a number of research groups using folic acid conjugates with a variety of therapeutic probes. The folate receptor (FR) has thus proven a valuable target for nuclear imagine using folic acid radioconjugates.

However, using folate-based radiopharmaceuticals for therapy has long been regarded as an unattainable goal because of their considerable renal accumulation. As for other radioconjugates, as mentioned herein, the present invention allows by the inventive strategy to reduce off-site accumulation of the radiopharmaceuticals *in vivo,* thus improving the tumor-to-kidney ratios.

Examples of folate conjugate radiopharmaceuticals use ^{99m}Tc (Guo et al., J Nucl Med. 1999; 40: 1563-1569; Mathias et al., Bioconjug Chem. 2000; 11:253-257; Leamon et al., Bioconjug Chem. 2002; 13:1200-1210; Reddy et al., J Nucl. Med. 2004; 45:857-866; Müller et al., J Nucl Med Mol Imaging 2006; 33:1007-1016; Müller et al., Bioconjug Chem. 2006; 17:797-806), ¹¹¹In (Siegel et al., J Nucl Med. 2003; 44:700-707), ^{66/67/68}Ga (Mathias et al., Nucl Med Biol. 1999; 26:23-25; Mathias et al., Nucl Med Biol. 2003; 30:725-731) and ¹⁸F (Bettio et al., J Nucl Med. 2006; 47:1153-1160).

Representative folate conjugates according to the invention are e.g. ¹¹¹In-DTPA-folate, ¹⁷⁷Lu-EC0800, ¹⁷⁷Lu-cm09, ^{149/161}Tb-cm09, ^{99m}Tc(CO)₃, ^{99m}Tc-EC20, ¹¹¹In-DTPA-folate, ¹¹¹In/¹⁷⁷Lu-DOTA-click-folate, ⁶⁷Ga-DOTA-Bz-folate (⁶⁷Ga-EC0800), ⁶⁸Ga-NODAGA-folate and

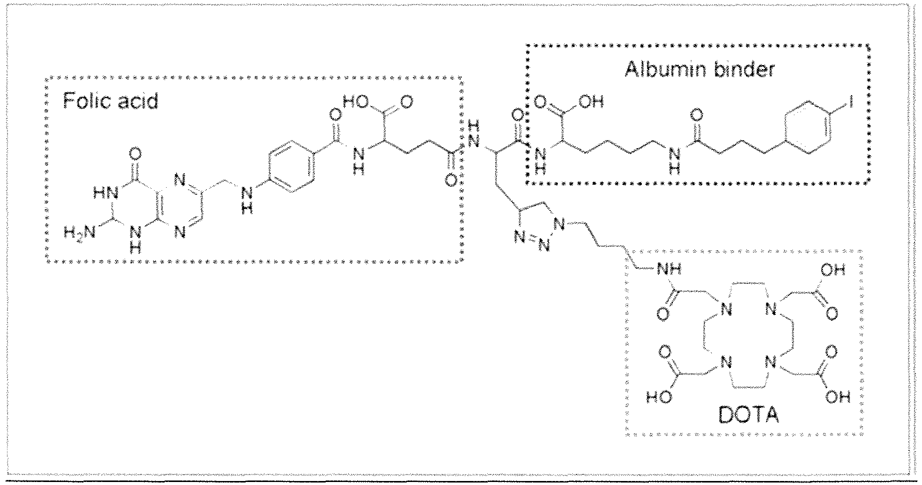

### CCK2 receptor-targeting compounds

PAH or a salt thereof can also be suitably used to reduce nephrotoxic side effects of (radio)pharmaceuticals targeting the CCK2 receptor.

The CCK2 receptor (cholecystokinin) is located in areas of the central and peripheral nervous system and is overexpressed in several types of human cancer, as medullar thyroid carcinomas, small cell lung cancers and stromal ovarian carcinomas. Exclusive research has been done on developing suitable radioligands for targeting the CCK2-receptor *in vivo.* A variety of radiolabeled CCK/gastrin-related peptides has been synthesized and characterized. All peptides have the C-terminal CCK receptor-binding tetrapeptide sequence Trp-Met-Asp-Phe-NH2 in common or derivatives thereof. The peptides can be categorized based on the sequence of their parent peptide (gastrin or CCK) and on their form (i.e. linear, cyclic, multimers).

Examples for CCK receptor ligands are gastrin analogs, such as Sargastrin (Gln-Gly-Pro-Trp-Leu-Glu-Glu-Glu-Glu-Glu-Ala-Tyr-Gly-Trp-Nle-Asp-Phe-NH₂), Minigastrin 0 (MG-0) D-Glu-(Glu)₅-Ala-Tyr-Gly-Trp-Met-Asp-Phe-NH₂), Minigastrin 11 (MG-11) (D-Glu-Ala-Tyr-Gly-Trp-Met-Asp-Phe-NH2), cyclo-Minigastrin 1 (cyclo-MG1) (cyclo[γ-D-Glu-Ala-Tyr-D-Lys]-Trp-Met-Asp-Phe-NH2), cyclo-Minigastrin 2 (cyclo-MG2) (cyclo[γ-D-Glu-Ala-Tyr-D-Lys]-Trp-Nle-Asp-Phe-NH2, Demogastrin 1 (D-Glu-(Glu)₅-Ala-Tyr-Gly-Trp-Met-Asp-Phe-NH₂), Demogastrin 2 (D-Glu-(Glu)₅-Ala-Tyr-Gly-Trp-Met-Asp-Phe-NH₂, H2-Met (His-His-Glu-Ala-Tyr-Gly-Trp-Met-Asp-Phe-NH₂), H2-Nle (His-His-Glu-Ala-Tyr-Gly-Trp-Nle-Asp-Phe-NH₂), H6-Met (His)₆-Glu-Ala-Tyr-Gly-Trp-Met-Asp-Phe-NH₂); and CCK8 analogs, such as CCK8 (D-Asp-Tyr-Met-Gly-Trp-Met-Asp-Phe-NH2), CCK8(Nle) (D-Asp-Tyr-Nle-Gly-Trp-Nle-Asp-Phe-NH2), sCCK8 (D-Asp-Tyr(OSO₃H)-Met-Gly-Trp-Met-Asp-Phe-NH₂), sCCK8[Phe²(*p-*CH₂SO₃H), Nle^{3,6}] (D-Asp-Phe(p-CH₂SO₃H)-Nle-Gly-Trp-Nle-Asp-Phe-NH₂), sCCK8[Phe²(*p-*CH₂SO₃H), HPG^{3,6}] (D-Asp-Phe(*p*-CH₂SO₃H)-HPG-Gly-Trp-HPG-Asp-Phe-NH₂).

The CCK receptor targeting peptides are preferably radiolabeled with the radionuclides for imaging or therapeutic applications. Suitable radionuclides comprise the radionuclides specified above, and in particular comprise the radionuclides ¹¹¹In, ⁶⁸Ga, ¹³¹I, ⁹⁰Y, and ¹⁷⁷Lu. To allow radiolabeling with a radionuclide, a chelator conjugated to the peptide is preferably used. As a chelator, the chelators specified above can be used, wherein DOTA, DOTAGA, DOTAM, DTPA and HYNIC are preferred.

Therefore, in a preferred embodiment of the present invention, PAH is used to reduce the nephrotoxic side effects of CCK2 receptor targeting (radio)pharmaceuticals including, but not limited to ¹⁷⁷Lu-DOTA-Sargastrin, ¹¹¹In-DTPA-MG0, ¹¹¹In-DOTA-MG11, ¹¹¹In-DOTA-MG11(Nle), ¹¹¹In-DOTA-H2-Met, ¹¹¹In-DOTA-H2-Nle, ¹¹¹In-DOTA-H6-Met,; and CCK8 analogs, such as ¹¹¹In-DTPA-CCK8, ¹¹¹In-DTPA-CCK8(Nle),¹¹¹In-DOTA-sCCK8[Phe²(*p-*CH₂SO₃H), Nle^{3,6}], and ¹¹¹In-DOTA-sCCK8[Phe²(*p*-CH₂SO₃H), HPG^{3,6}].

### Integrin-binding molecules

PAH or a salt thereof can also be suitably used to reduce nephrotoxic side effects of (radio)pharmaceuticals targeting integrins.

Integrins are heterodimeric glycoproteins consisting of an *α*- and *β*-subunit. There are 24 different combinations of the eight *β*-units and the eighteen *α*-units known. The integrins mediate cell-cell and cell-matrix interactions and transduce signals across the plasma membrane via insight-out and outside-in signaling. Some of the integrins play an important role during migration of endothelial as well as tumor cells during tumor-induced angiogenesis and tumor metastasis. Angiogenesis, the formation of new blood vessels out of the preexisting vasculature, is a critical step in the development and dissemination of various human tumors. A variety of therapeutic strategies in oncology are focused on the inhibition of tumor-induced angiogenesis. Concerning the integrins, significant attention has been paid to the role of integrin *α*V*β*3 and *α*V*β*5, as they are prominent on proliferating vascular endothelial cells. Thus, one of the most prominent target structures used for the development of radiopharmaceuticals for imaging angiogenesis is the integrin *α*V*β*3.

Tumor-induced angiogenesis can be blocked *in vivo* by antagonizing the αᵥβ₃ integrin with small peptides containing the Arg-Gly-Asp (RGD) amino acid sequence. This tripeptidic sequence, naturally present in extracellular matrix proteins, is the primary binding site of the αᵥβ₃ integrin. Because of selective expression of αᵥβ₃ integrin in tumors, radiolabeled RGD peptides are attractive candidates for αᵥβ₃ integrin targeting in tumors. Over the last decade, many radiolabeled linear and cyclic RGD peptides have been evaluated as radiotracers for imaging tumors by SPECT or PET, as well as therapeutic agents.

Therefore, PAH or a salt thereof can be particularly suitably used to reduce nephrotoxic side effects of (radio)pharmaceuticals comprising radiolabeled RGD peptides.

The RGD peptides are preferably radiolabeled with radionuclides for imaging or therapeutic applications. Suitable radionuclides comprise the radionuclides specified above, and in particular comprise the radionuclides ⁶⁸Ga, ¹¹¹In, ⁹⁰Y, and ¹⁷⁷Lu. To allow radiolabeling with a radionuclide, a chelator conjugated to the peptide is preferably used. As a chelator, any suitable the chelators, e.g. as specified above, can be used, wherein NOTA, DOTA, DOTAGA, DOTAM, DTPA, HYNIC are preferred.

For example, PAH or a salt thereof can suitably be used to reduce nephrotoxic side effects of ⁶⁶Ga-NOTA-RGD, and ¹¹¹In-DOTA-E-[c(RGDfK)]₂.

### Neurotensin receptor-targeting compounds

Neurotensin receptor 1 (NTR1) is overexpressed in ductal pancreatic adenocarcinoma, which is one of the deadliest cancers. Several NTR1 antagonists have been developed, such as SR142948A and SR48692, and ¹⁷⁷Lu-3BP-2273, which is a ¹⁷⁷Lu-labeled DOTA-conjugated NTR1 antagonist that has been developed on the basis of SR142948A. It has been used for the treatment of ductal pancreatic adenocarcinoma (Baum RP et al., The Journal of Nuclear Medicine, Vol. 59, No. 5, May 2018).

Therefore, PAH or a salt thereof can also be suitably used to reduce nephrotoxic side effects of (radio)pharmaceuticals targeting the Neurotensin receptor 1, in particular of radiolabeled NTR1 antagonists for cancer diagnosis or therapy, preferably ¹⁷⁷Lu- or ⁶⁸Ga-labeled NTR1 antagonists, more preferably ¹⁷⁷Lu-3BP-2273, even though other radionuclides, for example the radionuclides mentioned above, as well as other chelators, for example the chelators mentioned above, may be contemplated.

### Glucagon-like peptide-1 (GLP-1) receptor targeting compounds

The GLP-1 receptor is overexpressed on essentially all benign insulinomas and also on gastrinomas. Benign insulinomas which emerge from β-cells of the pancreas and are present as small nodules, secrete insulin leading to potentially life-threatening hypoglycemia.

Therefore, PAH or a salt thereof can also be suitably used to reduce nephrotoxic side effects of (radio)pharmaceuticals targeting the GLP-1 receptor. Non-limiting examples thereof include ¹¹¹In-,and ⁶⁸Ga-labeled peptides based on the 39-mer peptide exendin-4, such as Lys⁴⁰(Ahx-DOTA-¹¹¹In)NH₂-extendin-4, for example. However, other radionuclides, for example the radionuclides mentioned above, as well as other chelators, for example the chelators mentioned above, may be contemplated.

### Gastrin releasing peptide (GRP) receptor targeting compounds

PAH or a salt thereof can also be suitably used to reduce nephrotoxic side effects of (radio)pharmaceuticals targeting the GRP receptor.

GRP receptors have been demonstrated in major human tumors, such as breast cancer and prostate cancer. Bombesin is a tetradecapeptide neurohormone and an amphibian homolog of mammalian GRP (a 27mer peptide). Various bombesin analogs have been developed for ^{99m}Tc labeling and SPECT. In particular, truncated bombesin was coupled to an N₃S-chelator via a Gly-5-aminovaleric acid spacer (^{99m}Tc-RP527). Moreover, several bombesin analogs and bombesin antagonists have been developed and labeled with different radioisotopes (e.g. ⁶⁸Ga, ⁶⁴Cu, ¹⁸F) using different chelators. Examples thereof include a pan-bombesin analog ⁶⁸Ga-BZH3 (Zhang H et al., Cancer Res 2004; 64: 6707-6715), and a ¹⁷⁷Lu-labeled bombesin(7-14) derivative coupled to DOTA via a Gly-4-aminobenzoyl spacer (Bodei L et al., Eur J Nucl Med Mol Imaging 2007: 34(suppl 2): S221.

However, PAH or a salt thereof can also be suitably used to reduce nephrotoxic side effects of GRP receptor targeting (radio)pharmaceuticals comprising other radionuclides, for example the radionuclides mentioned above, as well as other chelators, for example the chelators mentioned above.

### Neurokinin type 1 receptor targeting compounds

The neurokinin type 1 receptor is consistently overexpressed on glioma cells and on tumor vessels (Hennig IM et al., Int J Cancer 1995; 61: 786-792). The radiolabeled 11-amino-acid peptide substance P (Arg Pro Lys Pro Gln Gln Phe Phe Gly Leu Met) acting via the neurokinin type 1 receptor can suitably be used to target malignant gliomas. In particular, substance P has been conjugated to the chelator DOTAGA, and ⁹⁰Y-labeled DOTAGA-substance P has been used in clinically studies (Kneifel S et al., Eur J Nucl Med Mol Imaging. 2007; 34: 1388-1395. In another study, the feasibility and effectiveness of targeted α-radionuclide therapy for brain tumors was assessed using the α-radiation-emitting conjugate ²¹³Bi-DOTA-[THi8,Met(O2)11]-substance P (Cordier et al., Eur J Nucl Med Mol Imaging. 2010; 37: 1335-1344).

Therefore, PAH or a salt thereof can also be suitably used to reduce nephrotoxic side effects of therapeutic and diagnostic compounds targeting the neurokinin type 1 receptor, in particular substance P conjugates comprising a radionuclide for diagnostic or therapy, and a chelator coordinating the radionuclide.

### Affilins

PAH or a salt thereof can also be suitably used to reduce nephrotoxic side effects of antibody mimetics used as therapeutic and diagnostic compounds.

Affilins are artificial proteins designed to selectively bind antigens. Affilin proteins are structurally derived from human ubiquitin or gamma-B crystallin, respectively. Affilin proteins are constructed by modification of surface-exposed amino acids of these proteins and isolated by display techniques such as phage display and screening. They resemble antibodies in their affinity and specificity to antigens but not in structure, which makes them a type of antibody mimetic. Affilin^{®} was developed by Scil Proteins GmbH as potential biopharmaceutical drugs, diagnostics and affinity ligands. Affilin molecules can be easily modified and are suitable to label tumor cells for diagnostic purposes or to kill tumor cells specifically by irradiation.

Multispecific Affilin molecules can be generated, binding different targets simultaneously. Radionuclides or cytotoxins can be conjugated to Affilin proteins, making them potential tumor therapeutics and diagnostics. Radionuclide-chelator-Affilin conjugates, e.g. ¹⁷⁷Lu-DOTA-Affilin and ⁶⁸Ga-DOTA-Affilin, have been designed for imaging and therapy purposes. PAH can be used to effectively reduce nephrotoxic side effects of these Affilin conjugates. It may also be used to reduce nephrotoxic side effects of further Affilin conjugates comprising other radionuclides (for example as specified above) and chelators (for example as specified above), respectively.

Thus, in a preferred embodiment, PAH or a pharmaceutically acceptable salt thereof is used to reduce nephrotoxic side effects of radiolabeled therapeutic and diagnostic compounds for therapy or imaging of cancer, such as for example neuroendocrine tumors, prostate cancer, pancreatic cancer, renal cancer, bladder cancer, medullar thyroid carcinomas, small cell lung cancers, stromal ovarian carcinomas, ductal pancreatic adenocarcinoma, insulinomas, gastrinomas, breast cancer etc.

For example, PAH or a pharmaceutically acceptable salt thereof is used to reduce the nephrotoxic side effects of radiolabeled therapeutic and diagnostic compounds used for therapy or imaging of prostate cancer, such as (radio)pharmaceuticals targeting the somatostatin receptor or of radiolabeled therapeutic and diagnostic compounds targeting prostate-specific membrane antigen (PSMA). In a preferred embodiment, PAH or a pharmaceutically acceptable salt thereof is used to reduce the nephrotoxic side effects of ¹⁷⁷Lu-DOTATOC (¹⁷⁷Lu-DOTA°-[Tyr3]-octreotide).

In an embodiment of the present invention, PAH or a pharmaceutically acceptable salt thereof is used in combination with a further substance which reduces nephrotoxic side effects of radiolabeled therapeutic and diagnostic compounds. Examples of further substances which reduce nephrotoxic side effects of radiolabeled and nonradiolabeled therapeutic and diagnostic compounds are amino acids, such as lysine and arginine and mixtures thereof, gelatine, Amifostine, albumin-derived peptides, trypsinised albumin, PSMA-binding molecules, such as PMPA, Vitamins, Gelofusine, or FRALB-C (bovine serum albumin fragmented by cyanogen bromide).

In a particularly preferred embodiment of the present invention, the pharmaceutically acceptable salt of PAH is aminohippurate sodium. Preferably, PAH or a pharmaceutically acceptable salt thereof, preferably aminohippurate sodium, is used in a salt solution, preferably water for injection (WFI) or a NaCl solution, more preferably in a 20% NaCl solution.

In the present invention, PAH or a pharmaceutically acceptable salt thereof, preferably aminohippurate sodium, is used in an amount which is sufficient to effectively reduce the nephrotoxic side effects of the therapeutic and/or diagnostic compound(s). The effective amount of PAH may be determined by routine experiments, e.g. by using animal models. Such models include, without implying any limitation, rabbit, sheep, mouse, rat, dog and non-human primate models.

For instance, the administered amount of PAH may range (per kg body weight) from about 0.1 mg/kg to 10 g/kg, preferably from about 0.5 mg/kg to 5 g/kg, more preferably from about 1 mg/kg to 1 g/kg.

In a preferred embodiment of the present invention, PAH or a pharmaceutically acceptable salt thereof, preferably aminohippurate sodium, is used in an amount of about 5 mg to about 500 mg per kilogram of body weight, for example in an amount of about 5, 10, 20, 30, 40, 50, 60, 70, 80, 90, 100, 150, 200, 250, 300, 350, 400, 450, 500 mg per kilogram of body weight up to 500 mg per kilogram of body weight. More preferably, PAH or a pharmaceutically acceptable salt thereof, preferably aminohippurate sodium, is used in an amount of about 50 mg to about 500 mg per kilogram of body weight, more preferably, from about 50 mg to about 250 mg per kilogram of body weight, for example in an amount of about 50, 55, 60, 65, 70, 75, 80, 85, 90, 95, 100, 110, 120, 130, 140, 150, 160, 170, 180, 190, 200, 210, 220, 230, 240 or 250 mg per kilogram of body weight. More preferably, PAH or a pharmaceutically acceptable salt is used in an amount of about 75 mg to about 200 mg per kilogram of body weight, for example in an amount of about 75, 80, 85, 90, 95, 100, 105, 110, 115, 120, 125, 130, 135, 140, 145, 150, 155, 160, 165, 170, 175, 180, 185, 190, 195, up to 200 mg per kilogram of body weight or 200 mg per kilogram of body weight. Most preferably, PAH or a pharmaceutically acceptable salt thereof is used in an amount of about 80 mg to about 160 mg per kilogram of body weight, for example in an amount of about 80, 85, 90, 95, 100, 105, 110, 115, 120, 125, 130, 135, 140, 145, 150, 155, up to 160 mg per kilogram of body weight, or 160 mg per kilogram of body weight.

Typically, PAH or a pharmaceutically acceptable salt thereof is used in a larger (molar and/or w/w) quantity than the (co-administered) therapeutic or diagnostic compound.

For instance, the therapeutic or diagnostic compound and PAH or a pharmaceutically acceptable salt thereof, preferably aminohippurate sodium, are used in a ratio of about 1/1.000.000 to 1/10 (w/w), preferably of about 1/500.000 to 1/100 (w/w), more preferably from about 1/250.000 to about 1/500 (w/w).

In a preferred embodiment of the present invention, the therapeutic or diagnostic compound and PAH or a pharmaceutically acceptable salt thereof, preferably aminohippurate sodium, are used in a ratio of about 1/250.000 to about 1/5.000 (w/w), for example in a ratio of about 1/250.000, 1/200.000, 1/150.000, 1/100.000, or 1/50.000 to about 1/5.000 (w/w), more preferably in a ratio of about 1/240.000 to about 1/8.000 (w/w), for example in a ratio of about 1/240.000, 1/230.000, 1/220.000, 1/210.000, 1/200.000, 1/190.000, 1/180.000, 1/170.000, 1/160.000, 1/150.000, 1/140.000, 1/130.000, 1/120.000, 1/110.000, 1/100.000, 1/90.000, 1/80.000, 1/70.000, 1/60.000, 1/50.000, 1/40.000, 1/30.000, 1/20.000, 1/19.000, 1/18.000, 1/17.000, 1/16.000, 1/15.000, 1/14.000, 1/13.000, 1/12.000, 1/11.000, 1/10.000, 1/9.000, or 1/8.000 (w/w). In a further preferred embodiment, the therapeutic and/or diagnostic compound(s) and PAH or a pharmaceutically acceptable salt thereof, preferably aminohippurate sodium, are used in a ratio of about 1/100.000 to about 1/10.000 (w/w), for example in a ratio of about 1/100.000, 1/95.000, 1/90.000, 1/85.000, 1/80.000, 1/75.000, 1/70.000, 1/65.000, 1/60.000 1/55.000, 1/50.000, 1/45.000, 1/40.000, 1/35.000, 1/30.000, 1/25.000, 1/20.000, 1/15.000, 1/10.000 (w/w). In a further preferred embodiment, the therapeutic and/or diagnostic compound(s) and PAH or a pharmaceutically acceptable salt thereof, preferably aminohippurate sodium, are used in a ratio of about 1/50.000 to about 1/40.000 (w/w), for example in a ratio of about 1/50.000, 1/49.000, 1/48.000, 1/47.000, 1/46.000, 1/45.000, 1/44.000, 1/43.000, 1/42.000, 1/41.000, 1/40.000 (w/w).

In a second aspect, the present invention relates to a pharmaceutical composition comprising (a) radiolabeled pharmaceutical compound(s) in combination with para-aminohippuric acid (PAH) or a pharmaceutically acceptable salt thereof, and a pharmaceutically acceptable excipient, diluent, carrier or a combination thereof as defined by claim 10.

The radiolabeled pharmaceutical compound may be any nephrotoxic therapeutic or diagnostic compound specified above potentially exhibiting nephrotoxic side effects, as a radiolabelled diagnostic and/or therapeutic compound. The pharmaceutical composition comprises a radiolabeled pharmaceutical compound which is a radionuclide complex conjugated with a carrier molecule, thus comprising a carrier molecule, a chelator and a radionuclide.

Preferably, the carrier molecule of the radionuclide complex is selected from the carrier molecules specified above, in particular from small organic molecules, peptides, peptidomimetics, antibody fragments, antibody mimetics, small molecules, and knottings, and is more preferably selected from somatostatin analogues, PSMA-inhibitors, gastrin analogues, integrin binding molecules, and antigen binding proteins specified above, e.g. from Tyr3-octeotride, Tyr3-octreotate, JR11, PSMA-11, Sargastrin, RGD, Affilin or folate conjugates. In a particular preferred embodiment, the carrier molecule is selected from Tyr3-octeotride, and Tyr3-octreotate.

Preferably, the chelator of the radionuclide complex is selected from the chelators specified above, more preferably selected from the group consisting of DOTA, DOTAM, DOTAG, HBED-CC, NOTA, NODAGA, DOTAGA, TRAP, NOPO, PCTA, DFO, DTPA, DO3AP, DO3AP^{PrA}, DO3AP^{ABn}, HYNIC or derivatives thereof.

The radionuclide of the radionuclide complex is selected from tri-valent radionuclides, preferably selected from the group consisting of ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³ Sc, ⁴⁴Sc, ⁴⁷Sc, ²²⁵Ac, ²²⁷Th, ¹⁵³Sm, ¹⁶⁶Ho, ²²⁵Ac and ¹⁶⁶Dy, more preferably selected from the group consisting of ¹⁷⁷Lu, ⁶⁸Ga, ¹¹¹In, ⁹⁰Y, ²²⁵Ac and ¹⁶¹Tb or, most preferably, selected from ¹⁷⁷Lu, ⁶⁸Ga, ¹¹¹In, ⁹⁰Y, ²²⁵Ac and ¹⁶¹Tb, in particular ¹⁷⁷Lu, ⁹⁰Y, ⁶⁷Ga, ⁶⁸Ga, ¹¹¹In, ²²⁵Ac, ¹⁶¹Tb, ⁴⁴Sc and ⁴⁷Sc.

It is also preferred that the radionuclide is adapted for being complexed by DOTATOC (DOTA). In particular, ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, ²²⁵Ac, ¹⁵³Sm, ¹⁶⁶Ho, ²²⁵Ac and ¹⁶⁶Dy may be combined with DOTATOC as the chelator.

As mentioned above, suitable combinations of carrier molecules, chelator and radionuclides can appropriately be selected by the skilled person. For example, the radionuclide complex may be selected from [¹⁷⁷Lu-DOTA°-Tyr3]-octreotide, ¹⁷⁷Lu-DOTA-JR11, ¹⁷⁷Lu-RGD, ¹⁷⁷Lu-DOTA-Affilin 2, ¹⁷⁷Lu-DOTA-Sargastrin, ⁶⁸Ga-HBED-CC-PSMA-11. Preferably, the radionuclide complex is selected from [¹⁷⁷Lu-DOTA°-Tyr3]-octreotide and [¹⁷⁷Lu-DOTA°-Tyr3]-octreotate.

The pharmaceutical composition preferably comprises a safe and effective amount of the radiolabeled therapeutic or diagnostic compound.

As used herein, "safe and effective amount" means an amount of the agent(s) that is sufficient to allow for diagnosis and/or significantly induce a positive modification of the disease to be treated. At the same time, however, a "safe and effective amount" is small enough to avoid serious side-effects, that is to say to permit a sensible relationship between advantage and risk. A "safe and effective amount" will furthermore vary in connection with the particular condition to be diagnosed or treated and also with the age and physical condition of the patient to be treated, the severity of the condition, the duration of the treatment, the nature of the accompanying therapy, of the particular pharmaceutically acceptable excipient or carrier used, and similar factors.

In a particular preferred embodiment of the present invention, the pharmaceutical composition comprises, as a pharmaceutically acceptable salt of PAH, aminohippurate sodium.

Typically, PAH or a pharmaceutically acceptable salt thereof, preferably aminohippurate sodium, is present in the pharmaceutical composition in a larger quantity than the (co-administered) therapeutic or diagnostic compound contained in the pharmaceutical composition.

For instance, the therapeutic or diagnostic compound and PAH or a pharmaceutically acceptable salt thereof, preferably aminohippurate sodium, are present in the pharmaceutical composition in a ratio of about 1/1.000.000 to 1/10 (w/w), preferably of about 1/500.000 to 1/100 (w/w), more preferably from about 1/250.000 to about 1/500 (w/w).

In a preferred embodiment of the present invention, the therapeutic or diagnostic compound and PAH or a pharmaceutically acceptable salt thereof, preferably aminohippurate sodium, are present in the pharmaceutical composition in a ratio of about 1/250.000 to about 1/5.000 (w/w), for example in a ratio of about 1/250.000, 1/200.000, 1/150.000, 1/100.000, or 1/50.000 to about 1/5.000 (w/w), more preferably in a ratio of about 1/240.000 to about 1/8.000 (w/w), for example in a ratio of about 1/240.000, 1/230.000, 1/220.000, 1/210.000, 1/200.000, 1/190.000, 1/180.000, 1/170.000, 1/160.000, 1/150.000, 1/140.000, 1/130.000, 1/120.000, 1/110.000, 1/100.000, 1/90.000, 1/80.000, 1/70.000, 1/60.000, 1/50.000, 1/40.000, 1/30.000, 1/20.000, 1/19.000, 1/18.000, 1/17.000, 1/16.000, 1/15.000, 1/14.000, 1/13.000, 1/12.000, 1/11.000, 1/10.000, 1/9.000, or 1/8.000 (w/w). More preferably, the therapeutic and/or diagnostic compound(s) and PAH or a pharmaceutically acceptable salt thereof, preferably aminohippurate sodium, are present in the pharmaceutical composition in a ratio of about 1/100.000 to about 1/10.000 (w/w), for example in a ratio of about 1/100.000, 1/95.000, 1/90.000, 1/85.000, 1/80.000, 1/75.000, 1/70.000, 1/65.000, 1/60.000 1/55.000, 1/50.000, 1/45.000, 1/40.000, 1/35.000, 1/30.000, 1/25.000, 1/20.000, 1/15.000, 1/10.000 (w/w). In a further preferred embodiment, the therapeutic and/or diagnostic compound(s) and PAH or a pharmaceutically acceptable salt thereof, preferably aminohippurate sodium, are present in the pharmaceutical composition in a ratio of about 1/50.000 to about 1/40.000 (w/w), for example in a ratio of about 1/50.000, 1/49.000, 1/48.000, 1/47.000, 1/46.000, 1/45.000, 1/44.000, 1/43.000, 1/42.000, 1/41.000, 1/40.000 (w/w).

In an embodiment of the present invention, the pharmaceutical composition, in addition to PAH, comprises a further substance which reduces nephrotoxic side effects of radiolabeled therapeutic and diagnostic compounds, wherein the substance reducing nephrotoxic side effects of radiolabeled therapeutic and diagnostic compounds other than PAH is preferably selected from amino acids, e.g. lysin and arginine, gelatine, Amifostine, albumin-derived peptides, PSMA-binding molecules, such as PMPA, vitamins.

The pharmaceutical composition comprising PAH or a pharmaceutically acceptable salt thereof, comprises a pharmaceutical compound(s), and optionally comprising a further substance which reduces nephrotoxic side effects of radiolabeled and therapeutic and diagnostic compounds, further comprises a pharmaceutically acceptable excipient, diluent, carrier or a combination thereof. The pharmaceutical composition is preferably a liquid or semi-liquid composition, which is more preferably a liquid or semi-liquid composition, which is more preferably an aqueous solution, which may be buffered and/or exhibit isotonic properties.

The term "pharmaceutically acceptable" refers to a compound or agent that is compatible with the components of the inventive pharmaceutical composition, in particular the diagnostic or therapeutic pharmaceutical compound(s), and does not interfere with and/or substantially reduce its diagnostic or therapeutic activities. Pharmaceutically acceptable carriers preferably have sufficiently high purity and sufficiently low toxicity to make them suitable for administration to a subject to be treated.

### Formulations, carriers and excipients

Pharmaceutically acceptable excipients can exhibit different functional roles and include, without limitation, diluents, fillers, bulking agents, carriers, disintegrants, binders, lubricants, glidants, coatings, solvents and co-solvents, buffering agents, preservatives, adjuvants, antioxidants, wetting agents, anti-foaming agents, thickening agents, sweetening agents, flavouring agents and humectants.

Suitable pharmaceutically acceptable excipients are typically chosen based on the formulation of the pharmaceutical composition.

For pharmaceutical compositions in liquid form, useful pharmaceutically acceptable excipients in general include solvents, diluents or carriers such as (pyrogen-free) water, (isotonic) saline solutions such phosphate or citrate buffered saline, fixed oils, vegetable oils, such as, for example, groundnut oil, cottonseed oil, sesame oil, olive oil, corn oil, ethanol, polyols (for example, glycerol, propylene glycol, polyetheylene glycol, and the like); lecithin; surfactants; preservatives such as benzyl alcohol, parabens, chlorobutanol, phenol, ascorbic acid, thimerosal, and the like; isotonic agents such as sugars, polyalcohols such as manitol, sorbitol, or sodium chloride; aluminum monostearate or gelatin; antioxidants such as ascorbic acid or sodium bisulfite; chelating agents such as ethylenediaminetetraacetic acid (EDTA); buffers such as acetates, citrates or phosphates and agents for the adjustment of tonicity such as sodium chloride or dextrose. pH can be adjusted with acids or bases, such as hydrochloric acid or sodium hydroxide. Buffers may be hypertonic, isotonic or hypotonic with reference to the specific reference medium, i.e. the buffer may have a higher, identical or lower salt content with reference to the specific reference medium, wherein preferably such concentrations of the aforementioned salts may be used, which do not lead to damage of cells due to osmosis or other concentration effects. Reference media are e.g. liquids occurring in *in vivo* methods, such as blood, lymph, cytosolic liquids, or other body liquids, or e.g. liquids, which may be used as reference media in *in vitro* methods, such as common buffers or liquids. Such common buffers or liquids are known to a skilled person.

Liquid pharmaceutical compositions administered via injection and in particular via intravasal, more preferably intravenous (i.v.) injection should preferably be sterile and stable under the conditions of manufacture and storage. Such compositions are typically formulated as parenterally acceptable aqueous solutions that are pyrogen-free, have suitable pH, are isotonic and maintain stability of the active ingredient(s).

For liquid pharmaceutical compositions, suitable pharmaceutically acceptable excipients and carriers include water, typically pyrogen-free water; isotonic saline or buffered (aqueous) solutions, e.g. phosphate, citrate etc. buffered solutions. Particularly for injection of the inventive (pharmaceutical) compositions, water or preferably a buffer, more preferably an aqueous buffer, may be used, which may contain a sodium salt, e.g. at least 50 mM of a sodium salt, a calcium salt, e.g. at least 0,01 mM of a calcium salt, and optionally a potassium salt, e.g. at least 3 mM of a potassium salt.

The sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCI, Kl, KBr, K₂CO₃, KHCO₃, K₂SO₄, and examples of calcium salts include e.g. CaCl₂, Cal₂, CaBr2, CaCO₃, CaSO₄, Ca(OH)₂. Furthermore, organic anions of the aforementioned cations may be contained in the buffer.

Buffers suitable for injection purposes as defined above, may contain salts selected from sodium chloride (NaCI), calcium chloride (CaCl₂) and optionally potassium chloride (KCI), wherein further anions may be present additional to the chlorides. CaCl₂ can also be replaced by another salt like KCI. Typically, the salts in the injection buffer are present in a concentration of at least 50 mM sodium chloride (NaCl), at least 3 mM potassium chloride (KCl) and at least 0,01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects.

For pharmaceutical compositions in (semi-)solid form, suitable pharmaceutically acceptable excipients and carriers include binders such as microcrystalline cellulose, gum tragacanth or gelatine; starch or lactose; sugars, such as, for example, lactose, glucose and sucrose; starches, such as, for example, corn starch or potato starch; cellulose and its derivatives, such as, for example, sodium carboxymethylcellulose, ethylcellulose, cellulose acetate; disintegrants such as alginic acid; lubricants such as magnesium stearate; glidants such as stearic acid, magnesium stearate; calcium sulphate, colloidal silicon dioxide and the like; sweetening agents such as sucrose or saccharin; and/or flavoring agents such as peppermint, methyl salicylate, or orange flavoring.

Generally, pharmaceutical compositions for topical administration can be formulated as creams, ointments, gels, pastes or powders. Pharmaceutical compositions for oral administration can be formulated as tablets, capsules, liquids, powders or in a sustained release format. However, according to preferred embodiments, the inventive pharmaceutical composition is administered parenterally, in particular via intravenous or intratumoral injection, and is accordingly formulated in liquid or lyophilized form for parenteral administration as discussed elsewhere herein. Parenteral formulations are typically stored in vials, IV bags, ampoules, cartridges, or prefilled syringes and can be administered as injections, inhalants, or aerosols, with injections being preferred.

The pharmaceutical composition may be provided in lyophilized form. Lyophilized pharmaceutical compositions are preferably reconstituted in a suitable buffer, advantageously based on an aqueous carrier, prior to administration.

The pharmaceutical compositions or medicaments are preferably for use in a method for the reduction of nephrotoxic side effects of radiolabeled therapeutic and diagnostic compounds for imaging or treating diseases, in particular tumor diseases, such neuroendocrine tumors, prostate cancer, pancreatic cancer, renal cancer, bladder cancer, brain cancer, gastrointestinal cancer, medullar thyroid carcinomas, small or non-small cell lung cancers and stromal ovarian carcinomas, ductal pancreatic adenocarcinoma, insulinomas, gastrinomas, breast cancer, or sarcoma.

### Kits

According to a further aspect, the present invention provides a kit according to claim 18 comprising the pharmaceutical components used according to the present invention, e.g. para-aminohippuric acid (PAH) or a pharmaceutically acceptable salt thereof, and a radiolabeled therapeutic or diagnostic compound as specified above. For example, in an embodiment, the kit may comprise para-aminohippuric acid (PAH) or a pharmaceutically acceptable salt thereof in one part of the kit, and may comprise optionally a solution for e.g. solubility PAH or a pharmaceutically acceptable salt thereof in another part of the kit. The solution may be isotonic or hypertonic, it may be buffered, e.g. an optionally buffered aqueous solution, e.g. an aqueous NaCl solution or water for injection (WFI). According to the present invention, the kit may comprise para-aminohippuric acid (PAH) or a pharmaceutically acceptable salt thereof in one part of the kit, and a radiolabeled therapeutic or diagnostic compound as specified above in another part of the kit.

Optionally, the kit may comprise at least one further agent as defined herein in the context of the pharmaceutical composition, including e.g. amino acids, such as lysine and arginine and mixtures thereof, gelatine, Amifostine, albumin-derived peptides, PSMA-binding molecules, such as PMPA, vitamins, radionuclides, antimicrobial agents, solubilizing agents or the like.

The kit may be a kit of two or more parts comprising any of the components exemplified above in suitable containers. For example, each container may be in the form of vials, bottles, squeeze bottles, jars, sealed sleeves, envelopes or pouches, tubes or blister packages or any other suitable form, provided the container preferably prevents premature mixing of components. Each of the different components may be provided separately, or some of the different components may be provided together (i.e. in the same container).

A container may also be a compartment or a chamber within a vial, a tube, a jar, or an envelope, or a sleeve, or a blister package or a bottle, provided that the contents of one compartment are not able to associate physically with the contents of another compartment prior to their deliberate mixing by a pharmacist or physician.

The kit or kit-of-parts may furthermore contain technical instructions with information on the administration and dosage of any of its components.

### Therapeutic and diagnostic methods and uses

Also disclosed is the use of para-aminohippuric acid (PAH) or a pharmaceutically acceptable salt thereof, and/or of a pharmaceutical composition as described above, and/or of a kit as described above for the preparation of a medicament for para-aminohippuric acid (PAH) thereof. Another aspect refers to a pharmaceutical composition as described above for use or the reduction of nephrotoxic side effects of radiolabeled therapeutic and diagnostic compounds in a subject according to claim 19.

It also relates to a pharmaceutical composition as described above or of a kit as described above for use in a method for the reduction of nephrotoxic side effects of radiolabeled therapeutic and diagnostic compounds in a subject according to claim 19.

In a further aspect, the present application also provides a pharmaceutical composition or a kit as described above in a method for the reduction of nephrotoxic side effects of radiolabeled therapeutic and diagnostic compounds in a subject, the method comprising administering a pharmaceutical composition as described above or a kit as described above to a subject during imaging or therapy using a radiolabeled compound.

In a further aspect, the present application also provides PAH or a pharmaceutically acceptable salt thereof for use in a method for the reduction of nephrotoxic side effects of radiolabeled therapeutic and diagnostic compounds in a subject, the method comprising administering para-aminohippuric acid (PAH) or a pharmaceutically acceptable salt thereof in combination with a radiolabeled therapeutic or diagnostic compound in a subject, wherein the administration of PAH is prior and/or during and/or after administration of the radiolabeled therapeutic or diagnostic compound according to claim 20.

According to the invention, the use in the method is for the reduction of nephrotoxic side effects of radiopharmaceuticals in a subject in radio ligand therapy or diagnosis. The radiopharmaceutical is a radionuclide complex comprising a carrier molecule, a chelator and a tri-valent radionuclide as specified above. In a more preferred embodiment, the carrier molecule is selected from peptides, peptidomimetics, antibody fragments, antibody mimetics, small molecules, knottings, which may have the property of being an agonistic or antagonistic ligand of a cell receptor, in particular a cell surface receptor. In a particular preferred embodiment, the carrier molecule is selected from somatostatin analogues, PSMA-inhibitors, gastrin analogues, integrin binding molecules as specified above, and for example is selected from Tyr3-octeotride, Tyr3-octreotate, JR11, PSMA-11, Sargastrin, RGD.

In a further preferred embodiment, the chelator of the radiopharmaceutical compound used in the method of the present invention is selected from DOTA, HBED-CC, NOTA, NODAGA, DOTAGA, DOTAM, TRAP, NOPO, PCTA, DFO, DTPA, DO3AP, DO3AP^{PrA}, DO3AP^{ABn}, HYNIC or derivatives thereof.

In a further preferred embodiment, the radionuclide of the radiopharmaceutical compound used in the method of the present invention is selected from the group consisting of ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, and ²²⁵Ac, and is more preferably selected from ¹⁷⁷Lu, ²²⁵Ac and ⁶⁸Ga.

In a further preferred embodiment of the present invention, the radionuclide containing conjugate molecule used in the method of the present invention is selected from [¹⁷⁷Lu-DOTA°-Tyr3]-octreotide, ¹⁷⁷Lu-DOTA-JR11, ¹⁷⁷Lu-DOTA-RGD, ¹⁷⁷Lu-DOTA-Sargastrin, ⁶⁸Ga-HBED-CC-PSMA-11, PSMA11, and ¹⁷⁷Lu- PSMA I&T.

In a particularly preferred embodiment of the present invention, the pharmaceutically acceptable salt of PAH used in the method of the present invention is aminohippurate sodium.

In the use in a method of the present invention, PAH or a pharmaceutically acceptable salt thereof, preferably aminohippurate sodium, is preferably administered in an amount which is sufficient to effectively reduce the nephrotoxic side effects of the therapeutic and/or diagnostic compound, which is typically administered to the subject in parallel. The effective amount of PAH may be determined by routine experiments, e.g. by using animal models. Such models include, without implying any limitation, rabbit, sheep, mouse, rat, dog and non-human primate models.

For instance, the administered amount of PAH may range (per kg body weight) from about 0.1 mg/kg to 10 g/kg, preferably from about 0.5 mg/kg to 5 g/kg, more preferably from about 1 mg/kg to 1 g/kg.

In a particular preferred use in a method of the present invention, PAH or a pharmaceutically acceptable salt thereof, preferably aminohippurate sodium, is administered in an amount of about 5 mg to about 500 mg per kilogram of body weight, typically administered in parallel (e.g. prior, concurrently or after administration of the nephrotoxic therapeutic or diagnostic compound, for example in an amount of about 5, 10, 20, 30, 40, 50, 60, 70, 80, 90, 100, 150, 200, 250, 300, 350, 400, 450, 500 mg per kilogram of body weight. Thus, the amounts as exemplified herein may be given on the same day (e.g. as the diagnostic/therapeutic nephrotoxic compound). The administration regimen of PAH or its salttypically follows the administration regimen of the nephrotoxic compound. More preferably, PAH or a pharmaceutically acceptable salt thereof, preferably aminohippurate sodium, is administered in an amount of about 50 mg to about 500 mg per kilogram of body weight, more preferably, from about 50 mg to about 250 mg per kilogram of body weight, for example in an amount of about 50, 55, 60, 65, 70, 75, 80, 85, 90, 95, 100, 110, 120, 130, 140, 150, 160, 170, 180, 190, 200, 210, 220, 230, 240 or 250 mg per kilogram of body weight. More preferably, PAH or a pharmaceutically acceptable salt thereof, preferably aminohippurate sodium, is administered in an amount of about 75 mg to about 200 mg per kilogram of body weight, for example in an amount of about 75, 80, 85, 90, 95, 100, 105, 110, 115, 120, 125, 130, 135, 140, 145, 150, 155, 160, 165, 170, 175, 180, 185, 190, 195, or 200 mg per kilogram of body weight. Most preferably, PAH or a pharmaceutically acceptable salt thereof, preferably aminohippurate sodium, is administered in an amount of about 80 mg to about 160 mg per kilogram of body weight, for example in an amount of about 80, 85, 90, 95, 100, 105, 110, 115, 120, 125, 130, 135, 140, 145, 150, 155, 160 mg per kilogram of body weight. Generally, PAH or a pharmaceutically acceptable salt thereof, preferably aminohippurate sodium, is administered in a larger quantity than the (co-administered) therapeutic or diagnostic compound.

For instance, the therapeutic or diagnostic compound and PAH or a pharmaceutically acceptable salt thereof, preferably aminohippurate sodium, are administered in a ratio of about 1/1.000.000 to 1/10 (w/w), preferably of about 1/500.000 to 1/100 (w/w), more preferably from about 1/250.000 to about 1/500 (w/w).

In a preferred method of the present invention, the therapeutic or diagnostic compound and PAH or a pharmaceutically acceptable salt thereof, preferably aminohippurate sodium, are administered in a ratio of about 1/250.000 to about 1/5.000 (w/w), for example in a ratio of about 1/250.000, 1/200.000, 1/150.000, 1/100.000, or 1/50.000 to about 1/5.000 (w/w), more preferably in a ratio of about 1/240.000 to about 1/8.000 (w/w), for example in a ratio of about 1/240.000, 1/230.000, 1/220.000, 1/210.000, 1/200.000, 1/190.000, 1/180.000, 1/170.000, 1/160.000, 1/150.000, 1/140.000, 1/130.000, 1/120.000, 1/110.000, 1/100.000, 1/90.000, 1/80.000, 1/70.000, 1/60.000, 1/50.000, 1/40.000, 1/30.000, 1/20.000, 1/19.000, 1/18.000, 1/17.000, 1/16.000, 1/15.000, 1/14.000, 1/13.000, 1/12.000, 1/11.000, 1/10.000, 1/9.000, or 1/8.000 (w/w). In a further preferred method, the therapeutic and/or diagnostic compound(s) and PAH or a pharmaceutically acceptable salt thereof, preferably aminohippurate sodium, are administered in a ratio of about 1/100.000 to about 1/10.000 (w/w), for example in a ratio of about 1/100.000, 1/95.000, 1/90.000, 1/85.000, 1/80.000, 1/75.000, 1/70.000, 1/65.000, 1/60.000 1/55.000, 1/50.000, 1/45.000, 1/40.000, 1/35.000, 1/30.000, 1/25.000, 1/20.000, 1/15.000, 1/10.000 (w/w). In a further preferred method, the therapeutic and/or diagnostic compound(s) and PAH or a pharmaceutically acceptable salt thereof, preferably aminohippurate sodium, are administered in a ratio of about 1/50.000 to about 1/40.000 (w/w), for example in a ratio of about 1/50.000, 1/49.000, 1/48.000, 1/47.000, 1/46.000, 1/45.000, 1/44.000, 1/43.000, 1/42.000, 1/41.000, 1/40.000 (w/w).

In another embodiment of the present invention para-aminohippuric acid (PAH) or a pharmaceutically acceptable salt thereof, preferably aminohippurate sodium, is administered in the method of the present invention in combination with a further substance which reduces nephrotoxic side effects of radiolabeled therapeutic and diagnostic compounds. In a preferred embodiment, the substance reducing nephrotoxic side effects of radiolabeled therapeutic and diagnostic compounds other than PAH is selected from amino acids, gelatine, Amifostine, albumin-derived peptides, PSMA-binding molecules, such as PMPA, vitamins. The substance(s) reducing nephrotoxic side effects of radiolabeled therapeutic and diagnostic compounds other than PAH may be administered prior and/or during and/or after administration of PAH or a pharmaceutically acceptable salt thereof, preferably aminohippurate sodium.

The inventive pharmaceutical compositions or kits may be administered to a subject in need thereof several times a day, daily, every other day, weekly, or monthly. Preferably, treatment, diagnosis or prophylaxis is effected with an effective dose of the inventive pharmaceutical compositions or kits.

In the use in the method of the present invention, PAH or a pharmaceutically acceptable salt thereof, preferably aminohippurate sodium, may be administered to a subject prior and/or during and/or after administration of the radiolabeled therapeutic or diagnostic compound, pharmaceutical composition or kit, respectively. For example, PAH or a pharmaceutically acceptable salt thereof, preferably aminohippurate sodium, is administered prior to administration of the radiolabeled therapeutic or diagnostic compound, pharmaceutical composition or kit. In a preferred method of the present invention, PAH or a pharmaceutically acceptable salt thereof, preferably aminohippurate sodium, is administered prior to administration of a pharmaceutical composition or kit of the present invention, as defined above, i.e. PAH or a pharmaceutically acceptable salt thereof, preferably aminohippurate sodium. PAH or a pharmaceutically acceptable salt thereof, preferably aminohippurate sodium may alternatively be administered prior and during or prior and after administration of the radiolabeled therapeutic or diagnostic compound. The pre-administration of PAH may be about 60 min, 30 min, 10 min or 5 min prior administration of the therapeutic or diagnostic compound, pharmaceutical composition or kit, preferably PAH is administered about 0.5-5h or 10-60 min prior to prior administration of the therapeutic or diagnostic compound, pharmaceutical composition or kit, respectively. In certain embodiments PAH may be administered prior to the administration of the therapeutic or diagnostic compound and thereafter, e.g. 0.5-5h or 10-60 min thereafter, or prior, during and thereafter.

In the use in the method of the present invention, PAH or a pharmaceutically acceptable salt thereof, preferably aminohippurate sodium, is preferably administered a buffered aqueous solution, e.g. an isotonic or hypertonic solution, e.g. water for injection (WFI) or NaCl solution, e.g. a 20% NaCl solution.

In the use in the method of the present invention, PAH, the pharmaceutical compositions or kits are typically administered parenterally. Administration may preferably be accomplished systemically, for instance by intravasal, intravenous (i.v.), subcutaneous, intramuscular or intradermal injection. Alternatively, administration may be accomplished locally, for instance by intra-tumoral injection.

### Figures

Figure 1 shows the kidney uptake of [¹⁷⁷Lu-DOTA°-Tyr3]-octreotide with co-infusion of amino acids and PAH over time, wherein 0.9% NaCl is used as control.
Figure 2 shows the reduction of [¹⁷⁷Lu-DOTA°-Tyr3]-octreotide uptake in kidneys compared to baseline (0.9% NaCl infusion) in percent with co-infusion of amino acids and PAH over time.
Figure 3 shows the percent injected dose of [¹⁷⁷Lu-DOTA°-Tyr3]-octreotide in the kidney with co-injection of 0.9% NaCl, Lys/Arg and PAH at an early time point of 0.5 h p.i.
Figure 4 shows the percent injected dose of [¹⁷⁷Lu-DOTA°-Tyr3]-octreotide in the kidney with co-injection of 0.9% NaCl, Lys/Arg and PAH at late time point of 24 h p.i.
Figure 5 shows the *ex-vivo* organ uptake of [¹⁷⁷Lu-DOTA°-Tyr3]-octreotide 5 min and 60 min p.i. (N=5). PAH significantly reduced the uptake in the kidneys even at early time point. (^{∗∗}P≤0.005, ^{∗∗∗∗}*P*≤0.0005).
Figure 6 shows percentage of injected radioactivity present in kidneys at different time points after injection (0.5, 1, 4, 24 h) of different radiolabeled (Lu-177) agents (DOTA-RGD, Affilin, DOTA-Sargastrin, DOTA-JR11) with co-injection of PAH (200 mg/mL) and 0.9% NaCl, respectively.
Figure 7 shows percentage of injected radioactivity present in blood at different time points after injection (0.5, 1, 4, 24 h) of different radiolabeled (Lu-177) agents (DOTA-RGD, Affilin, DOTA-Sargastrin, DOTA-JR11) with co-injection of PAH (200 mg/mL) and 0.9% NaCl, respectively.
Figure 8 shows percentage of injected radioactivity present in kidneys (upper lane) and blood (lower lane) at different time points after injection (0.1, 0.5, 1, 2, 4 h) of ⁶⁸Ga-labeled HBED-CC-PSMA-11 with co-injection of PAH (200 mg/mL) and 0.9% NaCl, respectively.
Figure 9 graphically shows the results of a biodistribution analysis of ¹⁷⁷Lu-DOTATOC given intravenously to mice after intraperitoneal injection of PAH or physiological saline solution.
Figure 10 graphically shows the results of a biodistribution analysis of ^{99m}Tc-Etarfolatide given intravenously to mice after intraperitoneal injection of PAH or physiological saline solution.
Figure 11 shows a comparative bar diagram of three groups of rats injected with ¹⁷⁷Lu-PSMA I&T, injected with Saline VE, Probenecid KP2 or PAH KP1 solution, in % of injected radioactivity in the kidneys.
Figure 12 shows a comparative bar diagram of three groups of rats injected with ¹⁷⁷Lu-PSMA I&T, injected with Saline VE, Probenecid KP2 or PAH KP1 solution, in % of injected radioactivity in the left heart ventricle.
Figure 13 shows a comparative bar diagram of three groups of rats injected with ¹⁷⁷Lu-PSMA I&T, injected with Saline VE, Probenecid KP2 or PAH KP1 solution, in % of injected radioactivity in the left renal medulla.
Figure 14 shows a comparative bar diagram of three groups of rats injected with ¹⁷⁷Lu-PSMA I&T, injected with Saline VE, Probenecid KP2 or PAH KP1 solution, in % of injected radioactivity in the renal cortex.

### Examples

The following data indicate that co-medication with sodium aminohippurate solutions (PAH) have a significant effect in reducing renal retention and uptake of variou radiopharmaceuticals, comprising various carrier molecules, various chelators and various radionuclides, respectively, and that PAH administration is much more effective than Lysine and Arginine infusions. Further, it was found that bioavailability of radiopharmaceuticals is enhanced, and the tumor uptake of radiopharmaceuticals is increased by co-medication with PAH.

### Experimental protocols (Examples 1 and 2):

### SPECT/CT Experiments:

Determination of kidney biodistribution by volumes of interest (VOIs) in the three-dimensional radioactive images, acquired in the six groups of 3 animals per kidney protection agent with [177Lu-DOTA°-Tyr3]-octreotide in healthy Wistar rats by Single Photon Emission Computed Tomography (SPECT). 50 MBq of [¹⁷⁷Lu-DOTA°-Tyr3]-octreotide was co-administered with amino acid solution (L-Arg and L-Lys) - 200 mg/mL in water for injection, sodium para-aminohippurate solution - 200 mg/mL in water for injection (PAH) and NaCl 0.9% solution as reference. Imaging acquisition was performed after 0.5 h, 1 h, 4 h, 8 h, 24 h p.i.

### Ex-Vivo Organ Distribution:

All animals were pre-injected 10 min before the radiotracer injection with 1.0 mL of NaCl, Arg-Lys or PAH. The injection solutions for the biodistribution were prepared as a mix of 12 µL [¹⁷⁷Lu]Lu-DOTA-TOC, 1.5 mL NaCl or Arg-Lys or PAH.

The injection solutions for the SPECT animals were prepared as a mix of 80 µL [177Lu]Lu-DOTA-TOC, 1.5 mL NaCl or Arg-Lys or PAH. The animals were injected and sacrificed after 5 min or 60 min. Organs and tissue of interest were harvested and measured for activity. The male Wistar rats had an average body weight of 210±12 g (5 min) and 218±13 g (60 min). The average injected activity was 4.49± 0.38 MBq/kg body weight (5 min) and 159 ± 13 MBq/kg body weight (5 min) for the SPECT and 4.19 ± 0.57 MBq/kg body weight (60 min) or 139 ± 8 MBq/kg body weight for the SPECT animals (60 min).

For statistical analysis Microsoft Excel 2010 and Graphpad Prism 6.05 were employed. Data are presented as median and [25% and 75% percentile] and mean and standard deviation (SD) or standard error of mean (SEM). For comparison, ordinary one way ANOVA and Tukey's multiple comparisons test, two-sample two-tailed t-test were employed. Significance was accepted with P<0.05.

### Example 1: Kidneys uptake of [¹⁷⁷Lu-DOTA°-Tyr3]-octreotide

The kidneys uptake of [¹⁷⁷Lu-DOTA°-Tyr3]-octreotide was determined by quantitative small animal SPECT in rats with co-infusion of 0.9% NaCl (control), Lys/Arg (250 mg Lys/ 250 mg Arg) and PAH (500 mg), respectively. The results are presented in Tables 1 to 3.

**Table 1: Kidneys radioactivity [in % of injected radioactivity, decay corrected data] with co-infusion of 0.9% NaCl (control)**

| Time (hour) | rat04 | rat05 | rat06 | average | stdev |
|---|---|---|---|---|---|
| 0.5 | 21.60% | 4.80% | 13.50% | 13.30% | 8.40% |
| 1 | 19.70% | 3.30% | 13.70% | 12.20% | 8.30% |
| 4 | 2.70% | 2.70% | 2.70% | 2.70% | 0.00% |
| 8 | 2.70% | 2.70% | 2.70% | 2.70% | 0.00% |
| 24 | 2.40% | 2.30% | 2.40% | 2.40% | 0.10% |

**Table 2: Kidneys radioactivity [in % of injected radioactivity, decay corrected data] with co-infusion of Lys/Arg (250 mg Lys/ 250 mg Arg)**

| Time (hour) | rat04 | rat05 | rat06 | average | stdev |
|---|---|---|---|---|---|
| 0.5 | 13.30% | 10.20% | 12.50% | 12.00% | 1.60% |
| 1 | 16.20% | 10.50% | 9.60% | 12.10% | 3.60% |
| 4 | 2.60% | 1.60% | 2.10% | 2.10% | 0.50% |
| 8 | 2.40% | 1.60% | 2.00% | 2.00% | 0.40% |
| 24 | 2.30% | 1.50% | 1.80% | 1.90% | 0.40% |

**Table 3: Kidneys radioactivity [in % of injected radioactivity, decay corrected data] with co-infusion of PAH (500 mg)**

| Time (hour) | rat04 | rat05 | rat06 | average | stdev |
|---|---|---|---|---|---|
| 0.5 | 6.10% | 4.60% | 9.10% | 6.60% | 2.30% |
| 1 | 5.00% | 5.00% | 9.80% | 6.60% | 2.80% |
| 4 | 1.30% | 1.30% | 1.60% | 1.40% | 0.20% |
| 8 | 1.30% | 1.20% | 1.10% | 1.20% | 0.10% |
| 24 | 1.10% | 1.20% | 1.10% | 1.20% | 0.00% |

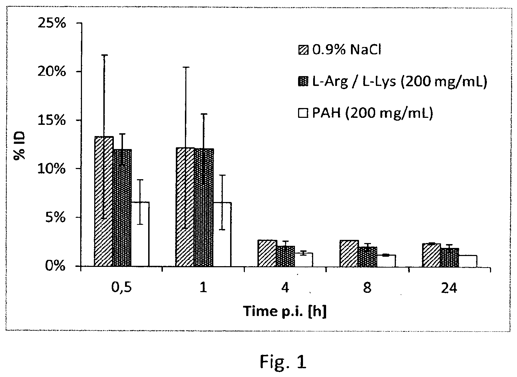

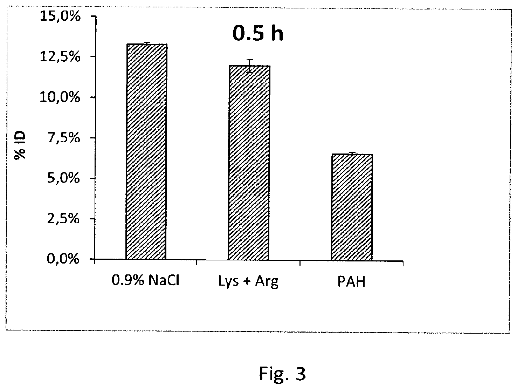

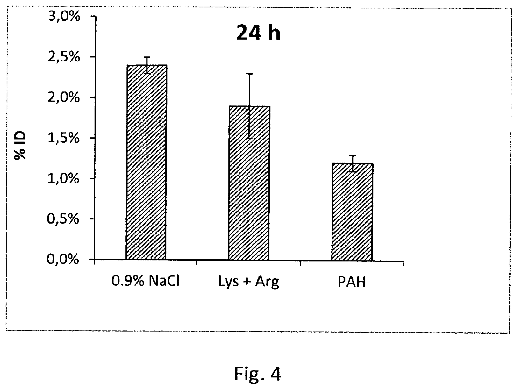

The values presented in Tables 1 to 3 are graphically illustrated in Figures 1 to 4.

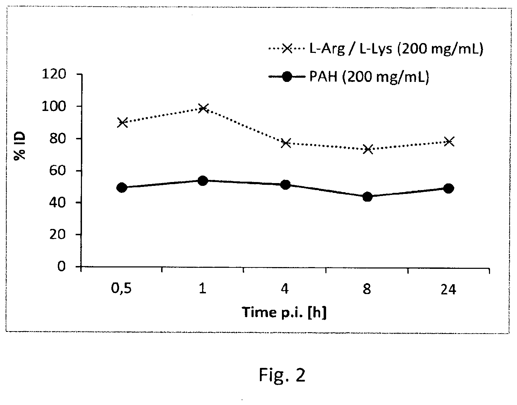

As it is obvious from the presented results, co-infusion of Lys/Arg resulted in kidney uptake reduction of 21% and 26 % after 8 h and 24 h, respectively, with a high variation between individuals (Table 2, Figures 1, 2 and 4). The outcome of amino acid infusion was similar to what is reported in the literature (33%+/-23%, Rolleman EJ et al., supra). In contrast, co-infusion of PAH resulted in a reduction of 56% and 50% at the same time points with a low standard deviation (Table 3, Figures 1 and 2,). Infusions of PAH also result in lower kidney uptakes at early time points. At 0.5 h post injection the Lys/Arg mixture showed only minor reduction of 10% in contrast to PAH which showed a reduction of 50% (Tables 2 and 3, Figures 1-3).

### Example 2: Ex vivo Organ Uptake of [¹⁷⁷Lu-DOTA°-Tyr3]-octreotide

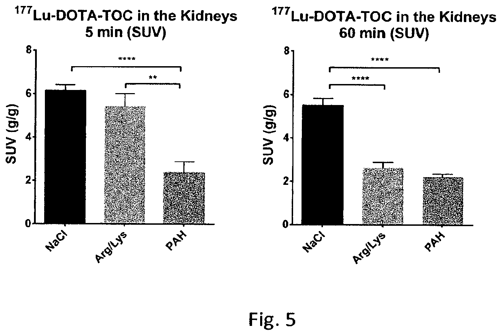

Ex-vivo organ concentration of [¹⁷⁷Lu-DOTA°-Tyr3]-octreotide was determined at an early time points of 5 min and 60 min p.i. according to the above protocol. The results are graphically illustrated in Figure 5. Even at early time points, the uptake of [¹⁷⁷Lu-DOTA°-Tyr3]-octreotide was significantly reduced (P 0.005) with respect to NaCl infusions when using PAH-infusions as a co-medication in comparison to Lys/Arg.

In summary, the above results demonstrate that sodium aminohippurate solutions have a significant higher effect in reducing renal retention and uptake of [¹⁷⁷Lu-DOTA°-Tyr3]-octreotide than a combination of Lysine and Arginine known from the prior art.

### Experimental Protocols (Examples 3-5)

The imaging study was designed to evaluate the effectiveness of PAH in reducing the renal uptake of peptides labeled with either ¹⁷⁷Lu or ⁶⁸Ga which were coordinated by chelators of distinct structures and binding scaffolds. The tracer distribution of the radiolabeled test compounds were performed in two distinct cohorts of healthy Wistar rats with focus on the kidney uptake/clearance and blood levels. One cohort was administered with the ¹⁷⁷Lu/⁶⁸Ga-tracer in combination with saline for control (MBq/kg and mg/kg to be defined individually), while the other cohort first received an intraperitoneal injection of PAH (10 min prior to inj.) before administration of the ¹⁷⁷Lu/⁶⁸Ga-peptide together with two intravenous injections of PAH solution. The kidney clearance and the overall pharmacokinetic of each ¹⁷⁷Lu/⁶⁸Ga-peptide (administered with or without pre-dose of PAH) were assessed using PET or SPECT. Each cohort comprises at least 3 rats for statistic matters.

¹⁷⁷Lu-peptides: ¹⁷⁷Lu labeled compounds provided by ITG were tested for radiochemical purity upon receipt using iTLC. Testing parameters were provided for each compound, RCP > 95%. The nominal dose level, concentration and volume for each ¹⁷⁷Lu-peptide to be investigated are summarized in Table 4.

**Table 4**

| ¹⁷⁷Lu-test compound | **Saline Group (0.9% NaCl)** | **Ki group (200 mg/mL)** |
|---|---|---|
| No. animals | 3 | 3 |
| i.p. injection @ 0 min | 2.0 mL | 2.0 mL |
| Bolus injection i.v. + ¹⁷⁷Lu-test compound @ 10 min | 0.5 mL + 50 MBq ¹⁷⁷Lu-compound (0.1 - 0.2 mL) | 0.5 mL + 50 MBq ¹⁷⁷Lu-compound (0.1 - 0.2mL) |
| Bolus injection i.v. @ 11 min | 0.5 mL | 0.5 mL |
| SPECT/CT (ROI/VOI) | Kidney + Blood (Heart) | Kidney + Blood (Heart) |
| Measurement time points | 0.5h, 1h, 4h, 24h p.i. | 0.5h, 1h, 4h, 24h p.i. |

**⁶⁸Ga-peptides:** ⁶⁸Ga labeled compounds were prepared directly prior to application and were tested for radiochemical purity using iTLC. (Labeling and QC testing parameters were provided for each compound, RCP >95%). The nominal dose level, concentration and volume for each ⁶⁸Ga-peptide to be investigated are summarized in Table 5.

**Table 5**

| ⁶⁸Ga-test compound | **Saline Group (0.9% NaCl)** | **Ki group (200 mg/mL)** |
|---|---|---|
| No. animals | 3 | 3 |
| i.p. injection @ 0 min | 2.0 mL | 2.0 mL |
| Bolus injection i.v. + ⁶⁸Ga-test compound @ 10 min | 0.5 mL + 35 MBq ⁶⁸Ga-compound (0.1 - 0.2 mL) | 0.5 mL + 35 MBq ⁶⁸Ga-compound (0.1 - 0.2mL) |
| Bolus injection i.v. @ 11 min | 0.5 mL | 0.5 mL |
| PET/CT (ROI/VOI) | Kidney + Blood (Heart) | Kidney + Blood (Heart) |
| Measurement scheme | Dynamic measurement from 0-60 min p.i. | Dynamic measurement from 0-60 min p.i. |
| | Static measurement at 2h and 4 h p.i. | Static measurement at 2h and 4 h p.i. |

### Example 3: Reduction of kidney uptake of various radiolabeled compounds by concomitant administration of PAH

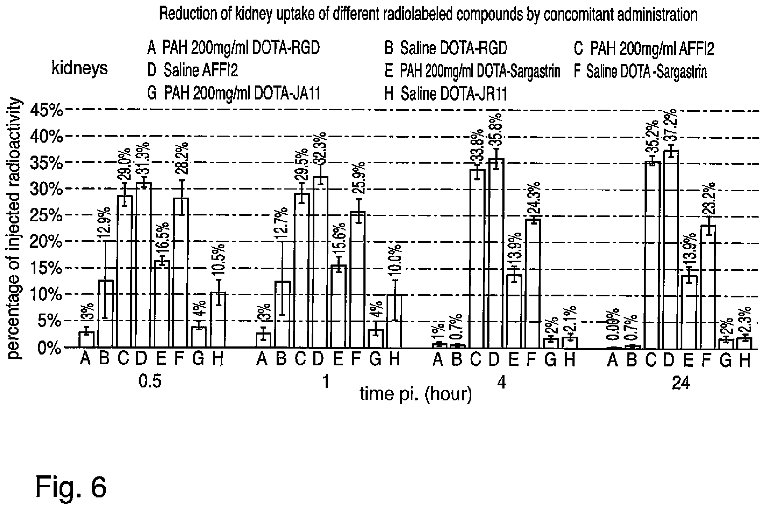

Mice were injected with four different molecules conjugated with the cyclic chelator DOTA and radiolabeled with therapeutic radionuclide Lutetium-177: ¹⁷⁷Lu-DOTA-RGD, ¹¹⁷Lu-DOTA-Affilin, ¹⁷⁷Lu-DOTA-Sargastrin, and ¹⁷⁷Lu-DOTA-JR11, respectively, and co-injected with PAH (200 mg/mL) or saline (0.9% NaCl) as a control. The percentage of injected radioactivity present in the kidneys over time was determined according to the above protocol. The results are graphically illustrated in Figure 6.

According to Figure 6, there is a significant effect in reduction of kidney uptake by concomitant administration of PAH with all compounds tested, in particular with small peptides, such as DOTA-RGD, DOTA-JR11 and DOTA-Sargastrin. It is also obvious from Figure 6 that the effect of reduction of kidney uptake is already present at early time points of 0.5 to 1 h after administration.

Therefore, the above experimental results show that administration of PAH reduces kidney uptake of various kinds of radiolabeled compounds, i.e. DOTA-linked peptides, peptidomimetics etc., which are accumulated in kidney via different mechanisms.

### Example 4: Influence of PAH on the blood activities of different radiolabels compounds

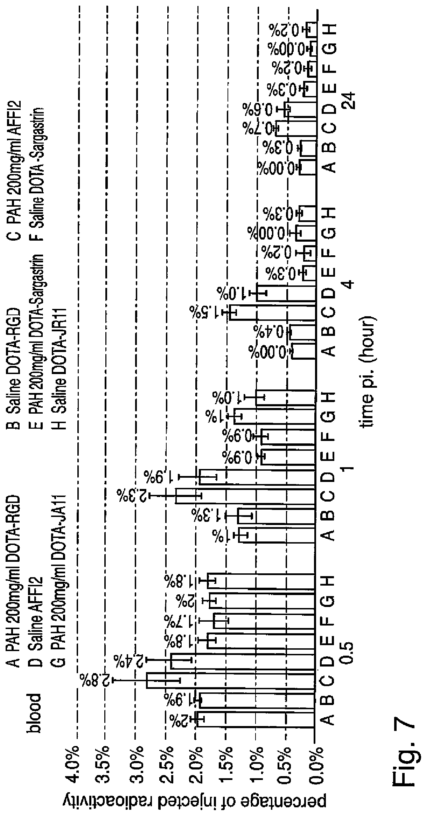

Mice were injected with four different molecules conjugated with the cyclic chelator DOTA and radiolabeled with therapeutic radionuclide Lutetium-177: ¹⁷⁷Lu-DOTA-RGD, ¹⁷⁷Lu-DOTA-Affilin, ¹⁷⁷Lu-DOTA-Sargastrin, and ¹⁷⁷Lu-DOTA-JR11, respectively, and co-injected with PAH (200 mg/mL) or saline (0.9% NaCl) as a control. The percentage of injected radioactivity present in blood over time was determined according to the above protocol. The results are graphically illustrated in Figure 7.

According to Figure 7, there is a significant increase of blood activity by co-injected PAH for Affilin. Therefore, while kidney uptake of Affilin is less prominently reduced by PAH versus the other compounds tested (cf. Figure 6), the data indicate that at the same time blood activities of Affilin are increased by co-injection of PAH resulting in an enhanced bioavailability of the radiopharmaceutical.

### Example 5: Influence of PAH on the kidney uptake and blood activity of ⁶⁸Ga-labeled PSMA-11

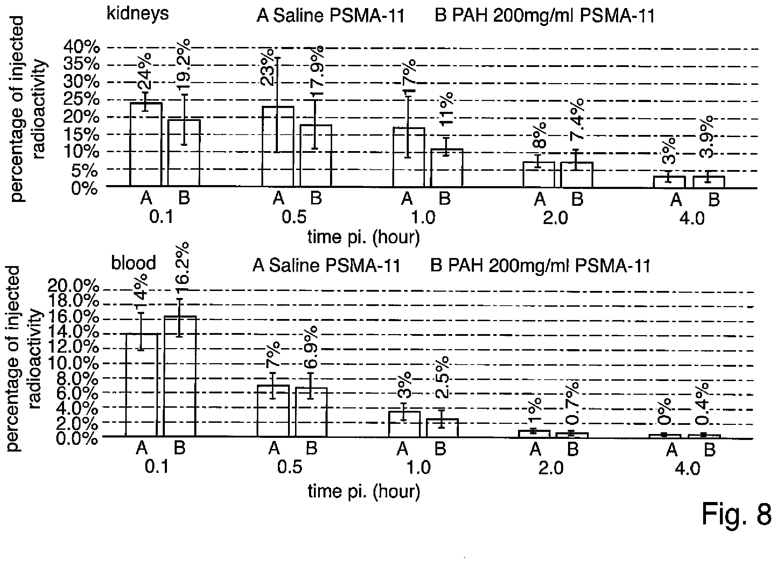

Mice were injected with diagnostic ⁶⁸Ga-labeled PSMA-11 conjugated with acyclic chelator HBED-CC, and co-injected with PAH (200 mg/mL) or saline (0.9% NaCl) as a control. The percentage of injected radioactivity present in kidney (upper lane) and blood (lower lane) over time was determined according to the above protocol. The results are graphically illustrated in Figure 8.

As shown in Figure 8, there is a significant reduction of kidney uptake of ⁶⁸Ga-labeled HBED-CC conjugated PSMA-11 by concomitant administration of PAH. It is also obvious from Figure 8 that the effect of reduction of kidney uptake is already present at early time points of 0.1, 0.5 and 1 h after administration.

Therefore, the above experimental results of Examples 3-5 show that administration of PAH reduces kidney uptake of various kinds of radiolabeled compounds having different carrier molecules (peptide, peptidomimetics, recombinant proteins), different chelators (cyclic chelator, acyclic chelator), and different radionuclides (therapeutic radionuclide, diagnostic radionuclide), respectively, and can therefore suitably be used for the reduction of nephrotoxic side effects of a number of radiolabeled diagnostic and therapeutic compounds.

### Example 6: Comparative biodistribution analysis of ¹⁷⁷Lu-DOTATOC given intravenously to mice after intraperitoneal injection of PAH or physiological saline solution

Somatostatin receptor positive pancreatic tumor bearing CD1 nude mice (AR24J) received an i.p. injection of 50 µL NaCl 0.9% (group A) or 50 µL PAH 20% (group B) exactly 10 minutes before i.v. injection of ¹⁷⁷Lu-DOTATOC/NaCl (group A) or ¹⁷⁷Lu-DOTATOC/PAH (group B) via the retro-orbital venous sinus. The nominal dose level, concentration and volume are summarized in Table 6.

**Table 6**

| Test item | ¹⁷⁷Lu-DOTATOC/NaCl 0.9% | ¹⁷⁷Lu-DOTATOC/PAH 20% |
|---|---|---|
| Mice strain | AR42J tumor mouse model | AR42J tumor mouse model |
| Pre-dosing 10 min before ¹⁷⁷Lu-DOTATOC injection | NaCl 0.9 % (50 µL) | PAH 20% (50 µL) |
| ¹⁷⁷Lu-DOTATOC injection volume (mL)^{∗} | 0.2 | 0.2 |
| Concentration (MBq/mL)^{∗∗} | 7.4-5.4 | 7.4-5.4 |
| Dose (MBq/mouse)^{∗∗} | 1.5-1.1 | 1.5-1.1 |
| Number of mice/ termination time point | 3 | 3 |
| Number of termination time points | 4 | 4 |
| Number of mice | 12 | 12 |

| | | |
|---|---|---|
| ^{∗} Individual dose volume was calculated using individual body weight recorded on the day of treatment, and individual volumes and exact time of treatment were recorded and kept in study files ^{∗∗} Range of concentration and dose taking into account the radioactive decay of ¹⁷⁷Lu over a period of 3 days from the day of delivery | | |

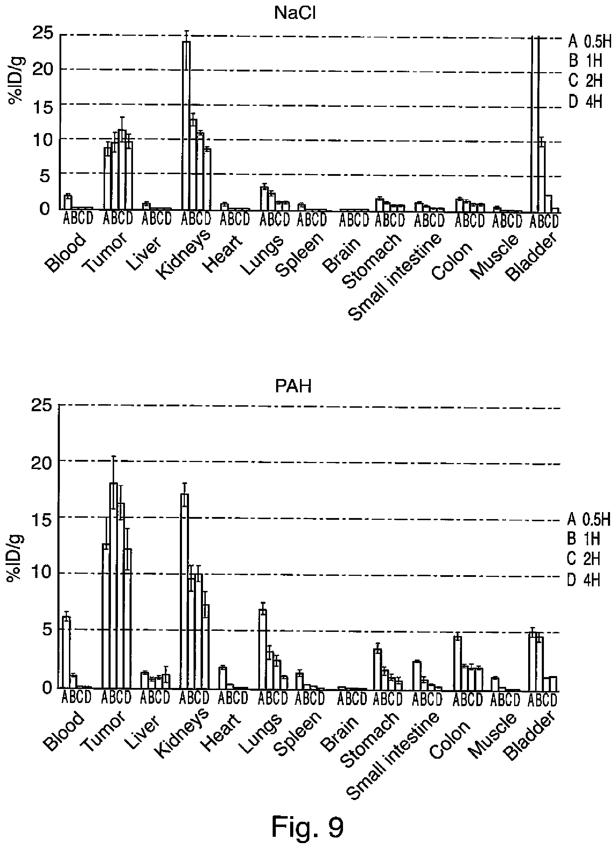

Mice (3 animals per group) were sacrificed at 0.5h, 1h, 2h, and 4h, respectively. The organs were quickly rinsed in 0.9% NaCl and dried before being weighed and counted to eliminate possible contaminating blood. Following organs/tissues were sampled or taken, weighed and counted for ¹⁷⁷Lu: blood, tumor, kidneys, liver, bladder (empty), heart, spleen, lungs, brain, muscle, stomach (without contents), small intestine (without contents), colon (without contents) residual carcass. Data from organ/tissue counting are expressed as percentage of injected dose (%ID/g)). The organs/tissues distribution results for ¹⁷⁷Lu-DOTATOC in NaCl 0,9% (Group A), as well as the organs/tissues distribution results for ¹⁷⁷Lu-DOTATOC with PAH (Group B) are graphically presented in Figure 9.

According to Figure 9 there is a significant decrease of ¹⁷⁷Lu-DOTATOC uptake in kidneys in group B (lower panel) which received ¹⁷⁷Lu-DOTATOC with PAH compared to group A (upper panel) which received ¹⁷⁷Lu-DOTATOC in NaCl. The decrease in renal uptake of the radiolabeled compound is particularly evident at early stages (0.5h, 1h) after injection of ¹⁷⁷Lu-DOTATOC. At the same time, ¹⁷⁷Lu-DOTATOC level of group B in tumor and blood are significantly increased compared to group A. Without wishing to be bound by specific theory, it is hypothesized that blockage of elimination of ¹⁷⁷Lu-DOTATOC via the kidneys by PAH leads to a prolongation of blood circulation of ¹⁷⁷Lu-DOTATOC and thus to an increased accumulation of the radiolabeled compound also at the tumor site. In summary, the results presented show an enhanced bioavailability of ¹¹⁷Lu-DOTATOC which received ¹⁷⁷Lu-DOTATOC with PAH compared to group A which received ¹⁷⁷Lu-DOTATOC in NaCl.

### Example 7: Comparative biodistribution analysis of ^{99m}Tc-Etarfolatide given in combination with PAH or physiological saline solution (not according to the invention)

### 7.1 Radiolabeling of Etarfolatide

### ^{99m}Tc-Etarfolatide has the following chemical structure:

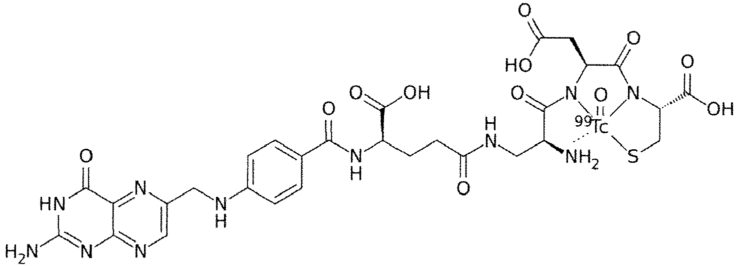

The radiolabelling was carried out by a method derived from the teaching of Kim et al. (Ann Nucl Med 2016; 30:369-379). The ligand exchange method was employed by using tartrate as a co-ligand. In an eppendorf, 100 µg of Etarfolatide, 50 µl of tartrate solution (20 mg/50 µl in millipore water), and 80 µl of SnCl₂ dihydrate solution were added (1 mg/ml in 0.01 M HCl solution). In a lead shielded fume hood, about 750 MBq (20 mCi) of freshly eluted ^{99m}Tc-pertechnetate was added, and the reaction vial was heated in a water bath for 30 min at 100 °C and cooled to room temperature.

Radiolabeling efficiency and stability was determined using: (i) Instant thin layer chromatography-silica gel (ITLC-SG) with water (Rf of ^{99m}Tc-Etarfolatide and free pertechnetate=0.9-1.0; Rf of colloid=0.0-0.1) and acetone (Rf of free pertechnetate =0.9-1.0; Rf of colloid and ^{99m}Tc-Etarfolatide = 0.0-0.1) as the mobile phases and (ii) RP-HPLC: Solvent A: 0.1% TFA in H₂O. Solvent B: 0.1% TFA in AcCN; Gradient Elution, flow rate 1 mL/min.

On each day of experimentation, a new radiolabeling procedure was performed with approximately the same ^{99m}Tc activity. RP-HPLC showed RCP = -100%, while TLC showed a maximum colloid formation of 5% or less. Stability of ^{99m}Tc-Etarfolatide was assessed by HPLC at 24 h post-preparation, and showed no loss of radiolabel.

The volume of the radiolabeled folate was measured to be 1300 µL, while the activity was 734 MBq (Day of experimentation: March 4^{th}). A sample corresponding to 300 MBq (i.e. 15 MBq x 20 mice) was prepared. 531 µL were taken from the radiolabeled folate, which was diluted with either 1469 µL Saline or 1469 µL PAH (2000 µL 99mTc-etarfolatide, 100 µL/15 MBq per mouse). Before injection, the sample was filtered through a 0.22 M sterile filter. The injected 15 MBq/mouse corresponds to 2.04 µg Etarfolatide/mouse (injected quantity of Etarfolatide was kept stable throughout the experiment).

Samples of ^{99m}Tc-Etarfolatide diluted with both Saline or PAH were assessed immediately after preparation, as well as 24 h post-dilution, and showed no signs of loss of radiolabel, when assessed by HPLC (100% RCP), however TLC assessment showed that ^{99m}Tc-Etarfolatide/PAH was stable (100% RCP), while ^{99m}Tc-Etarfolatide/saline showed 14% colloid formation.

### 7.2 Preparation of PAH solution

Weigh 2 g PAH sodium salt in a Corning Tube; Addition of 4 mL H₂O; Vortex - partial dissolution of PAH; Addition of 20 µL NaOH (provided by ITG), Vortex - partial dissolution of PAH; Addition of 20 µL NaOH, Vortex - complete dissolution of PAH; Addition of 2 mL H₂O, pH 10; Addition of 80 µL HCL 37% (in 20 µL increments), pH ~6. The final volume of the resulting solution was measuerd and found to be 7100 µL, to which we added 2900 µL (all this in another Corning Tube), for a final solution of 2g PAH in 10 mL H₂O, i.e. 200 mg PAH/mL. After filtration through a 0.22 µM filter, the Corning tube was covered with aluminum foil and cooled.

### 7.3 Biodistribution analysis

### 30 mice (male and female) were randomly assigned to two groups.

Group A: The mice were injected with ^{99m}Tc Etarfolatide diluted in PAH (2.04 µg Etarfolatide/mouse) after having received an IP injection of PAH 10 min prior to radiotracer injection. Five time-points were assessed: 5min, 30min, 1h, 2h, 4h (3 mice per time-point). Injected Doses at each time-point:

| | | Time | | Mouse | Injection (µCi) | Corrected |
|---|---|---|---|---|---|---|
| | 13:14 | | 0 | 5min_1 | 197 | 197 |
| | 13:26 | | 12 | 5min_2 | 194 | 199 |
| 5 min | 14:19 | | 65 | 5min_3 | 166 | 188 |

| | | Time | | Mouse | Injection (µCi) | Corrected |
|---|---|---|---|---|---|---|
| | 12:06 | | 0 | 30min_1 | 249 | 249 |
| | 12:10 | | 4 | 30min_2 | 230 | 232 |
| 30min | 12:14 | | 8 | 30min_3 | 217 | 220 |

| | | Time | | Mouse | Injection (µCi) | Corrected |
|---|---|---|---|---|---|---|
| | 13:46 | | 0 | 60min_1 | 181 | 181 |
| | 14:00 | | 14 | 60min_2 | 181 | 186 |
| 60min | 14:06 | | 20 | 60min_3 | 184 | 191 |

| | | Time | | Mouse | Injection (µCi) | Corrected |
|---|---|---|---|---|---|---|
| | 12:05 | | 0 | 120min_1 | 355 | 355 |
| | 12:13 | | 8 | 120min_2 | 367 | 373 |
| 120min | 12:41 | | 36 | 120min_3 | 317 | 340 |
| | 10:28 | | 0 | 240min_1 | 420 | 420 |
| | 10:33 | | 5 | 240min_2 | 431 | 435 |
| 240min | 10:37 | | 9 | 240min_3 | 417 | 424 |

Group B: The mice were injected with ^{99m}Tc Etarfolatide diluted in saline (2.04 µg Etarfolatide/mouse) after having received an IP injection of saline 10 min prior to radiotracer injection. Five time-points were assessed: 5min, 30min, 1h, 2h, 4h (3 mice per time-point).

Injected Doses at each time-point:

| | | Time | | Mouse | Injection (µCi) | Corrected |
|---|---|---|---|---|---|---|
| | 13:54 | | 0 | 5min_1 | 364 | 364 |
| | 14:03 | | 9 | 5min_2 | 384 | 391 |
| 5 min | 14:28 | | 34 | 5min_3 | 310 | 331 |

| | | Time | | Mouse | Injection (µCi) | Corrected |
|---|---|---|---|---|---|---|
| | 14:03 | | 0 | 30min_1 | 170 | 170 |
| | 14:23 | | 10 | 30min_2 | 155 | 158 |
| 30min | 14:40 | | 37 | 30min_3 | 171 | 184 |

| | | Time | | Mouse | Injection (µCi) | Corrected |
|---|---|---|---|---|---|---|
| | 12:34 | | 0 | 60min_1 | 414 | 414 |
| | 12:43 | | 9 | 60min_2 | 460 | 468 |
| 60min | 13:29 | | 55 | 60min_3 | 378 | 420 |

| | | Time | | Mouse | Injection (µCi) | Corrected |
|---|---|---|---|---|---|---|
| | 11:18 | | 0 | 120min_1 | 326 | 326 |
| | 11:22 | | 4 | 120min_2 | 326 | 329 |
| 120min | 11:26 | | 8 | 120min_3 | 324 | 329 |

| | | Time | | Mouse | Injection (µCi) | Corrected |
|---|---|---|---|---|---|---|
| | 10:50 | | 0 | 240min_1 | 385 | 385 |
| | 11:01 | | 11 | 240min_2 | 364 | 372 |
| 240min | 11:21 | | 31 | 240min_3 | 354 | 376 |

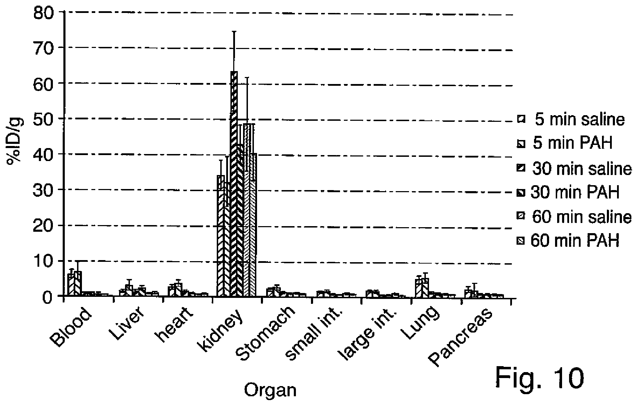

The results of the biodistribution analysis are shown in Figure 10. After 30 min, the decreased renal uptake of ^{99m}Tc-Etarfolatide by the protective effect of PAH becomes prominent. Thus, the effects observed for the ¹⁷⁷Lu-based conjugate molecule are confirmed for a ^{99m}Tc chelating conjugate molecule with ^{99m}Tc having another valence status than tri-valent ¹⁷⁷Lu.

### Example 8: Biodistribution analysis of ¹⁷⁷Lu-labelled PSMA I&T

The chemical structure of "PSMA I&T" is the following

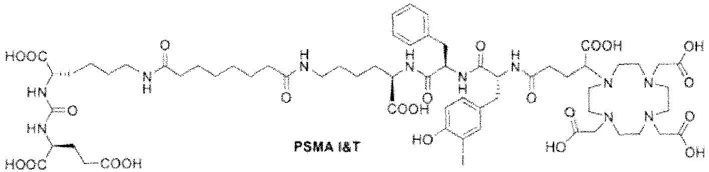

### Test articles for the imaging experiments

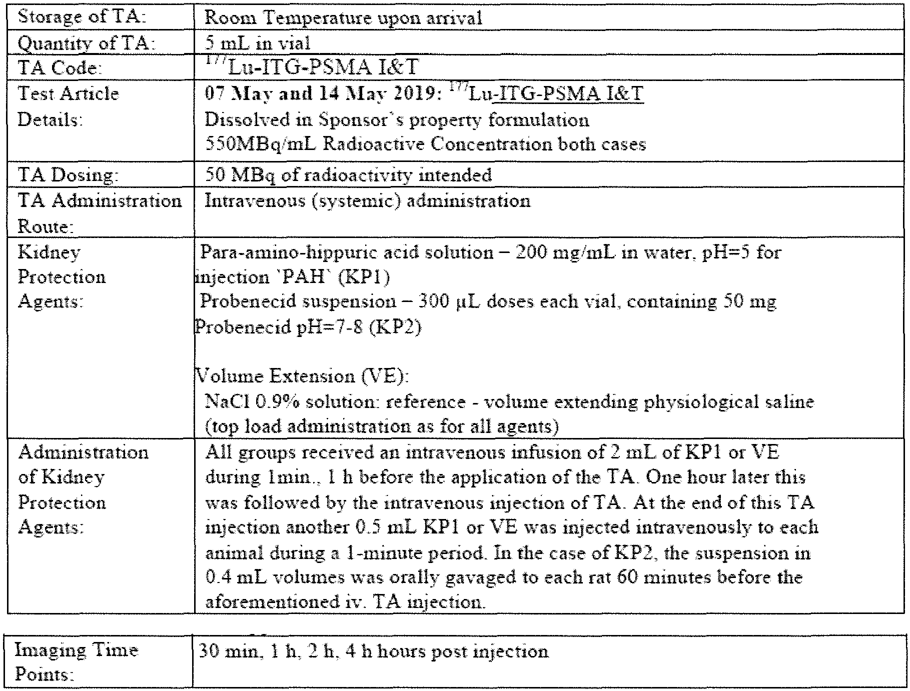

### Measurement systems and parameter settings for the imaging experiments

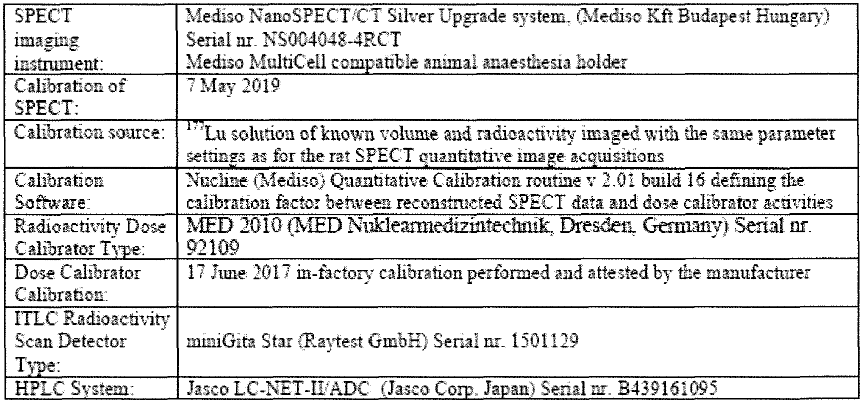

### EXECUTION OF PROTOCOL

The protocol was executed in a way to test the radioactivity concentration of the kidneys in groups of animals injected with one TA but three different KP agents in three separate groups. Thus, the following matrix of experimental groups was formed.

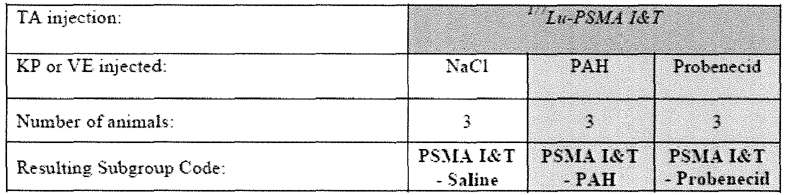

### 1. Data of the PSMA I&T-Probenecid imaging experiment

### 1.1. Rat 1

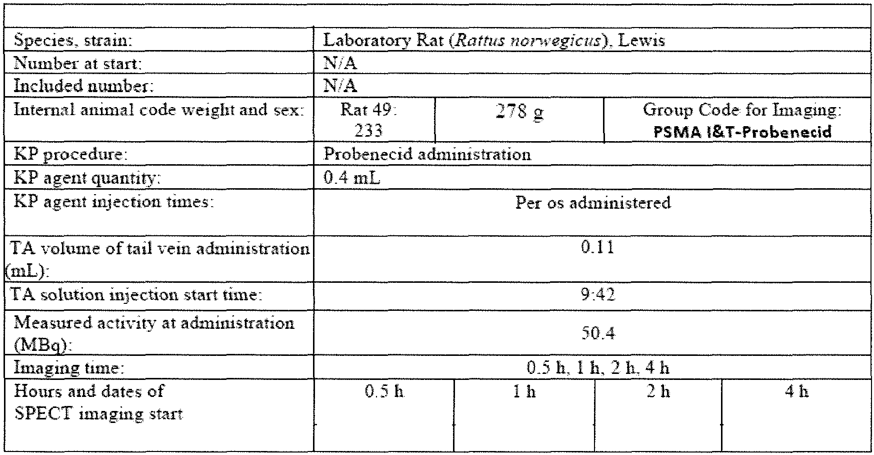

### 1.2. Rat 2

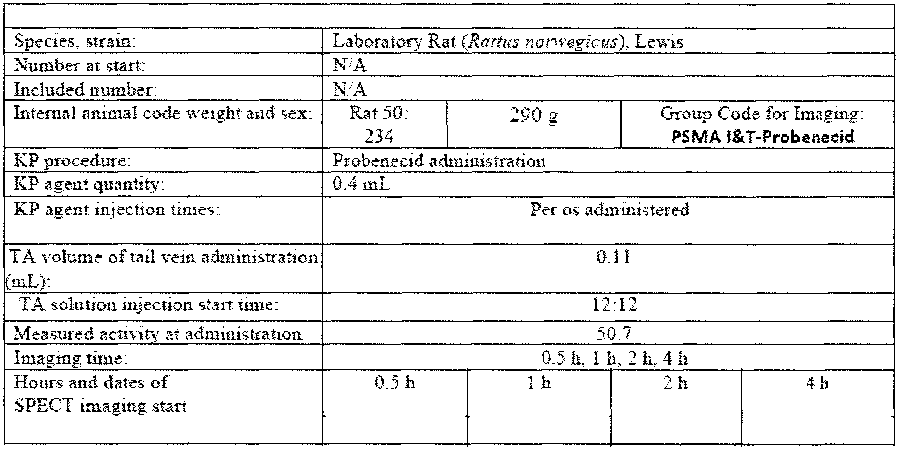

### 1.3.Rat 3

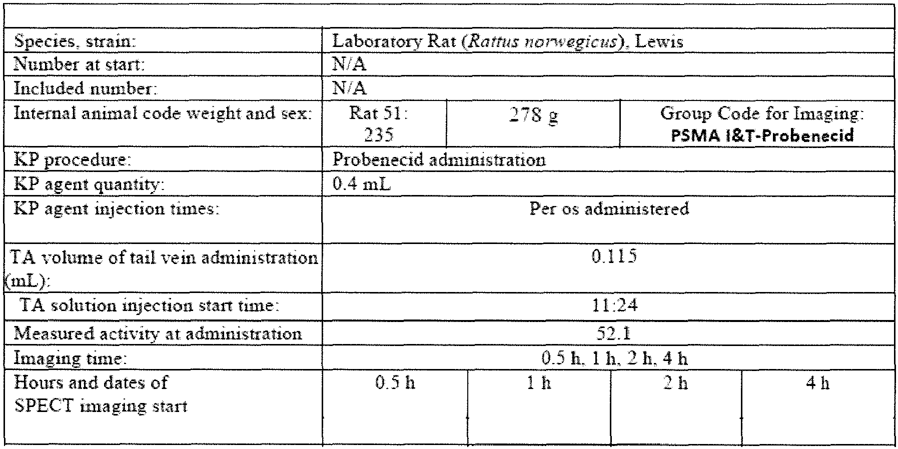

### 2. Data of the PSMA I&T-PAH imaging experiment

### 2.1 Rat 4

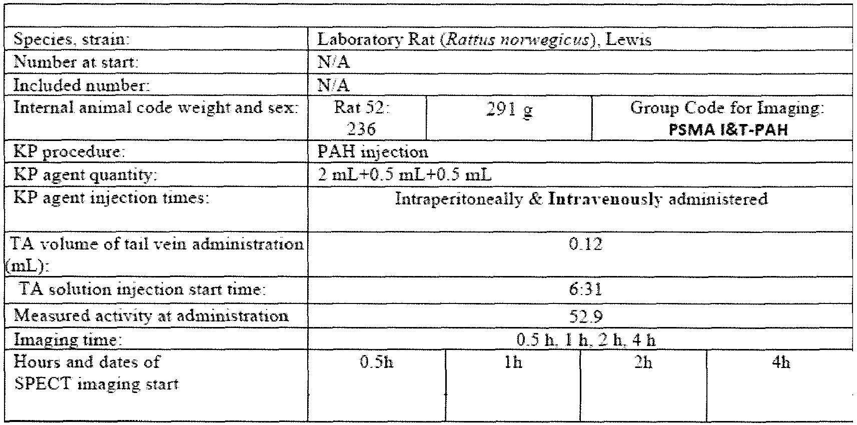

### 2.2 Rat 5

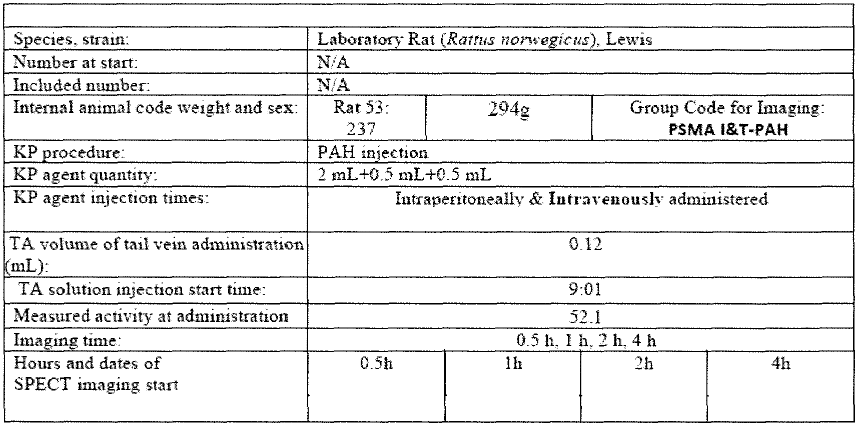

### 2.3 Rat 6

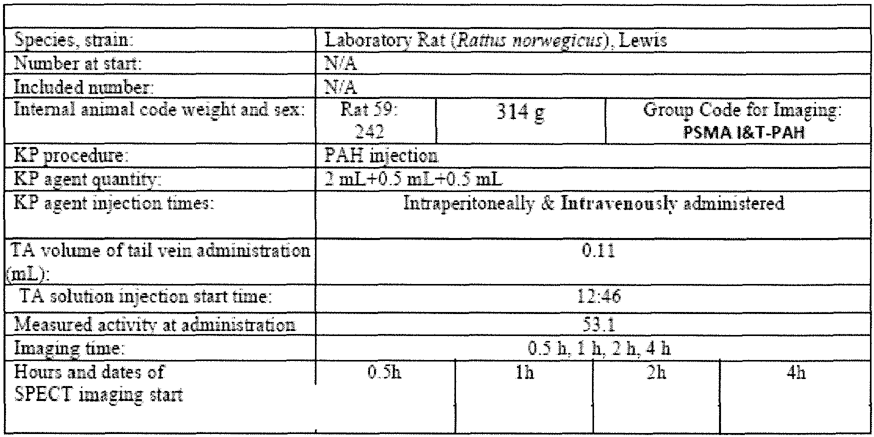

### 3. Data of the PSMA I&T-Saline imaging experiment

### 3.1 Rat 7

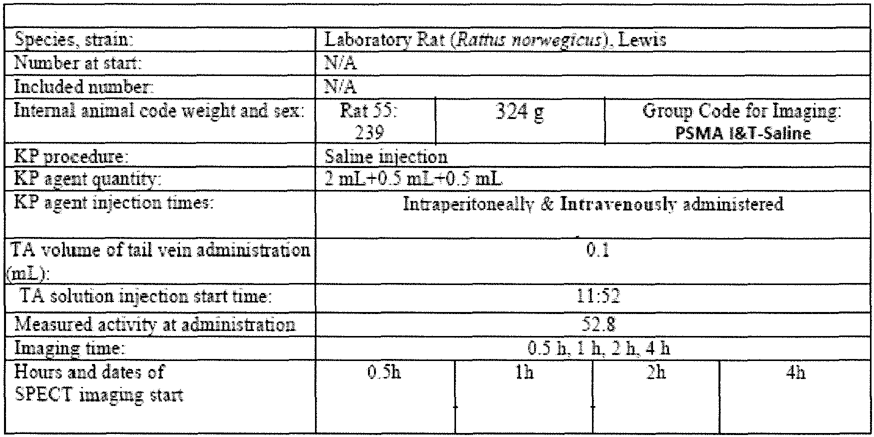

### 3.2 Rat 8

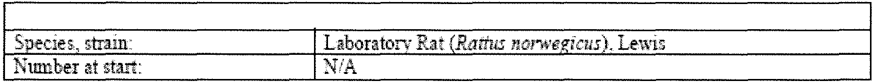

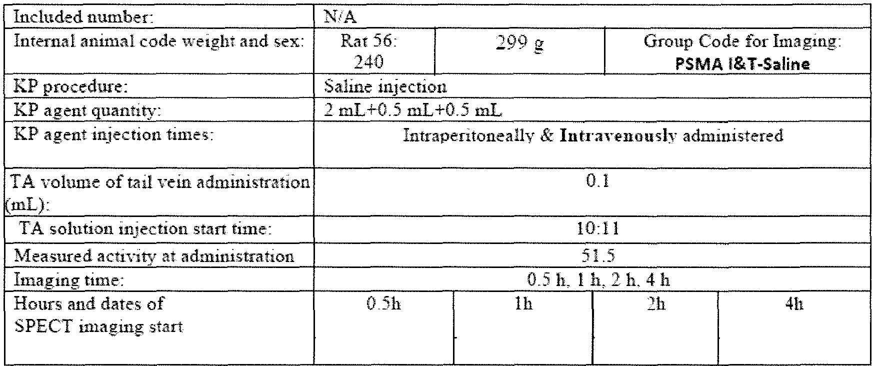

### 3.3 Rat 9

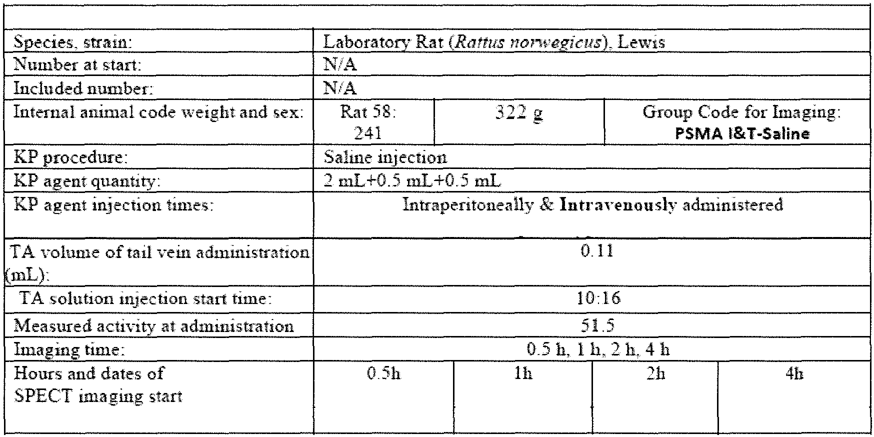

### RESULTS

### PE1: Toxicity observations

Result: No sign of acute immediate and delayed acute toxicity was observed.

### PE2: Radioactivity accumulation over time in the organs

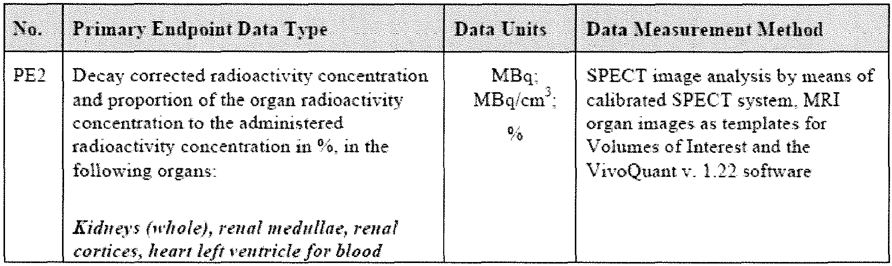

**Result: the following tables per time point** contain the decay corrected radioactivity concentration ratio in the sum of both kidneys in % of the injected whole body radioactivity concentration.

### Results-Table I.:

Kidney radioactivity in percentage of injected radioactivity in the group injected with ^{177Lu}-PSMA I&T and receiving the Probenecid suspension po.

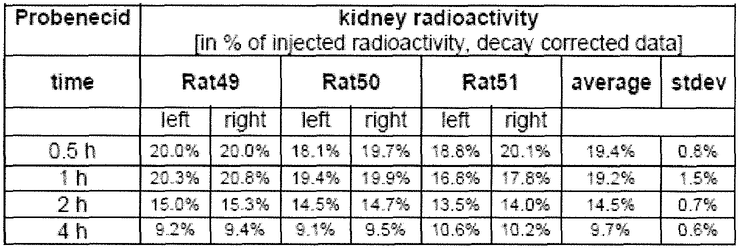

Results-Table II.: Kidney radioactivity in percentage of injected radioactivity in the group injected with ^{177Lu}-PSMA I&T and receiving PAH solution ip. and iv.

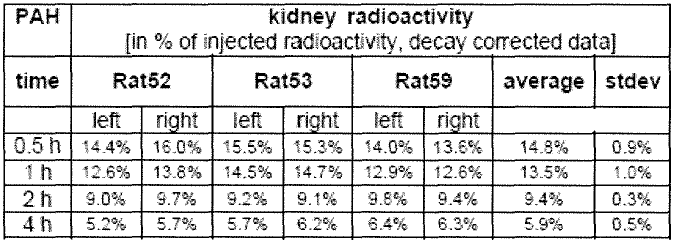

### Results-Table III.:

Kidney radioactivity in percentage of injected radioactivity in the group injected with ^{177Lu}-PSMA I&T and receiving the Saline ip. and iv.

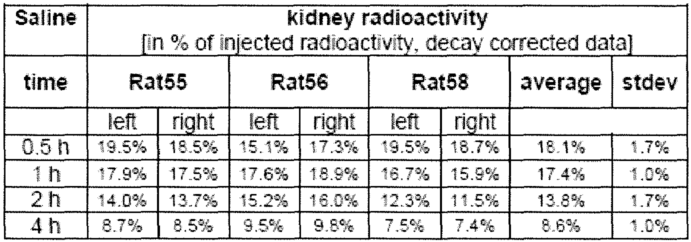

Results-Table IV.: Blood (left ventricle) radioactivity in percentage of injected radioactivity in the group injected with ^{177Lu}-PSMA I&T and receiving the Probenecid suspension po.

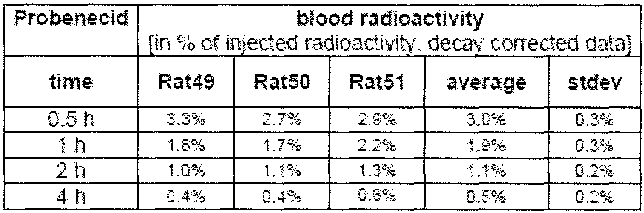

Results-Table V.: Blood (left ventricle) radioactivity in percentage of injected radioactivity in the group injected with ^{177Lu}-PSMA I&T and receiving PAH solution ip. and iv.

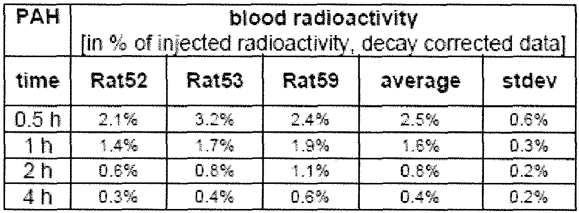

Results-Table VI.: Blood (left ventricle) radioactivity in percentage of injected radioactivity in the group injected with ^{177Lu}-PSMA I&T and receiving Saline ip. and iv.

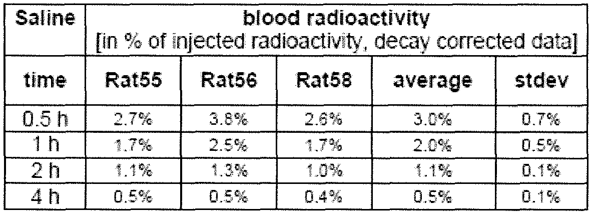

Results-Table VII.:

Renal medulla radioactivity in percentage of injected radioactivity in the group injected with ^{177Lu}-PSMA I&T and receiving the Probenecid suspension po.

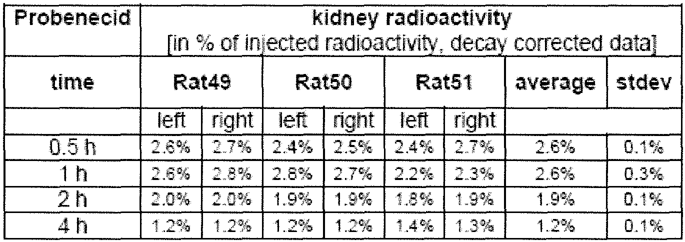

Results-Table VIII.:

Renal medulla radioactivity in percentage of injected radioactivity in the group injected with ^{177Lu}-PSMA I&T and receiving PAH solution ip. and iv.

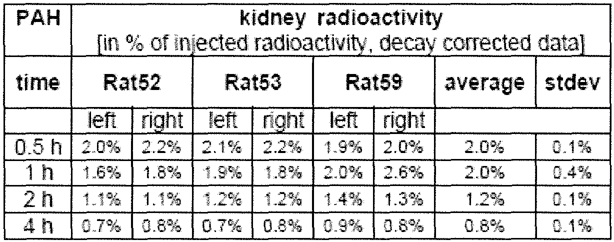

Results-Table IX.:

Renal medulla radioactivity in percentage of injected radioactivity in the group injected with ^{177Lu}-PSMA I&T and receiving the Saline ip. and iv.

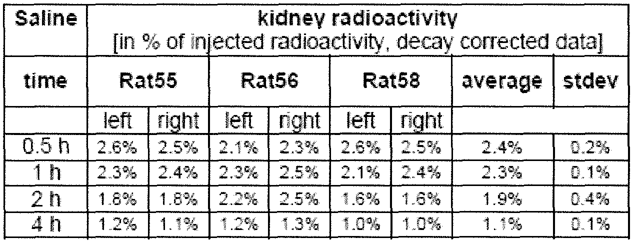

Results-Table X.:

Renal cortex radioactivity in percentage of injected radioactivity in the group injected with ^{177Lu}-PSMA I&T and receiving the Probenecid suspension po.

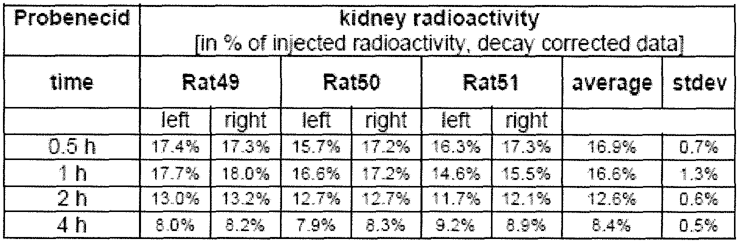

Results-Table XI.:

Renal cortex radioactivity in percentage of injected radioactivity in the group injected with ^{177Lu}-PSMA I&T and receiving PAH solution ip. and iv.

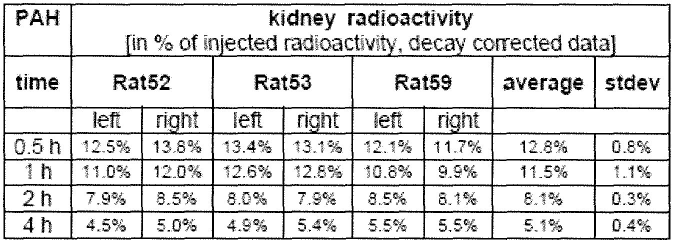

Results-Table XII.:

Renal cortex radioactivity in percentage of injected radioactivity in the group injected with ^{177Lu}-PSMA I&T and receiving the Saline ip. and iv.

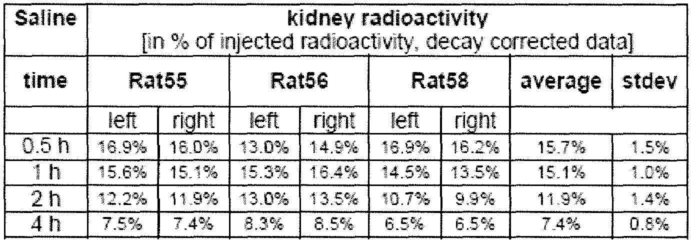

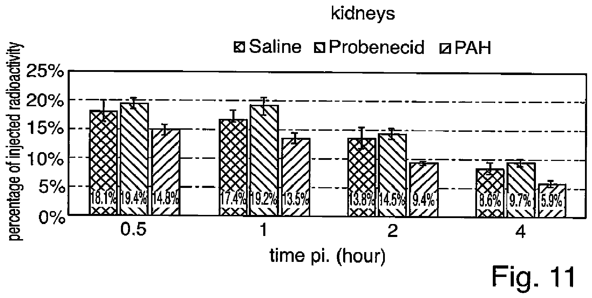

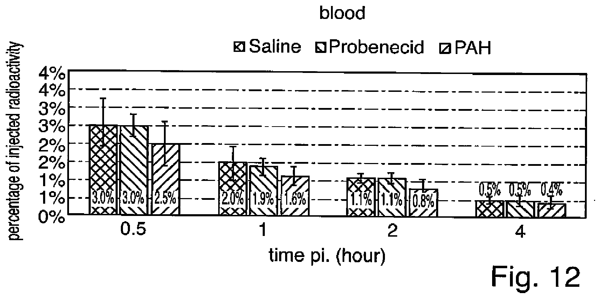

The results are depicted by Figures 11 to 14. All experiments show a marked reduction of radioactivity in kidney cells upon administration of PAH as compared to the saline control and also compared to the probenecid control experiment (Figure 11). Both the renal cortex

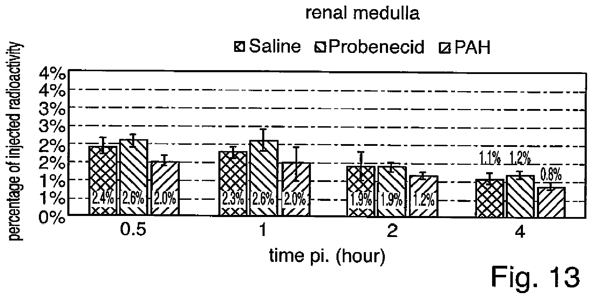

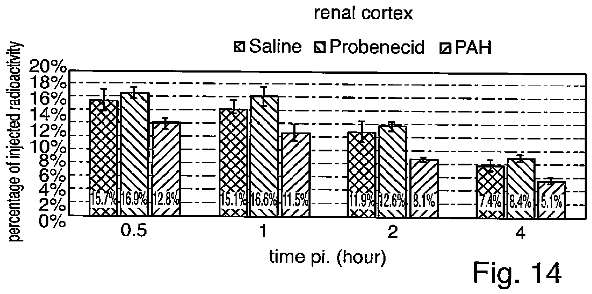

(Figure 13) and the renal medulla (Figure 14) show a reduced radioactivity at all time points in the course of the experiment upon administration of PAH.

Probenecid was tested as a further control. Probenecid was known as a strong inhibitor of OA⁻ (organic anions) secretion of the proximal tubular cells of the kidney. It inhibits the organic anion transporter Type 1 (OAT1) at the basolateral side of the cell. OAT1 is known for its uptake of organic anions from the blood into the kidney tubular cells in exchange with dicarboxylates, e.g. succinate or 2-oxoglutarate. The experiments according to Example 8 (see Figures 11 to 14) all shows no effect of the probenecid-based inhibitory mechanism on OAT1. Thus, it is to be concluded that the PAH-based effects on decreasing radioactivity in kidney cells are based on another mechanism than known for probenecid.

## Claims

1. Para-aminohippuric acid (PAH) or a pharmaceutically acceptable salt thereof for use in a method for the reduction of nephrotoxic side effects of radiolabeled therapeutic and diagnostic compounds as a radiopharmaceutical in radio ligand therapy or diagnosis in a subject,
wherein the radiopharmaceutical is a radionuclide containing conjugate molecule comprising a carrier molecule, a chelator and a radionuclide, wherein the radionuclide is a tri-valent radionuclide.

2. Para-aminohippuric acid (PAH) or a pharmaceutically acceptable salt thereof for use according to claim 1, wherein the carrier molecule is selected from peptides, peptidomimetics, antibody fragments, antibody mimetics, small molecules, and knottings,
wherein the carrier molecule is preferably selected from somatostatin analogues, PSMA-inhibitors, gastrin analogues, integrin binding molecules and folate,
wherein the carrier molecule is more preferably selected from the group consisting of Tyr3-octeotride, Tyr3-octreotate, NOC, JR10, JR11, BASS, LM3, PSMA-11, Sargastrin, RGD and folate.

3. Para-aminohippuric acid (PAH) or a pharmaceutically acceptable salt thereof for use according any one of claim 1 or 2, wherein the chelator is a macrocyclic chelator, preferably selected from the group consisting of DOTA, HBED-CC, NOTA, NODAGA, DOTAGA, DOTAM, TRAP, NOPO, PCTA, DFO, DTPA, DO3AP, DO3AP^{PrA}, DO3AP^{ABn}, and HYNIC or derivatives thereof.

4. Para-aminohippuric acid (PAH) or a pharmaceutically acceptable salt thereof for use according to any one of claims 1 to 3, wherein the radionuclide is selected from selected from the group consisting of ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, ²²⁵Ac, ²²⁷Th, ¹⁵³Sm, ¹⁶⁶Ho, ¹⁵²Gd, ¹⁵³Gd, ¹⁵⁷Gd, or ¹⁶⁶Dy, preferably from the group consisting of ¹⁷⁷Lu, ⁹⁰Y, ⁶⁷Ga, ⁶⁸Ga, ¹¹¹In, ²²⁵Ac, ¹⁶¹Tb, ⁴⁴Sc and ⁴⁷Sc, more preferably from the group consisting of ¹⁷⁷Lu, ²²⁵Ac and ⁶⁸Ga.

5. Para-aminohippuric acid (PAH) or a pharmaceutically acceptable salt thereof for use according to any one of claims 1 to 4, wherein the radionuclide containing conjugate molecule is selected from [¹⁷⁷Lu-DOTA°-Tyr3]-octreotide, ¹⁷⁷Lu-DOTANOC (¹⁷⁷Lu-DOTA-D-Phe-cyclo(Cys-1-Nal-D-Trp-Lys-Thr-Cys)Thr(ol)), ¹⁷⁷Lu-DOTATATE (¹⁷⁷Lu-DOTA-D-Phe-cy-clo(Cys-Tyr-D-Trp-Lys-Thr-Cys)Thr), ⁶⁸Ga-DOTATOC (⁶⁸Ga-DOTA-D-Phe-cyclo(Cys-Tyr-D-Trp-Lys-Thr-Cys)Thr(ol)), ⁶⁸Ga-DOTANOC (⁶⁸Ga-DOTA-D-Phe-cyclo(Cys-1-Nal-D-Trp-Lys-Thr-Cys)Thr(ol)), ⁹⁰Y-DOTATOC (⁹⁰Y-DOTA-D-Phe-cyclo(Cys-Tyr-D-Trp-Lys-Thr-Cys)Thr(ol)), ⁹⁰Y-DOTATATE (⁹⁰Y-DOTA-D-Phe-cyclo(Cys-Tyr-D-Trp-Lys-Thr-Cys)Thr), ¹¹¹In-DTPA-octreotide (¹¹¹In-DTPA-D-Phe-cyclo(Cys-Phe-D-Trp-Lys-Thr-Cys)Thr(ol)), ¹¹¹In-DOTA-BASS (¹¹¹ln-DOTA-p-NO₂-Phe-cyclo-(D-Cys-Tyr-D-Trp-Lys-Thr-Cys)D-Tyr-NH₂, ¹¹¹In-DOTA-JR11 (¹¹¹In-DOTA-Cpa-cyclo[D-Cys-Aph(Hor)-D-Aph(Cbm)-Lys-Thr-Cys]D-Tyr-NH₂), ⁶⁸Ga-DOTA-JR11 (Ga-OpS201) (⁶⁸Ga-DOTA-Cpa-cyclo[D-Cys-Aph(Hor)-D-Aph(Cbm)-Lys-Thr-Cys]D-Tyr-NH2), ⁶⁸Ga-DODAGA-JR11 (Ga-OPS202) (⁶⁸Ga-NODAGA-Cpa-cyclo[D-Cys-Aph(Hor)-D-Aph(Cbm)-Lys-Thr-Cys]D-Tyr-NH₂), ¹⁷⁷Lu-DOTA-JR11, ¹⁷⁷Lu-DOTA-RGD, ¹⁷⁷Lu-DOTA-Sargastrin, ⁶⁸Ga-HBED-CC-PSMA-11, and ¹⁷⁷Lu-PSMA I&T.

6. Para-aminohippuric acid (PAH) or a pharmaceutically acceptable salt thereof for use according to any one of claims 1 to 5, wherein para-aminohippuric acid (PAH) or a pharmaceutically acceptable thereof is administered in combination with a further substance which reduces nephrotoxic side effects of radiolabeled therapeutic and diagnostic compounds,
wherein the further substance is preferably selected from amino acids, gelatine, Amifostine, albumin-derived peptides, PSMA-binding molecules, such as PMPA, and vitamins.

7. Para-aminohippuric acid (PAH) or a pharmaceutically acceptable salt thereof for use according to any one of claims 1 to 6, wherein PAH or a pharmaceutically acceptable salt thereof is administered in an amount of 5 mg to 500 mg per kilogram of body weight, and/or
wherein the therapeutic or diagnostic compound and PAH or a pharmaceutically acceptable salt thereof are administered in a ratio from 1/240000 to 1/8000 (w/w).

8. Para-aminohippuric acid (PAH) or a pharmaceutically acceptable salt thereof for use according to any one of claims 1 to 7, wherein the pharmaceutically acceptable salt of PAH is aminohippurate sodium.

9. Para-aminohippuric acid (PAH) or a pharmaceutically acceptable salt thereof for use according to any one of claims 1 to 8, wherein the radio ligand therapy or diagnostic is for therapy or imaging of cancer, in particular neuroendocrine tumors, prostate cancer, pancreatic cancer, renal cancer, bladder cancer, medullar thyroid carcinomas, small cell lung cancers, stromal ovarian carcinomas, ductal pancreatic adenocarcinomas, insulinomas, gastrinomas and breast cancer.

10. A pharmaceutical composition comprising a radiolabeled pharmaceutical compound and para-aminohippuric acid (PAH) or a pharmaceutically acceptable salt thereof, and a pharmaceutically acceptable excipient, diluent, carrier or a combination thereof,
wherein the radiolabeled pharmaceutical compound is a radionuclide containing conjugate molecule comprising a carrier molecule, a chelator and a radionuclide, wherein the radionuclide is a tri-valent radionuclide.

11. The pharmaceutical composition according to claim 10, wherein the carrier molecule is selected from peptides, peptidomimetics, antibody fragments, antibody mimetics, small molecules, and knottings,
wherein the carrier molecule is preferably selected from somatostatin analogues, PSMA-inhibitors, gastrin analogues, integrin binding molecules and folate conjugates,
wherein the carrier molecule is more preferably selected from Tyr3-octeotride, Tyr3-octreotate, JR11, PSMA-11, Sargastrin, RGD and folate.

12. The pharmaceutical composition according any one of claim 10 or 11, wherein the chelator is selected from DOTA, HBED-CC, NOTA, NODAGA, DOTAGA, DOTAM, TRAP, NOPO, PCTA, DFO, DTPA, DO3AP, DO3A^{PrA}, DO3AP^{ABn}, and HYNIC or derivatives thereof.

13. The pharmaceutical composition according to any one of claims 10 to 12, wherein the radionuclide is selected from the group consisting of ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, ²²⁵Ac, ¹⁵³Sm, ¹⁶⁶Ho, ¹⁶⁶Dy, preferably selected from the group consisting of ¹⁷⁷Lu, ⁹⁰Y, ⁶⁷Ga, ⁶⁸Ga, ¹¹¹In, ²²⁵Ac, ¹⁶¹Tb, ⁴⁴Sc and ⁴⁷Sc, more preferably from ¹⁷⁷Lu, ²²⁵Ac and ⁶⁸Ga.

14. The pharmaceutical composition according to any one of claims 10 to 13, wherein the radionuclide containing conjugate molecule is selected from [¹⁷⁷Lu-DOTA°-Tyr3]-octreotide, ¹⁷⁷Lu-DOTA-JR11, ¹⁷⁷Lu-DOTA-RGD, ¹⁷⁷Lu-DOTA-Sargastrin, ⁶⁸Ga-HBED-CC-PSMA-11, and ¹⁷⁷Lu-PSMA I&T.

15. The pharmaceutical composition according to any one of claims 10 to 14, wherein the composition comprises in addition to PAH a further substance which reduces nephrotoxic side effects of radiolabeled therapeutic and diagnostic compounds,
wherein the further substance reducing nephrotoxic side effects of therapeutic and diagnostic compounds other than PAH is preferably selected from amino acids, gelatine, Amifostine, albumin-derived peptides, PSMA-binding molecules, such as PMPA, and vitamins.

16. The pharmaceutical composition according to any one of claims 10 to 15, wherein the therapeutic or diagnostic compound and PAH are present in the pharmaceutical composition in a ratio of from 1/240000 to 1/8000 (w/w).

17. The pharmaceutical composition according to any one of claims 10 to 16, wherein the pharmaceutically acceptable salt of PAH is aminohippurate sodium.

18. A kit comprising para-aminohippuric acid (PAH) or a pharmaceutically acceptable salt thereof in one part of the kit, and further comprising a radiolabeled therapeutic or diagnostic compound as a radiopharmaceutical as defined by any one of claims 1 to 5 in another part of the kit.

19. Composition according to any one of claims 10 to 17 or a kit according to claim 18 for use in a method for the reduction of nephrotoxic side effects of radiolabeled therapeutic and diagnostic compounds in a subject.

20. Para-aminohippuric acid (PAH) or a pharmaceutically acceptable salt thereof in combination with a radiolabeled therapeutic or diagnostic compound for use in a method for the reduction of nephrotoxic side effects of radiolabeled therapeutic and diagnostic compounds as a radiopharmaceutical in radio ligand therapy or diagnostic in a subject comprising administering to a subject para-aminohippuric acid (PAH) or a pharmaceutically acceptable salt thereof in combination with a radiolabeled therapeutic or diagnostic compound as a radiopharmaceutical in radio ligand therapy or diagnosis, wherein the administration of PAH is prior and/or during and/or after administration of the radiolabeled therapeutic or diagnostic compound, in particular (i) prior, during and after or (ii) prior and after administration of the radiolabeled therapeutic or diagnostic compound,
wherein the radiopharmaceutical is a radionuclide containing conjugate molecule comprising a carrier molecule, a chelator and a radionuclide, wherein the radionuclide is a tri-valent radionuclide.

21. Para-aminohippuric acid (PAH) or a pharmaceutically acceptable salt thereof in combination with a radiolabeled therapeutic or diagnostic compound for use according to claim 20, wherein the carrier molecule is selected from peptides, peptidomimetics, small molecules, and knottings,
wherein the carrier molecule is preferably selected from somatostatin analogues, PSMA-inhibitors, gastrin analogues, integrin binding molecules and folates;
wherein the carrier molecule is more preferably selected from Tyr3-octeotride, Tyr3-oc-treotate, JR11, PSMA-11, Sargastrin, RGD and folate.

22. Para-aminohippuric acid (PAH) or a pharmaceutically acceptable salt thereof in combination with a radiolabeled therapeutic or diagnostic compound for use according any one of claim 20 or 21, wherein the chelator is selected from DOTA, HBED-CC, NOTA, NODAGA, DOTAGA, DOTAM, TRAP, NOPO, PCTA, DFO, DTPA, DO3AP, DO3AP^{PrA}, DO3AP^{ABn}, and HYNIC or derivatives thereof.

23. Para-aminohippuric acid (PAH) or a pharmaceutically acceptable salt thereof in combination with a radiolabeled therapeutic or diagnostic compound for use according to any one of claims 20 to 22, wherein the radionuclide is selected from the group consisting of ¹⁷⁷Lu, ⁹⁰Y, ⁶⁷Ga, ⁶⁸Ga, ¹¹¹In, ²²⁵Ac, ¹⁶¹Tb, ⁴⁴Sc and ⁴⁷Sc, preferably from ¹⁷⁷Lu, ²²⁵Ac and ⁶⁸Ga.

24. Para-aminohippuric acid (PAH) or a pharmaceutically acceptable salt thereof in combination with a radiolabeled therapeutic or diagnostic compound for use according to any one of claims 20 to 23, wherein the radionuclide containing conjugate molecule is selected from [¹⁷⁷Lu-DOTA°-Tyr3]-octreotide, ¹⁷⁷Lu-DOTA-JR11, ¹⁷⁷Lu-DOTA-RGD, ¹⁷⁷Lu-DOTA-Sargastrin, ⁶⁸Ga-HBED-CC-PSMA-11, and ¹⁷⁷Lu-PSMA I&T.

25. Para-aminohippuric acid (PAH) or a pharmaceutically acceptable salt thereof in combination with a radiolabeled therapeutic or diagnostic compound for use according to any one of claims 20 to 24, wherein para-aminohippuric acid (PAH) or a pharmaceutically acceptable salt thereof is used in combination with a further substance which reduces nephrotoxic side effects of radiolabeled therapeutic and diagnostic compounds,
wherein the further substance reducing nephrotoxic side effects of radiolabeled therapeutic and diagnostic compounds other than PAH is preferably selected from amino acids, gelatine, Amifostine, albumin-derived peptides, PSMA-binding molecules, such as PMPA, and vitamins.

26. Para-aminohippuric acid (PAH) or a pharmaceutically acceptable salt thereof in combination with a radiolabeled therapeutic or diagnostic compound for use according to any one of claims 20 to 25, wherein PAH or a pharmaceutically acceptable salt is administered in an amount of 5 mg to 500 mg per kilogram of body weight,
and/or
wherein the therapeutic and/or diagnostic compound(s) and PAH or a pharmaceutically acceptable salt thereof are used in a ratio of from 1/240000 to 1/8000 (w/w).

27. Para-aminohippuric acid (PAH) or a pharmaceutically acceptable salt thereof in combination with a radiolabeled therapeutic or diagnostic compound for use according to any one of claims 20 to 26, wherein the pharmaceutically acceptable salt of PAH is aminohippurate sodium.

## Patentansprüche

1. Para-Aminohippursäure (PAH) oder ein pharmazeutisch annehmbares Salz davon zur Verwendung in einem Verfahren zur Verringerung der nephrotoxischen Nebenwirkungen von radioaktiv markierten therapeutischen und diagnostischen Verbindungen als Radiopharmazeutikum in der Radioligandentherapie oder -diagnose bei einem Patienten, wobei das Radiopharmazeutikum ein Radionuklid enthaltendes Konjugatmolekül ist, das ein Trägermolekül, einen Chelator und ein Radionuklid umfasst, wobei das Radionuklid ein dreiwertiges Radionuklid ist.

2. Para-Aminohippursäure (PAH) oder ein pharmazeutisch annehmbares Salz davon zur Verwendung gemäß Anspruch 1, wobei das Trägermolekül ausgewählt ist aus Peptiden, Peptidomimetika, Antikörperfragmenten, Antikörpermimetika, kleinen Molekülen und Knottings,
wobei das Trägermolekül vorzugsweise ausgewählt ist aus Somatostatin-Analoga, PSMA-Inhibitoren, Gastrin-Analoga, Integrin-bindenden Molekülen und Folat,
wobei das Trägermolekül weiter vorzugsweise ausgewählt ist aus der Gruppe, bestehend aus Tyr3-Octreotrid, Tyr3-Octreotat, NOC, JR10, JR11, BASS, LM3, PSMA-11, Sargastrin, RGD und Folat.

3. Para-Aminohippursäure (PAH) oder ein pharmazeutisch annehmbares Salz davon zur Verwendung nach einem der Ansprüche 1 oder 2, wobei der Chelator ein makrozyklischer Chelator ist, vorzugsweise ausgewählt aus der Gruppe, bestehend aus DOTA, HBED-CC, NOTA, NODAGA, DOTAGA, DOTAM, TRAP, NOPO, PCTA, DFO, DTPA, D03AP, DO3AP^{PrA}, DO3AP^{ABn} und HYNIC oder Derivaten davon.

4. Para-Aminohippursäure (PAH) oder ein pharmazeutisch annehmbares Salz davon zur Verwendung nach einem der Ansprüche 1 bis 3, wobei das Radionuklid ausgewählt ist aus der Gruppe, bestehend aus ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, ²²⁵Ac, ²²⁷Th, ¹⁵³Sm, ¹⁶⁶Ho, ¹⁵²Gd, ¹⁵³Gd, ¹⁵⁷Gd oder ¹⁶⁶Dy, vorzugsweise aus der Gruppe bestehend aus ¹⁷⁷Lu, ⁹⁰Y, ⁶⁷Ga, ⁶⁸Ga, ¹¹¹In, ²²⁵Ac, ¹⁶¹Tb, ⁴⁴Sc und ⁴⁷Sc, besonders bevorzugt aus der Gruppe bestehend aus ¹⁷⁷Lu, ²²⁵Ac und ⁶⁸Ga.

5. Para-Aminohippursäure (PAH) oder ein pharmazeutisch annehmbares Salz davon zur Verwendung nach einem der Ansprüche 1 bis 4, wobei das Radionuklid enthaltende Konjugatmolekül ausgewählt ist aus [¹⁷⁷Lu-DOTA°-Tyr3]-octreotide, ¹⁷⁷Lu-DOTANOC (¹⁷⁷Lu-DOTA-D-Phe-cyclo(Cys-1-Nal-D-Trp-Lys-Thr-Cys)Thr(ol)), ¹⁷⁷Lu-DOTATATE (¹⁷⁷Lu-DOTA-D-Phe-cyclo(Cys-Tyr-D-Trp-Lys-Thr-Cys)Thr), ⁶⁸Ga-DOTATOC (⁶⁸Ga-DOTA-D-Phe-cyclo(Cys-Tyr-D-Trp-Lys-Thr-Cys)Thr(ol)), ⁶⁸Ga-DOTANOC (⁶⁸Ga-DOTA-D-Phe-cyclo(Cys-1-Nal-D-Trp-Lys-Thr-Cys)Thr(ol)), ⁹⁰Y-DOTATOC (⁹⁰Y-DOTA-D-Phe-cyclo(Cys-Tyr-D-Trp-Lys-Thr-Cys)Thr(ol)), ⁹⁰Y-DOTATATE (⁹⁰Y-DOTA-D-Phe-cyclo(Cys-Tyr-D-Trp-Lys-Thr-Cys)Thr), ¹¹¹In-DTPA-octreotide (¹¹¹In-DTPA-D-Phe-cyclo(Cys-Phe-D-Trp-Lys-Thr-Cys)Thr(ol)), ¹¹¹In-DOTA-BASS (¹¹¹In-DOTA-p-NO₂-Phe-cyclo-(D-Cys-Tyr-D-Trp-Lys-Thr-Cys)D-Tyr-NH₂, ¹¹¹In-DOTA-JR11 (¹¹¹In-DOTA-Cpa-cyclo[D-Cys-Aph(Hor)-D-Aph(Cbm)-Lys-Thr-Cys]D-Tyr-NH₂), ⁶⁸Ga-DOTA-JR11 (Ga-OpS201) (⁶⁸Ga-DOTA-Cpa-cyclo[D-Cys-Aph(Hor)-D-Aph(Cbm)-Lys-Thr-Cys]D-Tyr-NH₂), ⁶⁸Ga-DODAGA-JR11 (Ga-OPS202) (⁶⁸Ga-NODAGA-Cpa-cyclo[D-Cys-Aph(Hor)-D-Aph(Cbm)-Lys-Thr-Cys]D-Tyr-NH2), ¹⁷⁷Lu-DOTA-JR11, ¹⁷⁷Lu-DOTA-RGD, ¹⁷⁷Lu-DOTA-Sargastrin, ⁶⁸Ga-HBED-CC-PSMA-11, und ¹⁷⁷Lu-PSMA I&T.

6. Para-Aminohippursäure (PAH) oder ein pharmazeutisch akzeptables Salz davon zur Verwendung nach einem der Ansprüche 1 bis 5, wobei para-Aminohippursäure (PAH) oder ein pharmazeutisch akzeptables Salz davon in Kombination mit einer weiteren Substanz verabreicht wird, die nephrotoxische Nebenwirkungen von radiomarkierten therapeutischen und diagnostischen Verbindungen reduziert,
wobei die weitere Substanz vorzugsweise aus Aminosäuren, Gelatine, Amifostin, von Albumin abgeleiteten Peptiden, PSMA-bindenden Molekülen, wie PMPA, und Vitaminen ausgewählt ist.

7. Para-Aminohippursäure (PAH) oder ein pharmazeutisch akzeptables Salz davon zur Verwendung nach einem der Ansprüche 1 bis 6, wobei PAH oder ein pharmazeutisch akzeptables Salz davon in einer Menge von 5 mg bis 500 mg pro kg Körpergewicht verabreicht wird,
und/oder
wobei die therapeutische oder diagnostische Verbindung und PAH oder ein pharmazeutisch akzeptables Salz davon in einem Verhältnis von 1/240000 bis 1/8000 (w/w) verabreicht werden.

8. Para-Aminohippursäure (PAH) oder ein pharmazeutisch akzeptables Salz davon zur Verwendung nach einem der Ansprüche 1 bis 7, wobei das pharmazeutisch akzeptable Salz von PAH Aminohippurat-Natrium ist.

9. Para-Aminohippursäure (PAH) oder ein pharmazeutisch annehmbares Salz davon zur Verwendung nach einem der Ansprüche 1 bis 8, wobei die Radioligandentherapie oder -diagnose zur Therapie oder Bildgebung von Krebs dient, insbesondere von neuroendokrinen Tumoren, Prostatakrebs, Pankreaskrebs, Nierenkrebs, Blasenkrebs, medullären Schilddrüsenkarzinomen, kleinzelligen Lungenkarzinomen, stromalen Ovarialkarzinomen, duktalen Pankreasadenokarzinomen, Insulinomen, Gastrinomen und Brustkrebs.

10. Eine pharmazeutische Zusammensetzung, umfassend eine radiomarkierte pharmazeutische Verbindung und para-Aminohippursäure (PAH) oder ein pharmazeutisch annehmbares Salz davon und einen pharmazeutisch annehmbaren Hilfsstoff, Verdünnungsmittel, Träger oder eine Kombination davon,
wobei die radiomarkierte pharmazeutische Verbindung ein Radionuklid-enthaltendes Konjugatmolekül ist, das ein Trägermolekül, einen Chelatbildner und ein Radionuklid umfasst, wobei das Radionuklid ein dreiwertiges Radionuklid ist.

11. Pharmazeutische Zusammensetzung nach Anspruch 10, wobei das Trägermolekül ausgewählt ist aus Peptiden, Peptidomimetika, Antikörperfragmenten, Antikörpermimetika, kleinen Molekülen und Knottings,
wobei das Trägermolekül vorzugsweise ausgewählt ist aus Somatostatin-Analoga, PSMA-Inhibitoren, Gastrin-Analoga, Integrin-bindenden Molekülen und Folat-Konjugaten,
wobei das Trägermolekül weiter vorzugsweise ausgewählt ist aus Tyr3-Octreotrid, Tyr3-Octreotat, JR11, PSMA-11, Sargastrin, RGD und Folat.

12. Pharmazeutische Zusammensetzung nach einem der Ansprüche 10 oder 11, wobei der Chelator ausgewählt ist aus DOTA, HBED-CC, NOTA, NODAGA, DOTAGA, DOTAM, TRAP, NOPO, PCTA, DFO, DTPA, D03AP, DO3AP^{PrA}, DO3AP^{ABn} und HYNIC oder Derivaten davon.

13. Pharmazeutische Zusammensetzung nach einem der Ansprüche 10 bis 12, wobei das Radionuklid ausgewählt ist aus der Gruppe, bestehend aus ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, ²²⁵Ac, ¹⁵³Sm, ¹⁶⁶Ho, ¹⁶⁶Dy, vorzugsweise ausgewählt aus der Gruppe, bestehend aus ¹⁷⁷Lu, ⁹⁰Y, ⁶⁷Ga, ⁶⁸Ga, ¹¹¹In, ²²⁵Ac, ¹⁶¹Tb, ⁴⁴Sc und ⁴⁷Sc, besonders bevorzugt aus ¹⁷⁷Lu, ²²⁵Ac und ⁶⁸Ga.

14. Pharmazeutische Zusammensetzung nach einem der Ansprüche 10 bis 13, wobei das Radionuklid-enthaltende Konjugatmolekül ausgewählt ist aus [¹⁷⁷Lu-DOTA°-Tyr3]-Octreotid, ¹⁷⁷Lu-DOTA-JR11, ¹⁷⁷Lu-DOTA-RGD, ¹⁷⁷Lu-DOTA-Sargastrin, ⁶⁸Ga-HBED-CC-PSMA-11 und ¹⁷⁷Lu-PSMA I&T.

15. Pharmazeutische Zusammensetzung nach einem der Ansprüche 10 bis 14, wobei die Zusammensetzung zusätzlich zu PAH eine weitere Substanz enthält, die nephrotoxische Nebenwirkungen von radioaktiv markierten therapeutischen und diagnostischen Verbindungen reduziert,
wobei die weitere Substanz, die die nephrotoxischen Nebenwirkungen von therapeutischen und diagnostischen Verbindungen reduziert und nicht PAH ist, vorzugsweise aus Aminosäuren, Gelatine, Amifostin, von Albumin abgeleiteten Peptiden, PSMA-bindenden Molekülen, wie PMPA, und Vitaminen ausgewählt ist.

16. Pharmazeutische Zusammensetzung nach einem der Ansprüche 10 bis 15, wobei die therapeutische oder diagnostische Verbindung und PAH in der pharmazeutischen Zusammensetzung in einem Verhältnis von 1/240000 bis 1/8000 (w/w) vorliegen.

17. Pharmazeutische Zusammensetzung nach einem der Ansprüche 10 bis 16, wobei das pharmazeutisch akzeptable Salz von PAH Aminohippurat-Natrium ist.

18. Ein Kit, umfassend para-Aminohippursäure (PAH) oder ein pharmazeutisch annehmbares Salz davon in einem Teil des Kits und ferner umfassend eine radiomarkierte therapeutische oder diagnostische Verbindung als Radiopharmazeutikum, wie in einem der Ansprüche 1 bis 5 definiert, in einem anderen Teil des Kits.

19. Zusammensetzung nach einem der Ansprüche 10 bis 17 oder ein Kit nach Anspruch 18 zur Verwendung in einem Verfahren zur Verringerung der nephrotoxischen Nebenwirkungen von radiomarkierten therapeutischen und diagnostischen Verbindungen bei einem Patienten.

20. Para-Aminohippursäure (PAH) oder ein pharmazeutisch annehmbares Salz davon in Kombination mit einer radiomarkierten therapeutischen oder diagnostischen Verbindung zur Verwendung in einem Verfahren zur Verringerung der nephrotoxischen Nebenwirkungen von radiomarkierten therapeutischen und diagnostischen Verbindungen als Radiopharmazeutikum in der Radioligandentherapie oder -diagnose bei einem Patienten, umfassend die Verabreichung von para-Aminohippursäure (PAH) oder einem pharmazeutisch annehmbaren Salz davon in Kombination mit einer radiomarkierten therapeutischen oder diagnostischen Verbindung als Radiopharmazeutikum in der Radioligandentherapie oder -diagnose an einen Patienten, wobei die Verabreichung von PAH vor und/oder während und/oder nach der Verabreichung der radiomarkierten therapeutischen oder diagnostischen Verbindung erfolgt, insbesondere (i) vor, während und nach oder (ii) vor und nach der Verabreichung der radiomarkierten therapeutischen oder diagnostischen Verbindung,
wobei das Radiopharmazeutikum ein Radionuklid-enthaltendes Konjugatmolekül ist, das ein Trägermolekül, einen Chelatbildner und ein Radionuklid umfasst, wobei das Radionuklid ein dreiwertiges Radionuklid ist.

21. Para-Aminohippursäure (PAH) oder ein pharmazeutisch annehmbares Salz davon in Kombination mit einer radiomarkierten therapeutischen oder diagnostischen Verbindung zur Verwendung nach Anspruch 20, wobei das Trägermolekül ausgewählt ist aus Peptiden, Peptidomimetika, kleinen Molekülen und Knottings,
wobei das Trägermolekül vorzugsweise ausgewählt ist aus Somatostatin-Analoga, PSMA-Inhibitoren, Gastrin-Analoga, Integrin-bindenden Molekülen und Folaten;
wobei das Trägermolekül weiter vorzugsweise ausgewählt ist aus Tyr3-Octreotrid, Tyr3-Octreotat, JR11, PSMA-11, Sargastrin, RGD und Folat.

22. Para-Aminohippursäure (PAH) oder ein pharmazeutisch annehmbares Salz davon in Kombination mit einer radiomarkierten therapeutischen oder diagnostischen Verbindung zur Verwendung nach einem der Ansprüche 20 oder 21, wobei der Chelator ausgewählt ist aus DOTA, HBED-CC, NOTA, NODAGA, DOTAGA, DOTAM, TRAP, NOPO, PCTA, DFO, DTPA, DO3AP, DO3AP^{Pra}, DO3AP^{ABn} und HYNIC oder Derivaten davon.

23. Para-Aminohippursäure (PAH) oder ein pharmazeutisch annehmbares Salz davon in Kombination mit einer radiomarkierten therapeutischen oder diagnostischen Verbindung zur Verwendung nach einem der Ansprüche 20 bis 22, wobei das Radionuklid ausgewählt ist aus der Gruppe, bestehend aus ¹⁷⁷Lu, ⁹⁰Y, ⁶⁷Ga, ⁶⁸Ga, ¹¹In, ²²⁵Ac, ¹⁶¹Tb, ⁴⁴Sc und ⁴⁷Sc, vorzugsweise aus ¹⁷⁷Lu, ²²⁵Ac und ⁶⁸Ga.

24. Para-Aminohippursäure (PAH) oder ein pharmazeutisch akzeptables Salz davon in Kombination mit einer radiomarkierten therapeutischen oder diagnostischen Verbindung zur Verwendung nach einem der Ansprüche 20 bis 23, wobei das Radionuklid-erhaltende Konjugatmolekül ausgewählt ist aus [¹⁷⁷Lu-DOTA°-Tyr3]-Octreotid, ¹⁷⁷Lu-DOTA-JR11, ¹⁷⁷Lu-DOTA-RGD, ¹⁷⁷Lu-DOTA-Sargastrin, ⁶⁸Ga-HBED-CC-PSMA-11 und ¹⁷⁷Lu-PSMA I&T.

25. Para-Aminohippursäure (PAH) oder ein pharmazeutisch annehmbares Salz davon in Kombination mit einer radiomarkierten therapeutischen oder diagnostischen Verbindung zur Verwendung nach einem der Ansprüche 20 bis 24, wobei para-Aminohippursäure (PAH) oder ein pharmazeutisch annehmbares Salz davon in Kombination mit einer weiteren Substanz verwendet wird, die nephrotoxische Nebenwirkungen von radiomarkierten therapeutischen und diagnostischen Verbindungen reduziert,
wobei die weitere Substanz, die die nephrotoxischen Nebenwirkungen von radioaktiv markierten therapeutischen und diagnostischen Verbindungen reduziert und nicht PAH ist, vorzugsweise aus Aminosäuren, Gelatine, Amifostin, von Albumin abgeleiteten Peptiden, PSMA-bindenden Molekülen, wie PMPA, und Vitaminen ausgewählt ist.

26. Para-Aminohippursäure (PAH) oder ein pharmazeutisch annehmbares Salz davon in Kombination mit einer radiomarkierten therapeutischen oder diagnostischen Verbindung zur Verwendung nach einem der Ansprüche 20 bis 25, wobei PAH oder ein pharmazeutisch annehmbares Salz in einer Menge von 5 mg bis 500 mg pro kg Körpergewicht verabreicht wird,
und/oder
wobei die therapeutische(n) und/oder diagnostische(n) Verbindung(en) und PAH oder ein pharmazeutisch akzeptables Salz davon in einem Verhältnis von 1/240000 bis 1/8000 (w/w) verwendet werden.

27. Para-Aminohippursäure (PAH) oder ein pharmazeutisch akzeptables Salz davon in Kombination mit einer radiomarkierten therapeutischen oder diagnostischen Verbindung zur Verwendung nach einem der Ansprüche 20 bis 26, wobei das pharmazeutisch akzeptable Salz von PAH Aminohippurat-Natrium ist.

## Revendications

1. Acide para-aminohippurique (PAH) ou un sel pharmaceutiquement acceptable de celui-ci pour une utilisation dans un procédé de réduction d'effets secondaires néphrotoxiques de composés thérapeutiques et diagnostiques radiomarqués en tant que produit pharmaco-radioactif dans une thérapie ou un diagnostic par ligand radioactif chez un sujet,
dans lequel le produit pharmaco-radioactif est une molécule conjuguée contenant un radionucléide comprenant une molécule de support, un chélateur et un radionucléide, dans lequel le radionucléide est un radionucléide trivalent.

2. Acide para-aminohippurique (PAH) ou un sel pharmaceutiquement acceptable de celui-ci pour une utilisation selon la revendication 1, dans lequel la molécule de support est choisie parmi les peptides, les peptidomimétiques, les fragments d'anticorps, les mimétiques d'anticorps, les petites molécules et les nouages, dans lequel la molécule de support est de préférence choisie parmi les analogues de somatostatine, les inhibiteurs de PSMA, les analogues de gastrine, les molécules de liaison à l'intégrine et le folate,
dans lequel la molécule de support est de manière davantage préférée choisie dans le groupe constitué du Tyr3-octréotride, du Tyr3-octréotate, du NOC, du JR10, du JR11, du BASS, du LM3, du PSMA-11, de la Sargastrine, du RGD et du folate.

3. Acide para-aminohippurique (PAH) ou un sel pharmaceutiquement acceptable de celui-ci pour une utilisation selon l'une quelconque de la revendication 1 ou 2, dans lequel le chélateur est un chélateur macrocyclique, de préférence choisi dans le groupe constitué du DOTA, de l'HBED-CC, du NOTA, du NODAGA, du DOTAGA, du DOTAM, du TRAP, du NOPO, du PCTA, du DFO, du DTPA, du DO3AP, du DO3AP^{PrA}, du DO3AP^{ABn} et de I'HYNIC ou de dérivés de ceux-ci.

4. Acide para-aminohippurique (PAH) ou un sel pharmaceutiquement acceptable de celui-ci pour une utilisation selon l'une quelconque des revendications 1 à 3,
dans lequel le radionucléide est choisi dans le groupe constitué de ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, ²²⁵Ac, ²²⁷Th, ¹⁵³Sm, ¹⁶⁶Ho, ¹⁵²Gd, ¹⁵³Gd, ¹⁵⁷Gd ou ¹⁶⁶Dy, de préférence dans le groupe constitué de ¹⁷⁷Lu, ⁹⁰Y, ⁶⁷Ga, ⁶⁸Ga, ¹¹¹In, ²²⁵Ac, ¹⁶¹Tb, ⁴⁴Sc et ⁴⁷Sc, de manière davantage préférée dans le groupe constitué de ¹⁷⁷Lu, ²²⁵Ac et ⁶⁸Ga.

5. Acide para-aminohippurique (PAH) ou un sel pharmaceutiquement acceptable de celui-ci pour une utilisation selon l'une quelconque des revendications 1 à 4, dans lequel la molécule conjuguée contenant un radionucléide est choisie parmi le [¹⁷⁷Lu-DOTA-Tyr3]-octréotide, le ¹⁷⁷Lu-DOTANOC (¹⁷⁷Lu-DOTA-D-Phe-cyclo(Cys-1-Nal-D-Trp-Lys-Thr-Cys)Thr(ol)), le ¹⁷⁷Lu-DOTATATE (¹⁷⁷Lu-DOTA-D-Phe-cyclo(Cys-Tyr-D-Trp-Lys-Thr-Cys)Thr), le ⁶⁸Ga-DOTATOC (⁶⁸Ga-DOTA-D-Phe-cyclo(Cys-Tyr-D-Trp-Lys-Thr-Cys)Thr(ol)), le ⁶⁸Ga-DOTANOC (⁶⁸Ga-DOTA-D-Phe-cyclo(Cys-1-Nal-D-Trp-Lys-Thr-Cys)Thr(ol)), le ⁹⁰Y-DOTATOC (⁹⁰Y-DOTA-D-Phe-cyclo(Cys-Tyr-D-Trp-Lys-Thr-Cys)Thr(ol)), le ⁹⁰Y-DOTATATE (⁹⁰Y-DOTA-D-Phe-cyclo(Cys-Tyr-D-Trp-Lys-Thr-Cys)Thr), le ¹¹¹In-DTPA-octréotide (¹¹¹In-DTPA-D-Phe-cyclo(Cys-Phe-D-Trp-Lys-Thr-Cys)Thr(ol)), le ¹¹¹In-DOTA-BASS (¹¹¹In-DOTA-p-NO₂-Phe-cyclo-(D-Cys-Tyr-D-Trp-Lys-Thr-Cys)D-Tyr-NH₂, le ¹¹¹In-DOTA-JR11 (¹¹¹In-DOTA-Cpa-cyclo[D-Cys-Aph(Hor)-D-Aph(Cbm)-Lys-Thr-Cys]D-Tyr-NH₂), le ⁶⁸Ga-DOTA-JR11 (Ga-OpS201) (⁶⁸Ga-DOTA-Cpa-cyclo[D-Cys-Aph(Hor)-D-Aph(Cbm)-Lys-Thr-Cys]D-Tyr-NH₂), le ⁶⁸Ga-DODAGA-JR11 (Ga-OPS202) (⁶⁸Ga-NODAGA-Cpa-cyclo[D-Cys-Aph(Hor)-D-Aph(Cbm)-Lys-Thr-Cys]D-Tyr-NH₂), le ¹⁷⁷Lu-DOTA-JR11, le ¹⁷⁷Lu-DOTA-RGD, la ¹⁷⁷Lu-DOTA-Sargastrine, le ⁶⁸Ga-HBED-CC-PSMA-11 et le ¹⁷⁷Lu-PSMA I&T.

6. Acide para-aminohippurique (PAH) ou un sel pharmaceutiquement acceptable de celui-ci pour une utilisation selon l'une quelconque des revendications 1 à 5, dans lequel l'acide para-aminohippurique (PAH) ou un sel pharmaceutiquement acceptable de celui-ci est administré en combinaison avec une autre substance qui réduit les effets secondaires néphrotoxiques de composés thérapeutiques et de diagnostiques radiomarqués,
dans lequel l'autre substance est de préférence choisie parmi les acides aminés, la gélatine, l'Amifostine, les peptides dérivés de l'albumine, les molécules de liaison au PSMA, telles que le PMPA, et les vitamines.

7. Acide para-aminohippurique (PAH) ou un sel pharmaceutiquement acceptable de celui-ci pour une utilisation selon l'une quelconque des revendications 1 à 6, dans lequel le PAH ou un sel pharmaceutiquement acceptable de celui-ci est administré en une quantité de 5 mg à 500 mg par kilogramme de poids corporel, et/ou
dans lequel le composé thérapeutique ou diagnostique et le PAH ou un sel pharmaceutiquement acceptable de celui-ci sont administrés en un rapport de 1/240 000 à 1/8 000 (p/p).

8. Acide para-aminohippurique (PAH) ou un sel pharmaceutiquement acceptable de celui-ci pour une utilisation selon l'une quelconque des revendications 1 à 7, dans lequel le sel pharmaceutiquement acceptable de PAH est de l'aminohippurate de sodium.

9. Acide para-aminohippurique (PAH) ou un sel pharmaceutiquement acceptable de celui-ci pour une utilisation selon l'une quelconque des revendications 1 à 8, dans lequel la thérapie ou le diagnostic par ligand radioactif est pour la thérapie ou l'imagerie d'un cancer, en particulier de tumeurs neuroendocrines, du cancer de la prostate, du cancer pancréatique, du cancer rénal, du cancer de la vessie, de carcinomes médullaires de la thyroïde, de cancers du poumon à petites cellules, de carcinomes du stroma ovarien, d'adénocarcinomes canalaires pancréatiques, d'insulinomes, de gastrinomes et du cancer du sein.

10. Composition pharmaceutique comprenant un composé pharmaceutique radiomarqué et de l'acide para-aminohippurique (PAH) ou un sel pharmaceutiquement acceptable de celui-ci, et un excipient, diluant, support pharmaceutiquement acceptable ou une combinaison de ceux-ci,
dans laquelle le composé pharmaceutique radiomarqué est une molécule conjuguée contenant un radionucléide comprenant une molécule de support, un chélateur et un radionucléide, dans laquelle le radionucléide est un radionucléide trivalent.

11. Composition pharmaceutique selon la revendication 10, dans laquelle la molécule de support est choisie parmi les peptides, les peptidomimétiques, les fragments d'anticorps, les mimétiques d'anticorps, les petites molécules et les nouages,
dans laquelle la molécule de support est de préférence choisie parmi les analogues de somatostatine, les inhibiteurs de PSMA, les analogues de gastrine, les molécules de liaison à l'intégrine et les conjugués de folate,
dans laquelle la molécule de support est de manière davantage préférée choisie parmi le Tyr3-octréotride, le Tyr3-octréotate, le JR11, le PSMA-11, la Sargastrine, le RGD et le folate.

12. Composition pharmaceutique selon l'une quelconque de la revendication 10 ou 11, dans laquelle le chélateur est choisi parmi le DOTA, l'HBED-CC, le NOTA, le NODAGA, le DOTAGA, le DOTAM, le TRAP, le NOPO, le PCTA, le DFO, le DTPA, le D03AP, le DO3AP^{PrA}, le DO3AP^{ABn} et l'HYNIC ou les dérivés de ceux-ci.

13. Composition pharmaceutique selon l'une quelconque des revendications 10 à 12, dans laquelle le radionucléide est choisi dans le groupe constitué de ⁹⁰In, ¹¹¹In, ⁶⁷Ga, ⁶⁸Ga, ⁸⁶Y, ⁹⁰Y, ¹⁷⁷Lu, ¹⁶¹Tb, ⁴³Sc, ⁴⁴Sc, ⁴⁷Sc, ²²⁵Ac, ¹⁵³Sm, ¹⁶⁶Ho, ¹⁶⁶Dy, de préférence dans le groupe constitué de ¹⁷⁷Lu, ⁹⁰Y, ⁶⁷Ga, ⁶⁸Ga, ¹¹¹In, ²²⁵Ac, ¹⁶¹Tb, ⁴⁴Sc et ⁴⁷Sc, de manière davantage préférée parmi ¹⁷⁷Lu, ²²⁵Ac et ⁶⁸Ga.

14. Composition pharmaceutique selon l'une quelconque des revendications 10 à 13, dans laquelle la molécule conjuguée contenant un radionucléide est choisie parmi le [¹⁷⁷Lu-DOTA-Tyr3]-octréotide, le ¹⁷⁷Lu-DOTA-JR11, le ¹⁷⁷Lu-DOTA-RGD, la ¹⁷⁷Lu-DOTA-Sargastrine, le ⁶⁸Ga-HBED-CC-PSMA-11 et le ¹⁷⁷Lu-PSMA I&T.

15. Composition pharmaceutique selon l'une quelconque des revendications 10 à 14, dans laquelle la composition comprend en plus du PAH une autre substance qui réduit les effets secondaires néphrotoxiques de composés thérapeutiques et de diagnostiques radiomarqués,
dans laquelle l'autre substance réduisant les effets secondaires néphrotoxiques de composés thérapeutiques et de diagnostiques autres que le PAH est de préférence choisie parmi les acides aminés, la gélatine, l'Amifostine, les peptides dérivés de l'albumine, les molécules de liaison au PSMA, telles que le PMPA, et les vitamines.

16. Composition pharmaceutique selon l'une quelconque des revendications 10 à 15, dans laquelle le composé thérapeutique ou de diagnostique et le PAH sont présents dans la composition pharmaceutique en un rapport de 1/240 000 à 1/8 000 (p/p).

17. Composition pharmaceutique selon l'une quelconque des revendications 10 à 16, dans laquelle le sel pharmaceutiquement acceptable de PAH est de l'aminohippurate de sodium.

18. Kit comprenant l'acide para-aminohippurique (PAH) ou un sel pharmaceutiquement acceptable de celui-ci dans une partie du kit, et comprenant en outre un composé thérapeutique ou de diagnostique radiomarqué en tant que produit pharmaco-radioactif selon l'une quelconque des revendications 1 à 5 dans une autre partie du kit.

19. Composition selon l'une quelconque des revendications 10 à 17 ou kit selon la revendication 18 pour une utilisation dans un procédé de réduction d'effets secondaires néphrotoxiques de composés thérapeutiques et de diagnostiques radiomarqués chez un sujet.

20. Acide para-aminohippurique (PAH) ou un sel pharmaceutiquement acceptable de celui-ci en combinaison avec un composé thérapeutique ou de diagnostique radiomarqué pour une utilisation dans un procédé de réduction d'effets secondaires néphrotoxiques de composés thérapeutiques et de diagnostiques radiomarqués en tant que produit pharmaco-radioactif dans une thérapie ou un diagnostic par ligand radioactif chez un sujet comprenant l'administration à un sujet d'acide para-aminohippurique (PAH) ou d'un sel pharmaceutiquement acceptable de celui-ci en combinaison avec un composé thérapeutique ou de diagnostique radiomarqué en tant que produit pharmaco-radioactif dans une thérapie ou un diagnostic par ligand radioactif, dans lequel l'administration de PAH est avant et/ou pendant et/ou après administration du composé thérapeutique ou de diagnostique radiomarqué, en particulier (i) avant, pendant et après ou (ii) avant et après administration du composé thérapeutique ou de diagnostique radiomarqué,
dans lequel le produit pharmaco-radioactif est une molécule conjuguée contenant un radionucléide comprenant une molécule de support, un chélateur et un radionucléide, dans lequel le radionucléide est un radionucléide trivalent.

21. Acide para-aminohippurique (PAH) ou un sel pharmaceutiquement acceptable de celui-ci en combinaison avec un composé thérapeutique ou de diagnostique radiomarqué pour une utilisation selon la revendication 20, dans lequel la molécule de support est choisie parmi les peptides, les peptidomimétiques, les petites molécules et les nouages,
dans lequel la molécule de support est de préférence choisie parmi les analogues de somatostatine, les inhibiteurs de PSMA, les analogues de gastrine, les molécules de liaison à l'intégrine et les folates ;
dans lequel la molécule de support est de manière davantage préférée choisie parmi le Tyr3-octréotride, le Tyr3-octréotate, le JR11, le PSMA-11, la Sargastrine, le RGD et le folate.

22. Acide para-aminohippurique (PAH) ou un sel pharmaceutiquement acceptable de celui-ci en combinaison avec un composé thérapeutique ou de diagnostique radiomarqué pour une utilisation selon l'une quelconque de la revendication 20 ou 21, dans lequel le chélateur est choisi parmi le DOTA, l'HBED-CC, le NOTA, le NODAGA, le DOTAGA, le DOTAM, le TRAP, le NOPO, le PCTA, le DFO, le DTPA, le D03AP, le DO3AP^{PrA}, le DO3AP^{ABn} et l'HYNIC ou les dérivés de ceux-ci.

23. Acide para-aminohippurique (PAH) ou un sel pharmaceutiquement acceptable de celui-ci en combinaison avec un composé thérapeutique ou de diagnostique radiomarqué pour une utilisation selon l'une quelconque des revendications 20 à 22, dans lequel le radionucléide est choisi dans le groupe constitué de ¹⁷⁷Lu, ⁹⁰Y, ⁶⁷Ga, ⁶⁸Ga, ¹¹¹In, ²²⁵Ac, ¹⁶¹Tb, ⁴⁴Sc et ⁴⁷Sc, de préférence parmi ¹⁷⁷Lu, ²²⁵Ac et ⁶⁸Ga.

24. Acide para-aminohippurique (PAH) ou un sel pharmaceutiquement acceptable de celui-ci en combinaison avec un composé thérapeutique ou de diagnostique radiomarqué pour une utilisation selon l'une quelconque des revendications 20 à 23, dans lequel la molécule conjuguée contenant un radionucléide est choisie parmi le [¹⁷⁷Lu-DOTA-Tyr3]-octréotide, le ¹⁷⁷Lu-DOTA-JR11, le ¹⁷⁷Lu-DOTA-RGD, le ¹⁷⁷Lu-DOTA-Sargastrin, le ⁶⁸Ga-HBED-CC-PSMA-11 et le ¹⁷⁷Lu-PSMA I&T.

25. Acide para-aminohippurique (PAH) ou un sel pharmaceutiquement acceptable de celui-ci en combinaison avec un composé thérapeutique ou de diagnostique radiomarqué pour une utilisation selon l'une quelconque des revendications 20 à 24, dans lequel l'acide para-aminohippurique (PAH) ou un sel pharmaceutiquement acceptable de celui-ci est utilisé en combinaison avec une autre substance qui réduit les effets secondaires néphrotoxiques de composés thérapeutiques et de diagnostiques radiomarqués,
dans lequel l'autre substance réduisant les effets secondaires néphrotoxiques de composés thérapeutiques et de diagnostiques radiomarqués autres que le PAH est de préférence choisie parmi les acides aminés, la gélatine, l'Amifostine, les peptides dérivés de l'albumine, les molécules de liaison au PSMA, telles que le PMPA, et les vitamines.

26. Acide para-aminohippurique (PAH) ou un sel pharmaceutiquement acceptable de celui-ci en combinaison avec un composé thérapeutique ou de diagnostique radiomarqué pour une utilisation selon l'une quelconque des revendications 20 à 25, dans lequel du PAH ou un sel pharmaceutiquement acceptable est administré en une quantité de 5 mg à 500 mg par kilogramme de poids corporel,
et/ou
dans lequel le(s) composé(s) thérapeutique(s) et/ou de diagnostique(s) et le PAH ou un sel pharmaceutiquement acceptable de celui-ci sont utilisés en un rapport de 1/240 000 à 1/8 000 (p/p).

27. Acide para-aminohippurique (PAH) ou un sel pharmaceutiquement acceptable de celui-ci en combinaison avec un composé thérapeutique ou de diagnostique radiomarqué pour une utilisation selon l'une quelconque des revendications 20 à 26, dans lequel le sel pharmaceutiquement acceptable de PAH est de l'aminohippurate de sodium.
